# EUROPEAN PATENT SPECIFICATION

(11) **EP 1 131 340 B1**
(45) Date of publication and mention of the grant of the patent: **13.10.2004**
(21) Application number: 99968424.4
(22) Date of filing: 19.11.1999
(51) Int. Cl.: C07K 5/037, A61K 38/06, A61K 47/20, A61K 47/48, A61P 35/00

(54) **HEXAPEPTIDE WITH THE STABILIZED DISULFIDE BOND AND DERIVATIVES THEREOF REGULATING METABOLISM, PROLIFERATION, DIFFERENTIATION AND APOPTOSIS**
HEXAPEPTIDE MIT STABILISIERTER DISULFIDBINDUNG UND SEINE DERIVATE ZUR REGULATION DES METABOLISMUS, DER PROLIFERATION, DIFFERENZIERUNG UND APOPTOSE
HEXAPEPTIDE AYANT UNE LIAISON DISULFURE STABILISEE ET DERIVES DE CE DERNIER UTILISES DANS LA REGULATION DU METABOLISME, DE LA PROLIFERATION, DE LA DIFFERENTIATION ET DE L'APOPTOSE

(30) Priority: 23.11.1998 RU 98120753; 26.03.1999 RU 99105585
(43) Date of publication of application: 12.09.2001
(73) Proprietor: Novelos Therapeutics, Inc., Newton, MA 02458 (US)
(72) Inventor: KOZHEMYAKIN, Leonid Andreevich, St.Petersburg, 196240 (RU); KOZHEMYAKIN, Andrei Leonidovich, St.Petersburg, 195251 (RU); BALAZOVSKY, Mark Borisovich, St.Petersburg, 199106 (RU)
(74) Representative: Jump, Timothy John Simon
(86) International application number: PCT/RU1999/000453
(87) International publication number: WO 2000/031120

(56) References cited:
- EP-A- 0 265 719
- WO-A-97/21443
- WO-A-97/21444
- US-A- 5 104 852

## Description

### Field of the Invention

The present invention relates to medicine and, more particularly, to pharmacology, i.e., to methods for producing medicinal agents based on active metabolites of peptide origin that are intended to be used in clinical practice for preventing and treating various pathologic syndromes and diseases by way of differentiated influence on processes of metabolism, proliferation, differentiation and apoptosis of normal and transformed cells.

### Background of the Invention

Modern pharmacological industry constantly intensifying implementation into practical medicine of anti-infectious and anti-tumor chemotherapy facilitates spreading of two medical-biological problems: forming a resistance (tolerance and pharmacological efficacy decrease) to medicinal agents including cases due to activation of the MDR-genes system; and forming of undesirable resorptive effects manifested, first of all, by alteration of the immunocompetent cell system and hemopoiesis; cardio-, hepato-, nephro- and neurotoxicity. A typical cause for these problems is a wide administration of chemotherapeutic agents and ones produced by unicellular microorganisms that can be quite effective, as per their physical-chemical properties, but can also be foreign to an organism at their very nature.

Therefore, theoretical and practical medicinal research are now paying greater attention to natural key metabolites, i.e., intracellular biochemical factors usually of peptide origin that are naturally determined to trigger chain reactions for endogenous production and modification of many biologically active products for physiologically important and adequate processes.

Metabolites known to be researched extensively are, in particular, sulphur-containing peptides and derivatives thereof, first of all, this is a thiol group. In the given group, biological effects of the tripeptide "glutathione" (γ-glutamyl-cysteinyl-glycine; hereinafter - GSH) are known to be researched extensively.

The glutathione tripeptide dimer, oxidized glutathione (bis-(γ-L-glutamyl)-L-cysteinyl-bis-glycine; hereinafter - GSSG), where two molecules of the tripeptide with the aforementioned formula are linked via a covalent bond between cysteine residues, is also well known.

There were found unique properties of GSSG [1] to stimulate/modulate endogenous production of cytokine and hemopoietic factors (including colony-stimulating, i.e., growth ones). At that, stabilization with indicated in the [1] pharmaceutical agents of lifetime of exogenously introduced GSSG in biological media in an oxidized form enhanced these effects. Events developing in cells (tissues and, therefore, organs) after interaction with cytokines that are conditioned with universal influence of the cytokines on major signal-transducing systems and, through the latter, on cellular genome determining regulating effects of the cytokines on proliferation, differentiation and apoptosis are well known.

In the International application [2] there is given the group of medicinal agents that contain as an active principle the derivatives of the oxidized glutathione (GSSG) as: salts thereof; or composite drugs including combinations of GSSG and the salts thereof; or the derivatives as new compositions, i.e., formulas of new compounds when the latter is obtained through creation of the covalent bond between GSSG and another compound. At that, all technical solutions stabilizing in the varying degree the GSSG molecule disulfide form were first demonstrated to be significantly more effective in inducing the cytokine and hemopoietic factor production in normal conditions and to a greater degree in pathologic ones. Moreover, drug forms of the GSSG derivatives characterized with a greater degree of the GSSG molecule stability as the disulfide form featured new pharmacokinetics in the biological media and an ability to induce production of a greater variety of cytokines and hemopoietic factors determining largely modulating effects rather than only stimulating ones as well as reproduction of effects of a range of cytokines, for instance, IL-2.

Methods for obtaining the glutathione tripeptide dimer, i.e., the oxidized glutathione, GSSG, from the reduced glutathione (GSH) precursor are well known.

Now there are three main methods to oxidize the educed glutathione form. First, the rather labile mercapto-SH-groups of cysteine in GSH [3] can be oxidized with such soft oxidizers as air oxygen [4, 5]. However, the reaction rate is rather low in this case and desired quantitative yields require very long periods of time (many days). Catalysis by heavy metals ions and; especially, by toxic copper can create significant problems for obtaining pure pharmaceutical medicines.

Another oxidation method involves more potent oxidizers such as hydrogen peroxide, iodine, potassium ferrocyanide, etc. [6, 7]. These reactions generally proceed much faster (dozens of minutes to several hours), however, a disadvantage is a difficulty in controlling reaction conditions that can result in significant contamination of the product with oxidation products, e.g., derivatives of the corresponding acids. It is, therefore, necessary to add additional, sometimes rather labor-consuming purification procedures that can sharply appreciate the process.

Yet another oxidation method involves the use of gaseous substances (nitrogen oxides), sulfoxides and other compounds as oxidizers, however, these oxidizers can be rare and unacceptable for scaling [8, 9].

Another previously known method to obtain the oxidized glutathione as a composite with the stabilized disulfide bond that is the closest in regard of the technical essence involves the use of hydrogen peroxide as an oxidizer [6]. The process is performed in the water solution with pH about 8.0-8.5 using the hydrogen peroxide equivalent at the room temperature. The reaction time is about 1 hour and the product yield is 90%. The main impurities (up to 10%) are other oxidation products, which can be removed only by means of an expensive preparative HPLC separation that can sharply increase the drug cost.

EP-A-0 265 719 pertains to anti-neoplastic pharmaceutical compositions comprising GSH, and, interalia, cisplatin. US-A-5,104,852 relates to a method for the proliferation of cancer cells by treatment with Selenoglutathione. The combination with metal comprising material is not disclosed.

### Summary of the Invention

The present invention involves the creation of new pharmaceutically acceptable compound with predetermined properties based on GSSG, i.e., an oxidized glutathione structural analogue, videlicet, a hexapeptide with a stabilized disulfide bond as well as pharmaceutically acceptable derivatives thereof for treatment of various diseases based on regulation of the endogenous production of cytokines and hemopoietic factors and, therefore, regulation of metabolism, proliferation and differentiation in normal cells and induction of apoptotic mechanisms in tumor- and/or virus-transformed cells.

In the applied invention, including examples of the preferred embodiments, the following terminology accepted in the art is used.

Generally, a "composite" can refer to a mixture of different chemical species. The "mixture" can be a physical mixture or a chemical mixture, i.e., having a chemical interaction involving either a chemical bond or an electrostatic interaction. In one embodiment, the mixture can be prepared by dissolving and/or suspending the different chemical species in a solution and precipitating out or filtering out a resulting solid. In another embodiment, the mixture can be a homogeneous solution comprising the different chemical species, mostly not more than two agents linked with coordination (hydrogen) or weak covalent bonds.

An "oxidized glutathione-based compound" refers to any compound having a basic structure of a glutathione dimer where each unit of the dimer comprises a cation group whereupon the salt is obtained; or a glutamic acid derivative bonded to a cysteine group, or a salt derivative of glycine group, and each unit of the tripeptide is correspondingly bonded to each other by the cysteine sulfur atoms to form a sulfur-sulfur bond (disulfide bond). A "derivative" can be prepared by reacting, at least, one reactive group of the oxidized glutathione-based compound or precursor with another chemical species. An example of an oxidized glutathione-based compound is GSSG•Pt itself, i.e. the hexapeptide with the stabilized disulfide bond.

"Pharmaceutically acceptable salt" as used in this application comprises any composite derivative in the form of a salt that is acceptable for use in the body without unwanted detrimental effect on the body, and including, for example, sodium, lithium, zinc or vanadium cation, i.e. sodium, lithium, zinc or vanadium salt, respectively.

"Pharmaceutically acceptable composition" (derivative) as used in this application involves the composite, or derivative thereof, as a pharmaceutically acceptable substance and may include a group of active metabolites or other chemical compounds covalently bound to GSSG•Pt. For example, such the derivative is GSSG•Pt covalently bound to phenylalanine or to cystamine.

"Metabolism" as used in this application involves the totality of all biochemical reactions taking place within the living organism responsible for vital function maintenance in the said organism [12].

"Proliferation" as used in this application involves reproduction or multiplication of similar forms (cells) due to constituting (cellular) elements [13, 14].

"Differentiation" as used in this application involves cell changes including acquisition or possession of features distinguishing from an original with the cell conversion from relatively simple functions to more complex, specialized functions as is in morphological and/or functional heterogeneity incident to the given cellular type through the tissue-specific gene expression [15, 16, 17, 18].

"Apoptosis" as used in this application involves morphologically distinguishable forms of genetically programmed, physiological cell death initiated by extra- or intracellular signals [19, 20, 21].

"Cytokines" as used in this application comprises peptide-origin regulatory compounds produced by the different cell types playing a key role in the immune response development, hemopoiesis and different disease pathogenesis, performing their effect through gene activation, participating in regulation for all immune system elements [19, 22].

"Medicinal drug" as used in this application includes any drug form containing the composite of the present invention, e.g., GSSG•Pt and derivatives, which has a therapeutic effect on neoplastic, infectious, hematologic, immunologic, neurodegenerative or other diseases.

As used herein, the terms "neoplastic and infectious disease", "hemopoiesis and immunity depression of various origin", and "other diseases" mean any neoplastic or infectious disease, any conditions caused or accompanied by the erythroid or myeloid suppression, or a reduction in quantitative or functional immunity parameters, as well as any other disease or pathological condition, in which stimulation/modulation of the aforesaid cytokine and/or hemopoietic factor endogenous production and/or apoptosis mechanism induction would be considered advantageous by those skilled in the art.

The present invention discloses a number of oxidized glutathione-based compounds having a stabilized disulfide bond and in particular, a composite comprising the compound of this invention with a metal material in a ratio of between about 3000:1 to about 1:1 wherein the metal material includes a metal selected from the group consisting of platinum and palladium wherein the oxidized glutathione-based compound chemically interacts with the metal material. The given group of metals is selected due to catalytic properties of platinum and palladium in regard to the oxidative reactions. Preferably, the metal is platinum because it is an effective catalyst in less concentration. Ideally, the metal material, in combination with the oxidized glutathione-based compound, renders the composite soluble in biological media.

One aspect of the present invention provides a composite comprising an oxidized glutathione-based compound and a metal material. Small portions of the metal material can be insoluble, as long as the insoluble portion does not result in any toxic or hazardous effects to the biological system. A platinum material can be selected from the group consisting of a platinum salt, a coordination compound and an organometallic compound. Preferably, the platinum material is a platinum coordination compound such as cis-platin (cis-Pt(NH₃)₂Cl₂ or cis-diamminedichloroplatinum (platinum chloride or chloride salt of platinum) or potassium salt of platinum).

In a preferred embodiment, the present invention relates to the production of a new oxidized glutathione-based compound and cis-diamminedichloroplatinum or potassium platinate. A convenient short-form notation will be used herein, for example, a composite comprising GSSG itself and cis-platin will be denoted as GSSG•Pt. Derivatives will be denoted by the newly appended chemical group, e.g., bis-[histidyl]-GSSG.

The present invention also provides a new method for obtaining the oxidized glutathione as a composite with the stabilized disulfide bond having the formula: bis-(γ-L-glutamyl)-L-cysteinyl-bis-glycine disodium salt with a platinum material such as cis-diamminedichloroplatinum or potassium platinate, preferably in the mole ratio 3000:1, more preferably in a mole ratio of 1000:1, or 100:1, or 10:1.

According to the invention the composite is characterized as having a stabilized disulfide bond upon introduction thereof into biological media and, consequently, a longer drug half-life time is provided in the biological media in the disulfide form.

The general procedure of the present method for the composite production involves using the reduced glutathione for the oxidation reaction as a hydrogen peroxide oxidizer combined with a platinum material, in particular, cis-diamminedichloroplatinum or potassium platinate, as a catalyst. The method allows using lesser amounts of hydrogen peroxide (for example, 0.9 of an equivalent), resulting in an elimination of the superoxidation products along with very high yield for GSSG (more than 98% by the HPLC data). Thus, the product obtained has high purity and does not require additional purification.

It should be noted that simple mixing of the ready oxidized glutathione obtained through any of the aforementioned procedures with the complex platinum compound in the given proportion provide significantly less technological and economical effect. In the case one will incur additional expenses on purchase of the ready oxidized glutathione (its price is 2-3 times more than for the reduced one) or on performing the oxidized form synthesis, spending funds for the obtained drug purification, i.e., two-stage composite production takes place.

Another aspect of the invention provides a method for stabilizing a disulfide bond of the oxidized glutathione-based compound. The method comprises of interaction of the oxidized glutathione-based compound with the metal material. The metal material comprises of the metal selected from the group consisting of platinum and palladium. "Stabilizing a disulfide bond" refers to a process for maintaining a bond between two sulfur atoms of the GSSG cysteine and preventing facile reversion of the oxidized glutathione-based compound (e.g., GSSG) back to the reduced form (e.g., GSH). By maintaining the glutathione-based compound in a form of GSSG for a greater amount of time, the compound can be pharmaceutically effective for a correspondingly longer period of time in biological media.

In one embodiment, "interacting the oxidized glutathione-based compound with a metal material" comprises providing a glutathione-based compound and reacting this compound with an oxidant and a platinum material. A "glutathione-based compound" refers to any compound having a structure comprising a glutamic acid/salt/derivative bonded to a cysteine/salt/derivative bonded to a glycine/salt/derivative. Examples of glutathione-based compound include glutathione itself or any derivative, where a derivative can be prepared by reacting a reactive group with another chemical species. The resulting product will be a structural analogue of the oxidized glutathione, i.e. the hexapeptide with a stabilized disulfide bond. Thus, in this embodiment of the invention, a glutathione-based compound is in a reduced form, such as GSH, and the reaction with an oxidant involves oxidizing the glutathione-based compound to produce a sulfur-sulfur bond. The oxidant can be any species which can cleave a S-H bond of a glutathione-based compound to produce a hydrogen atom and a compound having a sulfur-based radical which ultimately can react with another sulfur-based radical to provide the sulfur-sulfur bond. Various oxidants that can perform this S-H bond cleavage are well known in the art. In a preferred embodiment, the oxidant is selected from the group consisting of oxygen and hydrogen peroxide.

In this method, reacting a glutathione-based compound with an oxidant and the platinum material comprises an oxidation reaction. Relative amounts of the reactants are preferably about 1 equivalent of the glutathione-based compound with less than about 1 equivalent of the oxidant such as hydrogen peroxide, and more preferably, with about 0.9 equivalent of the hydrogen peroxide. In another embodiment, the oxidation reaction comprises reacting about 1 equivalent of the glutathione-based compound with between about 0.0003 equivalent and about 1 equivalent of the platinum material, preferably between about 0.001 equivalent and about 1 equivalent of platinum material, more preferably between about 0.001 equivalent and about 0.1 equivalent, and even more preferably between about 0.001 equivalent and 0.01 equivalent, in the presence of less than 1 equivalent of the oxidant. In another embodiment, about 1 equivalent of the glutathione-based compound is reacted with about I equivalent of the platinum material in the presence of less than 1 equivalent of the oxidant.

In one embodiment, the method involves oxidizing the glutathione-based compound with about 0.9 equivalent of hydrogen peroxide and about 0.001 equivalent of cis-platin. One advantageous feature of this method is an increased rate of oxidation of the glutathione-based compound. Another advantageous feature of this method is that the yield of the resulting composite is increased to an amount greater than about 98% and this increased yield is accompanied by an increased purity. The purification of the composite is simplified to a significant degree in that liquid chromatography can be performed to obtain a purity of the composite of greater than 99%, which complies with pharmaceutical standards. Prior art methods have achieved a purity of only 75-93% of oxidized glutathione, depending on the method.

In a preferred embodiment, the composite is synthesized in one step by oxidizing the reduced glutathione in presence of cis-diamminedichloroplatinum, which may function as an oxidation reaction catalyst. The reaction conditions can be regulated accurately by using less than 1 equivalent of hydrogen peroxide. Consequently, formation of superoxidation products can be reduced, resulting in a near quantitative yield of the product. Thus, the one-step composite synthesis provides significant technological simplification and production of the composite GSSG•Pt with the stabilized disulfide bond.

In a preferred embodiment, the reaction is performed in a solution involving reduced glutathione as a monosodium salt and adding at room temperature with stirring about 0.9 equivalent of the hydrogen peroxide and about 0.001 equivalent of the following complex water-soluble platinum or palladium compounds with the following composition:
a) Pt [(NH₃)₂]Cl₂ cis-diamminedichloroplatinum (II),
b) K₂[PtCl₄] potassium tetrachloroplatinate (II),
c) K₂[PdCl₄] potassium tetrachloropalladate (II),
that function as catalysts for the oxidation reaction of GSH molecules. The oxidation reaction typically proceeds in about 1.5.-2 hours. Control for the completeness of the oxidation process can be conducted by an HPLC assay. The process is completed by the reaction solution lyophilic drying to produce the composite consisting of the oxidized glutathione and cis-diamminedichloroplatinum in a mole ratio of 1000: (confirmed by spectral analysis on platinum and sodium). The peptide constituent of the obtained composite according to the data of an amino acid assay, a NMR (¹H) spectrum, retention time by HPLC corresponds to the hexapeptide, bis-(γ-L-glutamyl)-L-cysteinyl-bis-glycine. The admixtures content do not exceed 2%, and the product yield as a disodium salt is 96-98% calculating for the dry composite.

Another aspect of the invention provides a method of stimulating endogenous production of cytokines and hemopoietic factors comprising stages of introduction to a mammalian body in need of stimulation of cytokines or hemopoietic factors or both, an effective amount of a composite comprising an oxidized glutathione-based compound and a metal material in a ratio of between about 3000:1 to about 1:1 wherein the metal material comprises a metal selected from the group consisting of platinum and palladium.

Another aspect of the invention provides a method of enhancing and prolonging the ability of the oxidized glutathione-based compound to stimulate endogenous production of cytokine and hemopoietic factors. The said method comprises stages of interaction of the oxidized glutathione-based compound with a metal material in a ratio of between about 3000:1 to about 1:1 wherein the metal material comprises a metal selected from the group consisting of platinum and palladium.

Moreover, another aspect of the invention provides a method for treating. a subject having a disease selected from the group consisting of oncological, infectious, ischemic or neurodegenerative ones. The method comprises administering to the subject in need of such treatment a composite comprising the oxidized glutathione-based compound and the metal material in a ratio of between about 3000:1 to about 1:1 in an amount effective to stimulate endogenous production of cytokines and or hemopoietic factors or both, to obtain a therapeutic effect. "Therapeutic effect" includes alleviation of the patient's state, prevention or curing of an unwanted body condition and can comprise a process selected from the group consisting of regulating proliferation in normal cells, regulating differentiation in normal cells, and inducing apoptosis in transformed ones where the transformed cells can include pathologically-altered ones, first of all, tumor-transformed and virus-transformed cells

In one embodiment, the oxidized glutathione-based compound has the general formula: 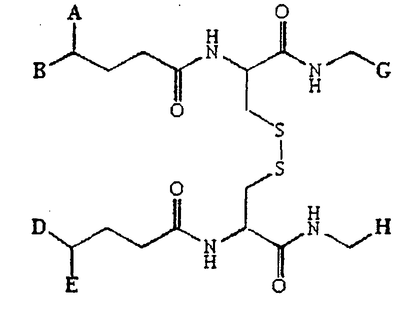 wherein A, B, D, E, G and H can each be selected from the group consisting of an organic unit and salts of the organic unit. Preferably, the "organic unit" allows the glutathione-based compound to remain soluble in biological media and in addition, the organic unit should not impart toxicity to the oxidized glutathione-based compound in an applied dosage. It is understood that A, B, D, E, G and H can be the same or different. Preferably, the groups A-H can each include a unit selected from the group consisting of amine groups; carboxyl groups, and amides. For example, A-H can represent amino acids or derivatives bonded via an amide bond. Alternatively, any two of A-H can be linked to each other by, at least, one covalent bond. Thus, A-H can be part of a cyclic structure.

In one embodiment, the composite comprises a large excess of the oxidized glutathione-based compound relative to the metal material, preferably in a ratio of between about 3000:1 and 1:1, more preferably in a ratio of between about 1000:1 and 1:1, more preferably in a ratio of between about 1000:1 and 10:1, even more preferably in a ratio of between about 1000:1 and 100:1. In another embodiment, the composite comprises equal amounts of the oxidized glutathione-based compound and the metal material, i.e., a ratio of about 1:1.

In one embodiment, the oxidized glutathione-based compound is oxidized glutathione itself (GSSG) and salts thereof, where both A and E are -CO₂H, both B and D are -NH₂ and both G and H are -CO₂M, M being a counterion. The counterion can be a proton, an organic-based ion such as tetralkyl-amononium, an alkaline metal, an alkaline earth metal, or a transition metal. It is understood that in aqueous media, any of A-H can comprise an ionized group, e.g., A and E can be --CO₂, and B and D can be -NH₂⁺ and the ionized groups are neutralized by an appropriate counterion.

The basic composite can be prepared by aforementioned methods, from interaction of a metal material with reduced glutathione in presence of an oxidant.

These compounds and the pharmaceutically acceptable drug forms thereof obtained, which include the GSSG•Pt material composite are applied as medicinal drugs capable in the therapeutic purposes depending on the initial subject's biological status of the subject in need thereof to stimulate/modulate the wide range cytokine and hemopoietic factor endogenous production, and/or to reproduce the cytokine effects as well as to perform the differentiated effect regarding the normal (the metabolism, proliferation and differentiation regulation) and the transformed cells (the apoptosis mechanism induction). "Transformed cells" refers to tumor- and/or virus-transformed cells.

Therapeutic effects of GSSG•Pt material and pharmaceutically acceptable derivatives thereof, particularly, salts thereof for the treating of oncological, infectious diseases including viral ones can be explained as a stimulation of the wide-ranged endogenous cytokine production with a unique ability to activate apoptotic death of the transformed cells exclusively.

Performed experimental and clinical investigations show that therapeutic effects of the drugs obtained from the GSSG•Pt material and derivatives thereof are based on the multicytokineactivating action and capacity to reproduce cytokine and hemopoietic factor effects that allows to consider as expedient their usage in treatment of various diseases.

According to the invention the medicinal agent having maximal affinity to particular organs/tissues and purposeful biological-pharmacological effects providing regulation of processes of metabolism, proliferation and differentiation in the normal cells and induction of the apoptosis mechanisms in the tumor- and/or viral-transformed ones comprises the aforementioned composite, GSSG•Pt, i.e. the hexapeptide with the stabilized disulfide bond, as an active principle.

According to the invention the medicinal agent is designated for modulation of the endogenous production of cytokines and hemopoietic factors, and/or reproduction of the cytokine effects, thus, providing regulation of processes of metabolism, proliferation, differentiation and apoptosis.

According to the invention the medicinal agent is designated for treatment of oncological, infectious, immunological, hematological, ischemical, neurodegenerative, metabolic disorders and endocrine diseases.

According to the invention the preferred medicinal agent designated for treatment of lung cancer consists of the composite comprising GSSG•Pt.

According to the invention the preferred medicinal agent designated for treatment of melanoma consists of the composite comprising bis-[3-iodine-tyrosyl]-GSSG•Pt.

According to the invention the preferred medicinal agent designated for treatment of cerebral tumors consists of the composite comprising bis-[dopamine]-GSSG•Pt.

According to the invention the preferred medicinal agent designated for treatment of colorectal cancer consists of the composite comprising bis-[cysteamine]-GSSG•Pt.

According to the invention the preferred medicinal agent designated for treatment of breast cancer consists of the composite comprising cysteamine-GSSG•Pt.

According to the invention the preferred medicinal agent designated for treatment of prostate cancer consists of the composite comprising dizinc salts of GSSG•Pt.

According to the invention the preferred medicinal agent designated for treatment of ovarian cancer consists of the composite comprising theophylline-GSSG• Pt.

According to the invention the preferred medicinal agent designated for treatment of acute lymphoblastic leukosis consists of the composite comprising lithium salt of GSSG•Pt.

According to the invention the preferred medicinal agent designated for treatment of acute myeloblastic leukosis consists of the composite selected from the group consisting of lithium salt of GSSG•Pt and cysteamine-GSSG•Pt and combinations thereof.

According to the invention the preferred medicinal agent designated for treatment of tuberculosis consists of the composite comprising bis-[histidyl]-GSSG• Pt.

According to the invention the preferred medicinal agent designated for treatment of diseases selected from the group consisting of viral hepatitis B, viral hepatitis C, and mixed-infections thereof consists of the composite selected from the group consisting of GSSG•Pt and inosine-5-monophosphatyl-GSSG•Pt.

According to the invention the preferred medicinal agent designated for treatment of herpes consists of the composite selected from the group consisting of GSSG•Pt and inosine-5-monophosphatyl-GSSG•Pt.

According to the invention the preferred medicinal agent designated for treatment of diseases selected from the group consisting of meningitis, sepsis consists of the composite comprising tetra-dopamine-GSSG•Pt.

According to the invention the preferred medicinal agent designated for treatment of peritonitis consists of the composite selected from the group consisting of GSSG•Pt and tetra-dopamine-GSSG•Pt and combinations thereof.

According to the invention the preferred medicinal agent designated for treatment of extra hazardous infections of viral origin, in particular, Rift Valley fever, and/or bacterial origin, in particular, tularemia consists of the composite selected from the group consisting of somatic antigen-GSSG•Pt and/or antibody(against somatic antigen)-GSSG•Pt.

According to the invention the preferred medicinal agent designated for treatment of acute pancreatitis consists of the composite selected from the group consisting of GSSG•Pt and inosine-5-monophosphatyl-GSSG•Pt and combinations thereof.

According to the invention the preferred medicinal agent designated for treatment of suppurative post-surgery sequalae consists of the composite selected from the group consisting of GSSG•Pt and inosine-5-monophosphatyl-GSSG•Pt and combinations thereof.

According to the invention the preferred medicinal agent designated for treatment of AIDS consists of the composite selected from the group consisting of GSSG•Pt and uridine-[5-monophosphatyl]-GSSG•Pt and combinations thereof.

According to the invention the preferred medicinal agent designated for treatment of immunosuppressions of infectious origin consists of the composite selected from the group consisting of GSSG•Pt and uridine-[5-monophosphatyl]-GSSG•Pt and combinations thereof.

According to the invention the preferred medicinal agent designated for treatment of glomerulonephritis consists of the composite selected from the group consisting of GSSG•Pt and lithium salt of GSSG•Pt and combinations thereof.

According to the invention the preferred medicinal agent designated for treatment of rheumatoid arthritis consists of the composite selected from the group consisting of GSSG•Pt and lithium salt of GSSG•Pt and combinations thereof.

According to the invention the preferred medicinal agent designated for treatment of coliagenosis consists of the composite selected from the group consisting of GSSG•Pt and a lithium salt of GSSG•Pt and combinations thereof.

According to the invention the preferred medicinal agent designated for treatment of systemic lupus erythematosus consists of the composite selected from the group consisting of GSSG•Pt and lithium salt of GSSG•Pt and combinations thereof.

According to the invention the preferred medicinal agent designated for treatment of an atopic form of an allergic condition consists of the composite selected from the group consisting of GSSG•Pt and dihydrofluoride-GSSG•Pt and combinations thereof.

According to the invention the preferred medicinal agent designated for treatment of diabetes-type I consists of the composite comprising vanadium salt of GSSG•Pt.

According to the invention the preferred medicinal agent designated for treatment of diabetes-type II consists of the composite comprising bis-[lipoyl]-GSSG• Pt.

According to the invention the preferred medicinal agent designated for treatment of an ischemic cerebral condition consists of the composite comprising bis-[phenylalanyl]-GSSG•Pt.

According to the invention the preferred medicinal agent designated for treatment of an ischemic heart disease consists of the composite comprising bis-[carnosyl]-GSSG•Pt ([β-alanyl-L-hystidyl]-GSSG•Pt).

According to the invention the preferred medicinal agent designated for treatment of an ischemic heart disease manifested mainly as a syndrome of functional myocardial failure consists of the composite comprising glycerol-[ 1,3-diphosphatyl]-GSSG•Pt.

According to the invention the preferred medicinal agent designated for treatment of a neurodegenerative disease consists of the composite comprising bis-[3,4-dihydroxyphenylalanyl]-GSSG•Pt.

According to the invention the preferred medicinal agent designated for treatment of a demyelinating disease consists of the composite comprising bis-[3,4-dihydroxyphenylalanyl]-GSSG•Pt.

According to the invention the preferred medicinal agent designated for treatment of cerebral hypoxia consists of the composite comprising gamma-hydroxy-[butanoyl]-GSSG•Pt.

According to the invention the preferred medicinal agent designated for treatment of manic-depressive psychosis consists of the composite comprising gamma-amino-[butanoyl]-GSSG•Pt.

According to the invention the preferred medicinal agent designated for treatment of a disease manifested with metabolic disorders in balance of vessel atherosclerosis-forming lipoproteins consists of the composite comprising bis-[nicotinoyl]-GSSG•Pt.

According to the invention the preferred medicinal agent designated for treatment of an endocrinal disease caused with alteration of hypothalamic-hypophysial-ovarian system consists of the composite comprising folliculyl-[succinyl]-GSSG•Pt.

The invention will be illustrated with the accompanying drawings, which are schematic and which are not intended to be drawn to scale. In the figures, each identical or nearly identical component that is illustrated in various figures is represented by a single numeral. For purposes of clarity, not every component is labeled in every figure, nor is every component of each embodiment of the invention shown where illustration is not necessary to allow those of ordinary skill in the art to understand the invention.

### Brief Description of the Drawings

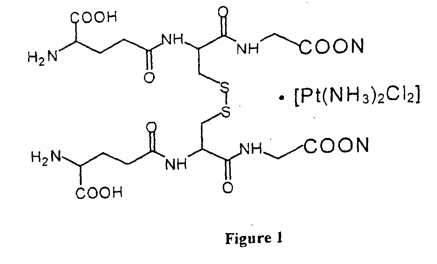

Fig. 1 shows the structure of bis-(γ-L-glutamyl)-L-cysteinyl-bis-glycine disodium salt with cis-diamminedichloroplatinum;
Fig. 2 shows the synthesis scheme of the composite of the oxidized glutathione disodium salt with cis-diamminedichloroplatinum;
Fig. 3 shows the donor-acceptor bond among platinum atom and two NH₃ groups due to the lone-pair electrons of nitrogen atoms;
Fig. 4 shows proposed mechanisms for the GSSG molecule disulfide bond stabilization due to the ligand exchange - NH₃-groups on disulfide bonds - and through 7forming of the donor-acceptor bond among platinum atom and two sulfur atoms due to lone-pair electrons of sulfur atoms;
Fig. 5 shows proposed mechanisms of the GSSG molecule stabilization through the mechanism given at Fig.4 as well as through exchange of the NH₃ ligands on NH₂ groups of the glutathione (the NH₂ group convergence and the GS fragments, correspondingly) forming new "biophysics" of the GSSG•Pt composite, with squares (□) denoting donor sites and circles (○) denoting acceptor sites;
Fig. 6 shows the main sites (encircled) for the GSSG•Pt molecule chemical modification;
Fig. 7 shows the structure of bis-phenylalanyl-GSSG•Pt;
Fig. 8 shows the synthesis scheme for bis-(L-phenylalanyl-γ-L-glutamyl)-L-cysteinyl-bis-glycine with cis-diamminedichloroplatinum;
Fig. 9 shows the structure of lithium salt of bis-(γ-L-glutamyl)-L-cystinyl-bis-glycine;
Fig. 10 shows the stage of processing of the source product, namely, reduced glutathione with hydrogen peroxide at pH=8 (stage of the synthesis scheme for the GSSG•Pt lithium salt);
Fig. 11 shows the extraction of the free hexapeptide bis-(γ-L-glutamyl)-L-cystinyl-bis-glycine (III, stage of the synthesis scheme for the GSSG•Pt lithium salt);
Fig. 12 shows the transfer of the oxidized GSSG (III) form in the lithium salt of GSSG•Pt;
Fig. 13 shows the DNA degradation character of the normal cells at the control group (1), after treatment with: GSSG (2), GSSG•Pt (3) incubation time - 48 hrs;
Fig. 14 shows the DNA degradation character of the HL-60 cells at the control group (2), after treatment with: GSSG (1), GSSG•Pt (3) incubation time - 48 hrs.
Fig. 15(a) shows the structure of S-thioethylamine•glutathione disulfide;
Fig. 15(b) shows the structure of *bis*-[DL-6,8-thioetic acid]•glutathione disulfide;
Fig. 15(c) shows the structure of [β-alanyl-L-histidyl]•glutathione disulfide;
Fig. 15(d) shows the structure of [9-β-D-ribofuranosyladenyl]•glutathione disulfide;
Fig. 15(e) shows the structure of *bis*-[L-2-amino-4-[methylthio]butanoic acid]•glutathione disulfide.
Fig. 16(a) shows the structure of *bis*-[methionyl]•glutathione disulfide;
Fig. 16(b) shows the structure of *bis*-[aspartyl]•glutathione disulfide;
Fig. 16(c) shows the structure of *bis*-[histidyl]•glutathione disulfide;
Fig. 16(d) shows the structure of *bis*-[3-iodine-tyrosyl]•glutathione disulfide;
Fig. 16(e) shows the structure of *bis*-[γ-aminobutanoyl]•glutathione disulfide;
Fig. 16(f) shows the structure of *bis*-[γ-hydroxybutanoyl]•glutathione disulfide;
Fig. 16(g) shows the structure of *bis*-[3,4-dihydroxyphenylalaninyl]•glutathione disulfide
Fig. 17(a) shows the structure of bis-nicotinoyl-glutathione disulfide (*bis*-[pyridine-3-carbonyl]•glutathione disulfide);
Fig. 17(b) shows the structure of uridine-5'-monophosphatyl•glutathione disulfide;
Fig. 17(c) shows the structure of inosine-5'-monophosphatyl•glutathione disulfide;
Fig. 17(d) shows the structure of folliculylsuccinyl•glutathione disulfide;
Fig. 17(e) shows the structure of glycerol-1,3-diphosphatyl•glutathione disulfide
Fig. 18(a) shows the structure of tetra-dopamine•glutathione disulfide;
Fig. 18(b) shows the structure of theophylline•glutathione disulfide;
Fig. 19(a) shows the structure of bis-carnosyl•glutathione disulfide (bis-[β-alanyl-L-hystidyl]-GSSG•Pt);
Fig. 20(a) shows the structure of a non-symmetric mixed disulfide compound;
Fig. 20(b) shows the structure of a symmetric mixed disulfide compound;
Fig. 20(c) shows the structure of a symmetric and doubly bridged mixed disulfide compound
Fig. 21(a) shows the structure of divanadate salts;
Fig. 21(b) shows the structure of dihydrofluoride salts;
Fig. 21(c) shows the structure of dilithium salts;
Fig. 21(d) shows the structure of zinc salts.
Fig. 22 shows the structure of the polipeptide [antigen or antibody]•glutathione disulfide.

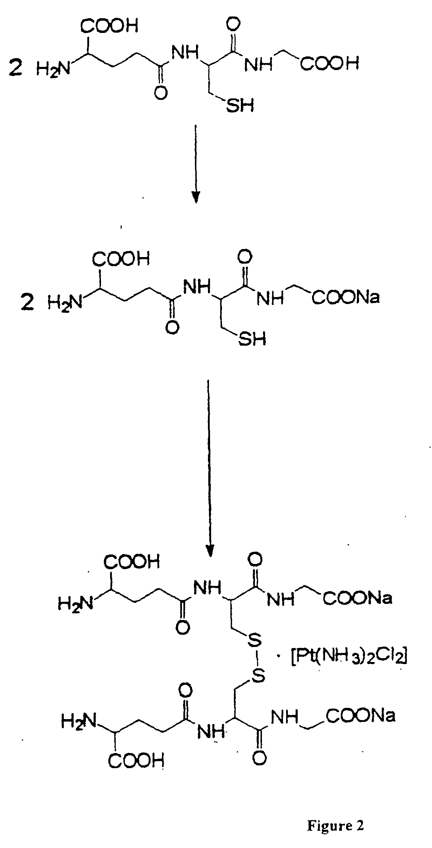

The composite synthesis scheme is provided in Fig.2.

The GSSG•Pt composite obtaining provides two directions of new effects for the given compound that is a structural analogue of the oxidized glutathione, i.e. the hexapeptide with the stabilized disulfide bond according to the chemical formula thereof.

First - stabilization of the disulfide bond with the platinum compounds significantly enhances the lifetime of GSSG•Pt in biological media in the oxidized form considerably extending the biological-pharmacological effects specific for GSSG. Besides, an interaction between the platinum material and the oxidized glutathione (GSSG) molecule provides possibility for ligand exchange, i.e., instead of the NH₃ groups, two sulphur atoms possessing two pairs of the lone-pair electrons can be involved in donor/acceptor bonds with the platinum atom. One can also consider the possibility for the addition in regard to the aforesaid stabilization of the disulfide bond due to the convergence of the NH₂ groups of the oxidized glutathione-based compound and stabilization of the general GSSG conformation (Fig. 5).

Second - presence of the platinum compounds within the composite in the similar significant way increases capacities of GSSG•Pt for chemical modification and obtaining a range of the derivatives thereof as new compounds (new substance formulas) based on the covalent bond between GSSG•Pt and another chemical (biochemical) compound. At that, Pt within a composite is a catalyst of formation of the covalent bond between GSSG•Pt and another chemical/biochemical component.

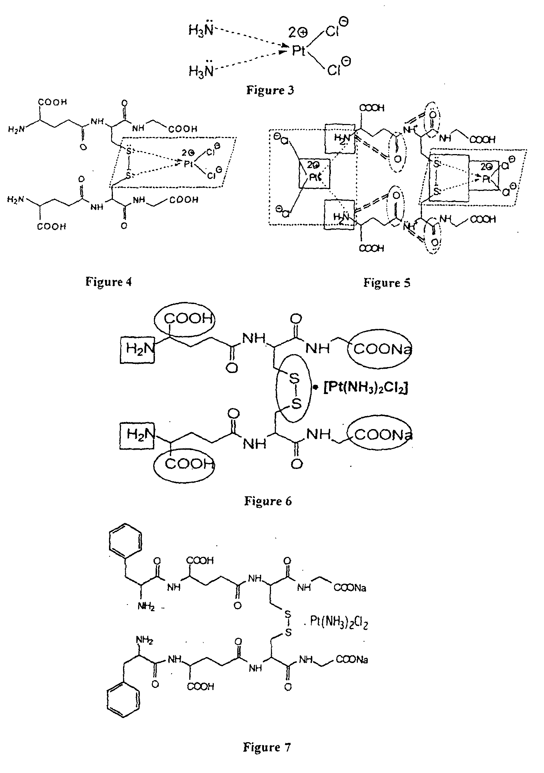

Through chemical modifications of the basic GSSG•Pt molecule, it is possible to create new drugs where, in molecules along with the basic structure that have already demonstrated high medical-biological activity based on the oxidized glutathione composite with cis-diamminedichloroplatinum, there are fragments of other covalently-bound biochemically active molecules. Usage of the covalently bound combinations allows enhancing of a range of important drug characteristics such as stability and standardized properties of composition. Foundation for the chemical modifications is the GSSG•Pt hexapeptide core with two primary glutamic acid aminogroups, cystine disulfide bond and carboxyl groups of glycine and α-carboxyl groups of glutamic acid (FIG. 6).

Purposely elected covalently-bound fragments of biochemically significant molecules can considerably improve medical-biological features of the basic GSSG•Pt material composite making them more selective for each particular therapeutic purpose, resulting in a sharp increase in a desirable treatment course. It might be conditioned with an additive effect of fragments in the biochemical activity mechanisms, sharp improvement of the drug molecule transport to a target-cell or a target-molecule, enhanced affinity for a receptor, necessary redistribution of oxidative-reductive (redox) potential and a range of other factors as well as combination thereof. Thus, the matter in hand is acquirement of a range of new chemical compounds with predetermined properties.

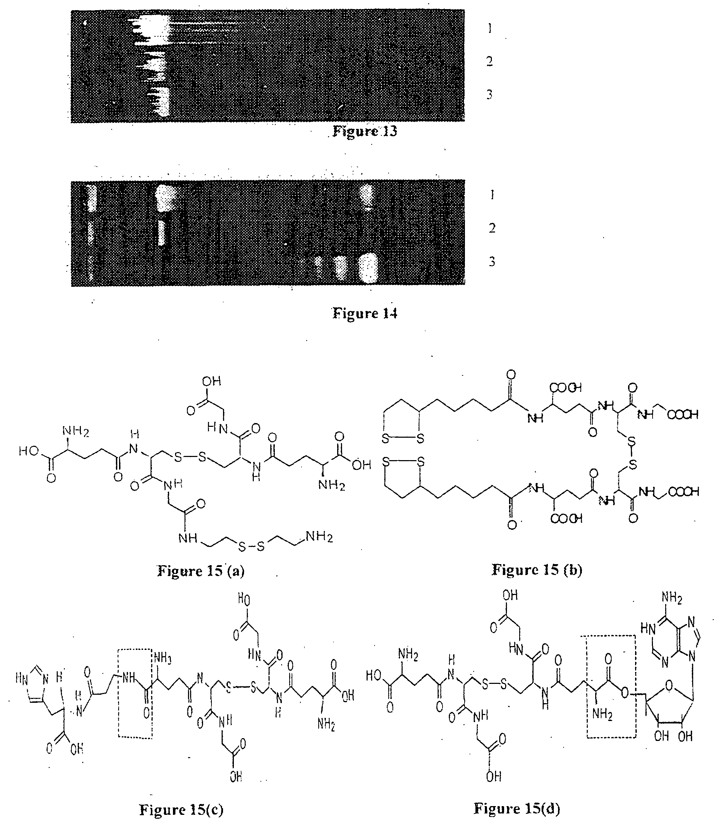

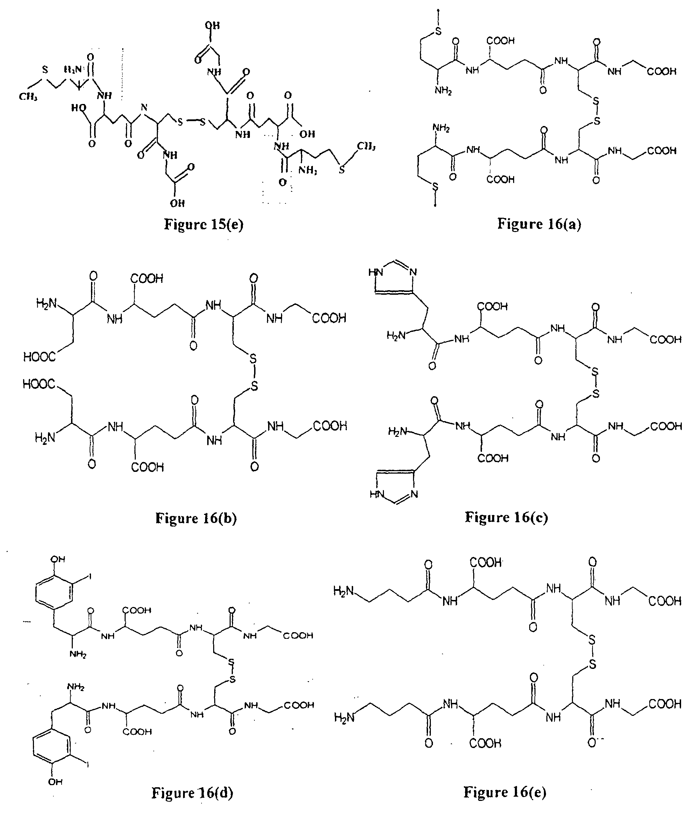

In one embodiment, the oxidized glutathione-based compound can be a derivative of glutathione and cysteamine. The glutathione can be derivatized after preparation of the composite, or it can be derivatized prior to preparation of the composite, i.e., the GSH can be derivatized prior to oxidation into the dimer. Fig. 15 (a, b, c, d) depicts various examples of different glutathione-based compounds with following obtaining of the oxidized glutathione-based compound.

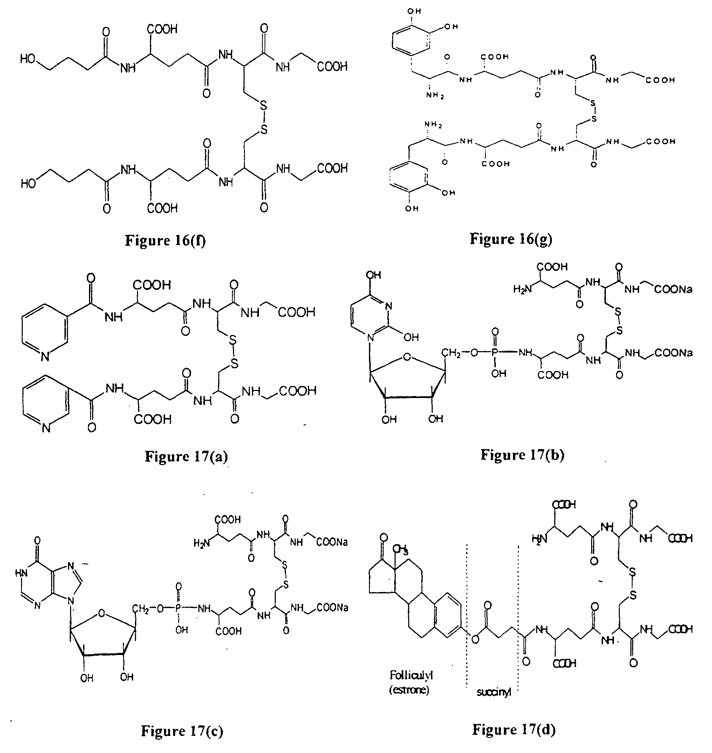

In one embodiment, the oxidized glutathione-based compound has an acylated primary glutamic acid amino group. This variant is most suitable for acylation by N-protected activated aminoacid derivatives, where after the stage of temporary protection of the cysteine mercapto-groups followed by condensation (activated ester method), and removal of the N- and S-protective groups and oxidation by the hydrogen peroxide with addition of cis-diamminedichloroplatinum results in the GSSG•Pt composite modified by amino-acids at the glutamic acid aminogroups. In this embodiment the oxidized glutathione-based compound can be selected from the group consisting of *bis*-[methionyl]•glutathione disulfide (Fig. 16a), *bis*-[aspartyl]•glutathione disulfide (Fig. 16b), *bis*-[histidyl]•glutathione disulfide (Fig. 16c), *bis*-[3-iodine-tyrosyl]•glutathione disulfide (Fig. 16d), [γ-aminobutanoyl]•glutathione disulfide (Fig. 16e), *bis*-[γ-hydroxybutanoyl]•glutathione disulfide (Fig. 16f), *bis*-[DL-6,8-thioetic acid]•glutathione disulfide (Fig. 15b), and *bis*-[3,4-dihydroxyphenylalaninyl]•glutathione disulfide (Fig. 16g).

When the composite includes phenylalanyl groups, the compound is bis-phenylalanyl-GSSG•Pt (see Fig. 7).

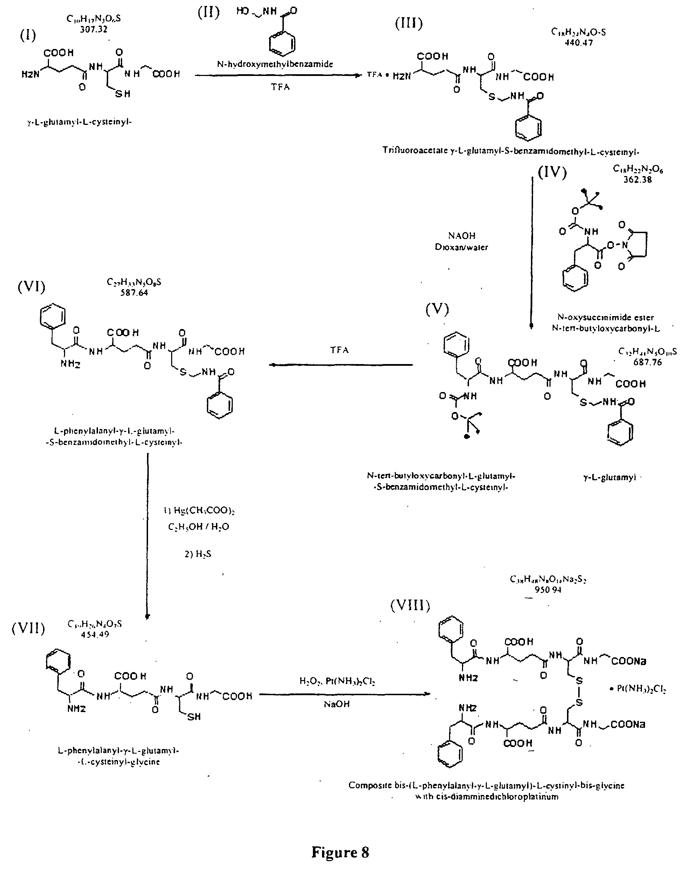

According to this basic scheme, all other GSSG•Pt modifications based on the aminogroups acylation by the derivatives of the protected aminoacids, oxy-acids, carbonic acids and derivatives thereof are synthesized. Slight changes of the methods are possible due to a specific nature of modifying molecule. Protecting groups for the initial GSH compound include Bam (N-hydroxymethylbenzamide). The amino acid (AA) can be protected with groups such as BOC (butyl carbamate) or O-Su (N-oxysuccinimide ester; see Fig. 8 for specific details).

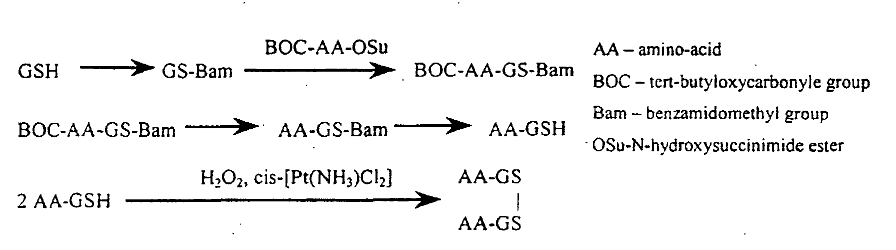

When the composite is bis-methionyl-GSSG•Pt, i.e., (Met)₂GSSG•Pt, incorporation of a methionine group may involve BOC-protective group deblocking after the condensation stage.

When the composite is bis-aspartyl-GSSG•Pt, an aspartic acid group can be introduced according to the general aforementioned scheme. A temporary protective group for the β-carboxyl group is preferably removed, if possible, with the BOC-protective group simultaneously if needed. A β-tert-butyl ester of N-BOC-aspartic acid, i.e., BOC-Asp(OBu^{t})-OSu, can be applied at the condensation stage. The protective groups can be removed by trifluoroacetic acid (TFA).

When the composite is bis-histidyl-GSSG•Pt, a histidine imidazole ring can be protected via a di-tert-butyl-oxycarbonyl histidine derivative, i.e., BOC-His(BOC)-OSu, which can be used at the condensation stage. As in previous cases, the protective groups can be removed by trifluoroacetic acid.

When the composite is bis-3-iodine-tyrosyl-GSSG•Pt, a possible protecting group is an acid-labile protective group such as tert-butyl ester. The tyrosine derivative used at the concentration stage can be BOC-Tyr(OBu^{t})-OSu.

When the composite is GABA-GSSG•Pt, (γ-aminobutanoyl-GSSG•Pt), an acid-labile protective tert-butyloxycarbonyl (BOC) group can be used at the condensation stage.

When the composite is GOBA-GSSG•Pt, (γ-hydroxyhutanoyl-GSSG•Pt), an acid-labile group tert-butyl ester (which can be removed by a trifluoroacetic acid solution) can be used to protect a GOBA hydroxyl group. A derivative, used at the condensation stage, can be GABA(OBu^{t})-OSu.

When the composite is bis-lipoyl-GSSG•Pt, it is believed that side functional groups of lipoic acid do not require special protection. At the condensation stage it is possible to apply an activated (hydroxysuccinimide) ester of lipoic acid. There may be no need for a TFA treatment.

When the composite is bis-3,4-dihydrooxyphenylalanyl-GSSG•Pt (bis-DOPA-GSSG•Pt), to introduce DOPA molecule, it may be necessary to previously protect two hydroxyl groups of 3,4-dihydroxyphenylalanine by tert-butyl esters and to protect the aminogroup by a BOC-protective group. For condensation with a composite precursor, an activated ester can be obtained (hydroxysuccinimide or pentafluorophenyl one) that is used in excessive mole amount. Removal of the protecting groups can occur simultaneously with a trifluoroacetic acid solution.

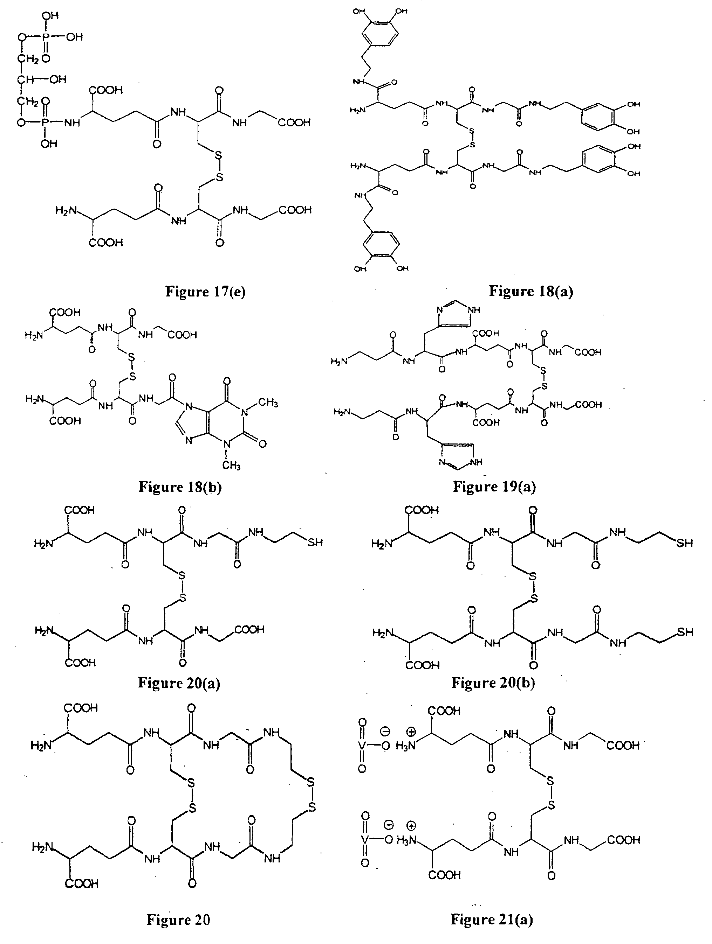

When the composite is bis-[camosyl]-GSSG□Pt (bis-β-alanyl-L-histidyl-GSSG•Pt), camosine can be protected as a di-BOC derivative of the β-alanine aminogroup and histidine imidazole group before condensation. Then condensation and following deblocking can be performed as previously described. A structure of bis-[carnosyl]-GSSG can be found in Fig.19a.

In another embodiment, the oxidized glutathione-based compound has an amide or phosphoramide bond to a unit selected from the group consisting of heterocyclic carbonic acids and nucleotides. In this embodiment, examples of the oxidized glutathione-based compound include bis-nicotinoyl-glutathione disulfide *(bis*-[pyridine-3-carbonyl]•glutathione disulfide) (Fig. 17a), uridine-5'-monophosphatoyl•glutathione disulfide (Fig. 17b), inosine-5'-monophosphatoyl•glutathione disulfide (Fig. 17c), folliculylsuccinyl•glutathione disulfide (Fig. 17d) and glycerol-1,3-diphosphatyl•glutathione disulfide (Fig. 17e).

When the composite is bis-nicotinoyl-GSSG•Pt (bis-pyridine-3-carbonoyl-GSSG•Pt), a nicotinic acid containing no side functional groups can be introduced into condensation with a composite precursor without obtaining protected derivatives as corresponding activated esters such as hydroxysuccinimide or pentafluorophenyl. TFA treatment for the removal of protecting groups may not be required.

When the composite is uridine-5'-monophosphatoyl-GSSG•Pt (UMP-5'-GSSG•Pt), uridine-5'-monophosphate in presence of N,N-dicyclohexyl-carbodiimide can form phosphoamide links in reactions with amides [10]. The composite precursor can have protected carboxyl groups, such as tetratrimethylsilyl derivatives to be used as an aminocomponent. Deblocking can proceed in mild water-alcohol systems.

When the composite is ionosine-5'-monophosphatoyl-GSSG•Pt, IMP-5'-GSSG•Pt, the synthetic scheme would most likely be similar to that of the previous derivative (UMP-5'-GSSG).

When the composite is folliculylsuccinyl-GSSG•Pt, a link between GSSG•Pt and estrone can be made by amide and ester bonds through a succinyl residue. Estrone can be transformed into an activated derivative by reaction with succinanhydride with following condensation by N,N-dicyclohexylcarbodiimide with a protected or blocked composite precursor and with tetra-trimethylsilyl derivatives as well. Deblocking can be performed in a water-alcohol system.

When the composite is glycerol-1,3-diphosphatyl-GSSG•Pt, the modification can proceed by a carbodiimide method using a composite precursor protected, as a tetra-trimethylsilyl derivative as an amino component, the synthesis is similar to that in the synthesis of phosphoamide derivatives (See Examples 13 and 14).

In another embodiment, the oxidized glutathione-based compound can be selected from the group consisting of tetra-dopamine•glutathione disulfide (Fig. 18a) and theophylline•glutathione disulfide (Fig. 18b). The formation of amide links can occur between composite carboxyl groups and amides. The reactivity of all four carboxyl groups is very similar and, therefore, a mixture of products can result.

When the composite is tetra-dopamine-GSSG•Pt, a 3,4-di-tert-butyl ester of dopamine can be used as an aminocomponent and di-tert-butyloxycarbonyl derivatives the composite can be used as a carboxyl component. Condensation can proceed by a N,N-dicyclohexyl-carbodiimide, and removal of the protective groups can be performed with a trifluoroacetic acid solution.

When the composite is GSSG•Pt-theophylline, theophylline can be used as an aminocomponent, a composite precursor can be used for a carboxyl component as a di-tert-butyloxicarbonyl derivative. Condensation can be performed with an "F" complex. The removal of protective group can be performed with a trifluoroacetic acid solution.

In another embodiment, the oxidized glutathione-based compound can include mixed disulfides. Possible combined structures can involve mixed disulfide formation (symmetric and non-symmetric). (See Fig. 20a-20c)

One structure (Fig. 20b) can be formed via mutual oxidation of mercaptogroups starting materials. There may be no need for additional protective groups and condensation methods.

Another compound (Fig. 20a) can be obtained by formation of an amide bond between the cysteamine aminogroup and one of the composite precursor carboxyl groups. It may be necessary to introduce N-protective groups and to activate the composite precursor carboxyl groups. Due to the presence of four carboxyl groups it may be necessary to manipulate the stoichiometry and/or perform chromatographic separation of the resulting products.

The synthesis conditions are different from the structure 20b by presence of the additional aminocomponent equivalent. At the chromatographic purification the structure 20b is used a witness.

It is obtained from the structure 20c through formation of an additional disulfide bond at the mercapto-group reaction. During the chromatographic separation of products, it may be necessary to have the structure 20c as a witness. _

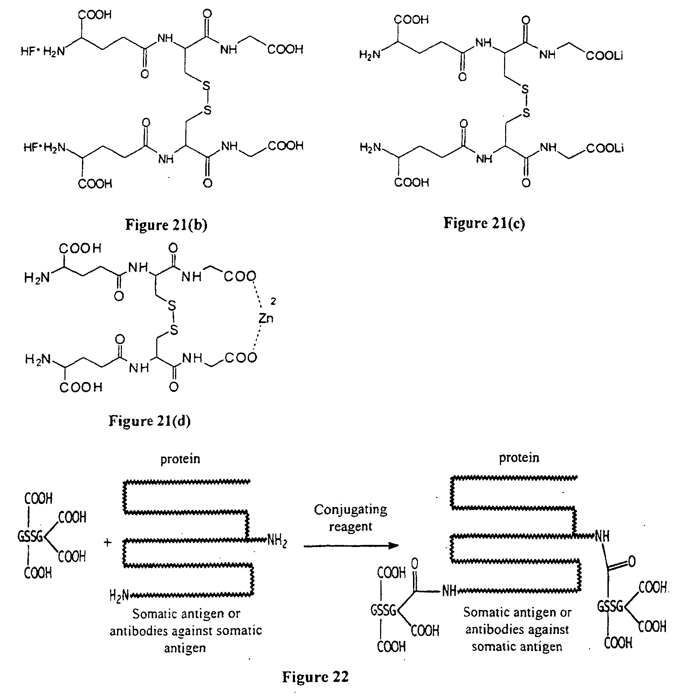

In another embodiment, the oxidized glutathione-based compound can be a salt selected from the group consisting of alkali metal salts, alkaline earth metal salts, and transition metal salts. Examples of such salts include divanadate salts (Fig. 21a), dihydrofluoride salts (Fig. 21b), dilithium salts (Fig. 21c), didopammonium salts, and dizinc salts (Fig. 21d).

The salts can be obtained through addition of the corresponding amount of the salt-forming components, a base or an acid. Examples of salts with aminogroups includes a divanadate of GSSG•Pt ((HVO₃)₂-GSSG•Pt) or a dihydrofluoride of GSSG•Pt ((HF)₂•GSSG•Pt). Examples of salts with carboxyl groups include a dilithium salt GSSG•Pt (see Example 2), a GSSG•Pt didopammonium salt or GSSG•Pt zinc salt.

In another aspect of the invention, a drug comprising the composite such as the hexapeptide bis-(γ-L-glutamyl)-L-cystinyl-bis-glycine disodium salt and cis-diamminedichloroplatinum (cis-platinum) is obtained according to the previously described method.

Other preferable derivatives include the GSSG•Pt material derivatives in the form of its sodium, lithium, potassium, calcium, zinc, molybdenum, vanadium and other salts, as well as the GSSG•Pt derivatives obtained through covalent binding to phenylalanine, or to methionine and some other aminoacids including D and L forms of the aminoacids herein; or to cysteamine, lipoic acid, or to inosine.

In one embodiment, manifestation of the immunological, biochemical and molecular-biological effects of the GSSG•Pt therapeutical impact can be obtained in the case when a combination comprising 50% of GSSG•Pt with all aminoacids in L-form and 50% of GSSG•Pt with two chemically equal aminoacids being represented in D-form and others being represented in L-form is used.

In one embodiment, there is formed a polypeptide compound based on formation of covalent bond between GSSG•Pt and a protein molecule (substance of polypeptide origin). It is of importance that as the protein substance there is used either an antigen obtained from microorganisms or an antibody obtained after immunization of animal body with the antigen. At the Fig.22 there is an example of a compound of GSSG•Pt with a protein substance based on formation of a peptide bond CO-NH between the carboxyl groups of one substance and amino-groups of the other. The protein molecule conjugates with the GSSG•Pt molecule can be obtained through formation of the peptide (amide) bonds between one of the GSSG•Pt carboxyl groups and α- or ε- NH₂ groups of the protein. The water-soluble carbodiimide or glutaraldehyde can be used as a reagent for conjugation. The target product can be purified through dialysis or gel-chromatography.

The new GSSG•Pt pharmacokinetics (comparing to GSSG by itself) in blood and tissues (organs) being introduced into biological media indicates that the GSSG•Pt molecule is much less available for the GSSG metabolism enzymes and, first of all, for the NADP•H⁺-dependent reductase, the main enzyme for the GSSG into GSH reduction. Thereupon, the GSSG•Pt half-life time in the disulfide form in biological media increases significantly (See Tables 5, 6 and 7 and Examples 3 and 4).

Basically novel pharmacokinetics of the hexapeptide with the stabilized disulfide bond (GSSG•Pt) compared to the structural analog, i.e., the oxidized glutathione (GSSG), provided an optimal manifestation for the newly determined the following biological-pharmacological effects:
- Stimulation/modulation of the endogenous production for a significantly large range of the cytokine, growth and hemopoietic factors in conditions of radiation and chemical immunosuppression (IL-1α and γ, IL-2, IL-3, IL-4, IL-6, IL-8, IL-10 and IL-12, TNF-α, IFN-α and IFN-γ, erythropoietin, colony-stimulating factors) (See Examples 5-7);
- Reproduction of some cytokine effects (IL-2, IL-12, IFN-α and IFN-γ) due to the mechanism induction for the redox-sensitive expression of the immunologically significant genes and the key protein "critical" cysteine modification for the cellular signal-transducing systems (See Example 8);
- Modulation on activity of main enzymes that form redox-contour in cells, especially, immune ones, neurons as well as in liver and renal ones;
- Restoration of the depressed bone-marrow haemopoiesis including erythrocyte, leukocyte and platelet counts as well as levels of CD3⁺, CD4⁺, CD8⁺, CD16/56⁺, CD19/20⁺, CD25⁺, CD34⁺, CD95⁺ (See Examples 9-13) at patients receiving radiation and high-dose combined chemotherapy;
- Hepatotropic effects as well as diminution of the cardio-, hephro- and neurotoxicity signs (in conditions of an active antibacterial, antiviral and antitumor chemotherapy);
- Differentiated, impact with regard to normal cells (including the ones being under functional stress) and transformed ones, namely, the metabolism, proliferation and differentiation stimulation at the normal cells/tissues and, simultaneously, capacity to induce the apoptosis mechanism only in the tumor- and/or virus-transformed cells.

Another advantageous feature of the drugs of the present invention is a correcting influence of the GSSG•Pt material and the salts thereof on the metabolic abnormalities, particularly, on the impairment of carbohydrate metabolism at diabetes, type II. In this case (See Example 7) restoration of the normal cAMP/cGMP ratio as well as the thiol-disulfide ratio in tissues due to the GSSG•Pt material impact (vanadium salt thereof) provided stable setting to normal values for the glucose content in the patient blood, which is a considerable therapeutic effect.

The method for the production of the composite makes possible synthesis of different composites as a basis for design of drugs possessing a range of new features, namely:
- Increased biochemical drug stability, i.e., "non-assailability" by the GSSG metabolism enzymes (i.e., far less accessible for these enzyme action), first of all, by NADP•H⁺-dependent glutathione reductase that basically increases the drug half-life time in the biological media exactly in the disulfide form;
- New biophysical component with high level of the donor-acceptor potential;
- Presence of new reactive sites within the said molecule and, therefore, entirely new capacity for chemical modification.

The composite properties allow it to function as a unique cellular "gyroscope" that in conditions of the extreme external environmental factors (physical, chemical and biological ones) provides restoration of a balance:
- Within the cytokine profile, i.e., the cytokines regulating proliferation mainly; and the cytokines regulating mainly differentiation of the immunocompetent cells;
- Ratio of the cellular redox potential including donor/acceptor balance of the electron dynamics due to restoration of the thiol-disulfide metabolism; NAD/NAD•H and NADP/NADP•H ratios;
- cAMP/cGMP ratio, changes of the extra- and intracellular ionized calcium;
- Relationship of the transcriptional differentiation factors (NFκB) and proliferation factors (AP-1); relationship of the functional activity manifestations of p53, p21 and Ras-proteins, therefore, balance of cellular proliferation, differentiation and apoptosis taking into account basically different exhibitions of these effects in the normal and transformed cells.

The presence of a chemical interaction between the disulfide bond of the oxidized glutathione-based compound and the platinum material forms new biophysical sense on provision of the electron balance of biological systems. While not wishing to be bound by any theory, the chemical interaction can be thought of as a donor/acceptor pair where the lone electron pairs on the sulfur atoms can potentially interact with an electron-deficient material, such as the platinum material. Again, while not wishing to be limited by any mechanism, if cellular activity and cellular physical state are determined, at least in part, by donor/acceptor interactions throughout a biological system, then a balance among electron donors and acceptors having equal biopotentials can be a life parameter. Such balance alteration can be used for regulation of different cell functions and physical properties [11]. Sources of the mobile electrons can include π-electrons such as lone-pair electrons of nitrogen, oxygen, and sulfur. There are few acceptor groups (e.g., -C=O- groups) in a normal cell which can be balanced via donor/acceptor dynamics of donor electrons such as π-electrons.

The malignant cell can be characterized as having dramatic disturbances of the donor/acceptor balance towards an excess of donors electrons. Acceptor molecules are almost absent in cancer cells. A possible solution to this imbalance in this situation is the presence of molecules that possess donor/acceptor features within the same molecule, such as a GSSG•Pt material. Introducing GSSG•Pt into biological media can cause a restoration of the electronic balance in the biological media. This restoration can involve catalysis by the platinum atom in a reaction involving the formation of an active oxygen form, e.g., superoxide-anion radicals, singlet oxygen.

In the presented situation the cellular electronic balance will be an interaction of the GSSG•Pt material with the cellular GSH generating oxidative-reductive, i.e. donor/acceptor pair (implying that the said donor is GSH). If there is a high GSH level in cells that is characteristic for tumor-transformed cells with a high proliferative impulse, the pro-oxidative, i.e. oxidative, properties of GSSG•Pt material exhibit in the most evident way. In the case the oxidative stress forms in the tumor cells only causing alteration of the tumor cells mitochondria functions forming intracellular signal for the apoptosis mechanisms induction.

For the normal but "tired", "exhausted" cells, there may be an oxidative-reductive potential optimization, bioenergetic supply for the metabolic transformations, redox-sensitive adequate expression of the genome functional sites, in particular, the immunologically significant genes and transcription factors.

For the transformed cells GSSG•Pt, there may be an incompatibility with vital functions involving chain transfer reaction of π-electrons, disturbance of the mitochondrial oxidative-reductive reactions of electron/protons transfer reactions along the respiratory chain (bringing forth release of cytochrome C from mitochondria) and dislocation of the NAD•H⁺/NADP•H ratio, i.e., forming the intracellular signal for the apoptosis mechanism induction.

The active principle, the composite of GSSG with the metal material selected from the group consisting of platinum and palladium (GSSG•Pt/Pd) having the stabilized disulfide bond capable of stimulating/beneficial modulating the endogenous cytokine and hemopoietic factor production as well as inducing of the transformed cells apoptosis, may be obtained by the following original, developed by the authors the peptide synthesis technique.

### SYNTHESIS METHOD OF THE GSSG COMPOSITE WITH THE COMPLEX PLATINUM (II) OR PALLADIUM (II) COMPOUND.

170 g (0.55 mole) of the reduced glutathione (GSH) are suspended in 200 ml of water and, along with stirring, 139 ml (0.55 mole) of the 4N NaOH solution and then one of the following solutions should be added:
a) 170 ml of 0.05% cis-diamminedichloroplatinum cis-[Pt(NH₃)₂Cl₂] water solution (0.28 mmole);
b) 235 ml of 0.05% potassium tetrachloroplatinate K₂ [PtCl₄] water solution (0.28 mmole);
c) 185 ml of 0.05% potassium tetrachloropalladate K₂ [PdCl₄] water solution (0.28 mmole)

The obtained transparent, slightly yellow solution is cooled to 18-20 °C and 283 ml of the 3% hydrogen peroxide solution (H₂O₂ solution) is added in little portions over a time period of five minutes with such a speed rate that the reaction mixture temperature will not exceed 22-25°C (immersed thermometer).

Thirty minutes after addition of the hydrogen peroxide solution (H₂O₂) the pH is measured, and then the 4N caustic soda solution is added drop-by-drop to reach pH=5.6-5.8 along with simultaneous temperature control, which should be within 22-25°C. Then the cooling is taken away and stirring continued at indoor (room) temperature for 30 minutes more.

Control of the oxidation reaction completeness is performed by the HPLC assay. A liquid chromatograph for HPLC, type Beckman, Sol. Module 126, Det. 168, with a column Luna Phenomenex ODS 4.6x250 mm, or an identical one, is prepared. To prepare the HPLC mobile phase 20 cm³ of acetonitrile and 1 cm³ of freshly distilled trifluoroacetic acid is introduced into a 1000-cm³ graduated flask, and the volume is increased up to the mark by the deionized water. The solution is mixed and degassed by shaking in vacuum.

Thirty minutes after addition of the entire hydrogen peroxide solution amount one will check the oxidation reaction completeness by means of the highly productive liquid chromatography (HPLC). Thereto with a microsyringe one will take 10 µl of the reaction mixture and dissolve them in 1 ml of the mobile phase (0.1% trifluoroacetic acid : acetonitril, 98 : 2). 20 µl of the obtained solution is introduced into the chromatograph Beckman 126 Solvent Module, Diod Array Detector Module 168, the column Luna Phenomenex ODS 4.6_250 mm, or the identical. Elution is performed in isocratic regime, 30 min., in the system 0.1% trifluoroacetic acid : acetonitrile, 98 : 2; the flow-speed rate ml/min, detection at 220 nm, scanning 190-600 nm.

The retention time in the aforesaid conditions is 5.0±0.5 min for reduced glutathione, 11.0±0.5 min for oxidized glutathione.

In case if, according to the HPLC data after the standard chromatogram integration, the oxidized glutathione content is less than 97%, the stirring is continued in the same regime 30 minutes more and the HPLC control is repeated.

In case when the result is equal or exceeds 97%, the reaction is considered as completed and one will pass to the reaction solution filtration. Thereto, there is used a filter having pore size not larger than 0.7 µm.

Weight loss at drying will not exceed 5% at drying to the constant weight at 100°C in vacuum (1 mm Hg) above CaCl₂ and P₂O₅.

The main material content in the ready product by the HPLC data will not be lower than 98%.

Thus, the oxidized glutathione as a composite with the complex compound of Pt(II) or Pd (II) is obtained.

Appearance: white odorless powder.

Solubility: soluble in water, 0.9% isotonic solution of sodium chloride for injections; insoluble in 95% alcohol, chloroform, ether and other organic dissolvents.

Solution transparency and color: 0.05 g of the drug solution in 10 ml of water is transparent and colorless solution.

pH of 1% solution: 5.0-6.0 potentiometrically, the device is pH/mV/°C meter Cole Parmer, model 59003-15 or identical.

Authenticity:
a) amino-acid analysis (6 n HCl, 110°C , 20 hrs.),
   glycine-2.0±15%; glutamic acid - 2.0±15%; cysteine-2.0±40%; amino-acid analyzer AAA T-339 M Prague or identical.
b) HPLC - at the outlet time it corresponds to the standard of bis-(γ-L-glutamyl)-L-cystinyl-bis-glycine disodium salt.

Chromatography conditions: device - BECKMAN "Gold Nouveau Chromatography Data System" Version 6.0, Diod Array Detector Module 126 or identical.

Assay - 20 µl of 0.1% drug solution in the mobile phase, chromatography on the column ULTRASPERE ODS 250±4.6 mm with the converted C₁₈ phase in the isocratic conditions acetonitrile-0.1% trifluorideacetic acid (2:98); flow rate 1 ml/min., detecting at 220 nm, scanning 190-600 nm.

Purity (main substance content):
a) at HPLC not less than 98%:
b) at the amino-acid analysis: not less than 85% (analysis according to Section "Authenticity", Item "a" with an exact weight).

### Method for element content determination:

The exact assay weight (about 50 mg) is dissolved in 50 ml of bidistilled water and the solution is used for the analysis.

The platinum content is determined quantifiably by the method of mass spectrometric analysis with inductively bound plasma at the device of the PQe model made by VG Elemental, England. The analysis relative precision is 5%.

The other element content is determined quantifiably by the method of the atomic-emission spectroscopy with inductive bound plasma on the device of the model TRACE 61E made by Thermo Jarell Ash, USA. The analysis relative precision is 5%.
c) Sodium (Na) content according to the emission spectral method is 7.0±0.5 %.
d) Platinum (Pt) content according to the mass spectrometric analysis is 0.032±0.01%.
e) Palladium (Pd) content according to the mass spectrometric analysis is 0.017±0.01%.

| ***Element content*, µ*g*/*g*:** | |
|---|---|
| Silver (Ag) | < 1.0 (less than 0.0001%) |
| Aluminum (Al) | 2.0 |
| Arsenic (As) | < 1.0 |
| Barium (Ba) | <0.50 |
| Beryllium (Be) | <0.05 |
| Calcium (Ca) | 7.0 |
| Cadmium (Cd) | < 0.05 |
| Cobalt (Co) | < 0.5 |
| Chromium (Cr) | 1.7 |
| Copper (Cu) | < 0.5 |
| Iron (Fe) | < 1.0 |
| Potassium (K) . | < 2.5 |
| Selenium (Se) | < 2.0 |
| Magnesium (Mg) | < 2.5 |
| Manganese (Mn) | < 0.2 |
| Molybdenum (Mn) | < 0.2 |
| Nickel (Ni) | < 0.5 |
| Lead (Pb) | < 0.40 |
| Strontium (Sr) | 1.9 |
| Titanium (Ti) | <0.5 |
| Vanadium (V) | < 0.5 |
| Zinc (Zn) | 0.65 |
| Antimony (Sb) | < 0.5 |

Thereby, the obtained hexapeptide composite (GSSG•Pt) with purpose for subsequent usage in animals and humans is applied as a pharmaceutically acceptable GSSG•Pt derivative in an injectable drug form prepared by dissolving of the bulk substance in sterile water for injections or in any pharmaceutically acceptable solvent with the resultant concentration 0.01-3.0 %. For an *in vitro* use in experimental settings, GSSG•Pt or the derivatives thereof may be dissolved in solvents acceptable for performance of corresponding experiments such as culture media, isotonic saline solutions, glucose solutions and the like.

Injectable medicinal forms of the GSSG•Pt, salts and compositions thereof have been tested in animal studies and as well in wide clinical studies and pilot trials on sick persons. The drug form for human and animal use should be prepared under sterile and pyrogen-free conditions while exerting every effort to prevent chemical or bacterial contamination of the medicinal form.

The present invention presents the advantageous feature that the drug comprising the composite or derivatives thereof has a regulating effect on the endogenous cytokine production processes and, thus, on the proliferation and differentiation processes of the T-and B-lymphocyte subpopulations (CD⁺-cells). Drug induction can result in production of a wide cytokine and hemopoietic factor range and CD⁺-lymphocytes. Therefore, in this range, from the point of view of cytokine interaction, there are both agonist-cytokines and antagonist-cytokines regarding the effects they stimulate (e.g., "relationship" of IL-1α and β and IL-4). In connection with that, depending on the initial patient's immunogenesis system state, hyper- or hypoactivity, the drugs of the present invention can restore a disturbed balance in the system.

The given provision is illustrated in Examples 9-13 which show that patients with depressed immunity (oncological patients receiving radiation or combined chemotherapy) the cytokine synthesis induction (IL-1α and β, IL-2, IL-3, IL-4, IL-6, IL-10 and IL-12, IFN-α and IFN-γ) can be accompanied by restoration of CD3⁺, CD4⁺, CD8⁺, CD16/56⁺, CD25⁺, CD34⁺ counts; and patients with the immunoautoagression signs - at the clones of cytotoxic lymphocytes or fibroblasts in case of viral hepatitis C the Fas-Ag (CD95⁺) is expressed that promotes the apoptosis mechanism induction and elimination of the virus-transformed and/or "aggressive" cells.

Another advantageous feature of the present invention involves the finding of the composite impact on the isolated human lymphocytes 10 minutes (a peak is observed at the 30^{th} minute (the maximal level of phosphorilating of the cytosol proteins obtained from the lymphocytes)) after parenteral introduction of GSSG•Pt material, a significant increase of the phosphorylating level on tyrosine for lymphocyte cytosole proteins that is an integrative characteristic for the cellular signal-transducing system activity. These changes in state for key factors of cAMP, cGMP, inositol-phosphate-dependent signal systems owing to the GSSG•Pt material influence (See Example 8) calls forth the redox-sensitive gene expression, first of all, for the immunologically significant genes responsible for the cytokine and hemopoietic factor synthesis. Therefore, the GSSG•Pt material application in the treatment purposes not only stimulates the cytokine and hemopoietic factor endogenous production but also provides reproduction of the biochemical and physiological cytokine effects, in particular, in the case of sensitivity loss of receptors to cytokines that is observed at oncological and retroviral pathology.

In the tumor- and/or virus-transformed cells the apoptosis mechanisms are induced through the GSSG•Pt material multicytokine-activating impact, its influence on p53-dependent and p53-independent apoptosis induction mechanisms as well as through changing of the donor/acceptor π-electron balance in malignant (cancer) cells (see Examples 14-16).

Depending on the initial patient's biological status including his immunity condition: immunodeficiency, i.e., hyporeactivity; or immunoautoaggression, i.e., hyperreactivity; presence of the tumor- or virus-transformed cells - the composite and/or pharmaceutically acceptable derivatives thereof are able to act as the endogenous cytokine production stimulators/modifiers and/or as the apoptosis mechanism inducers, respectively.

The composite can be administered by various methods: orally or as a solution form selected from the group consisting of inhalation solutions, local instillations, eye drops, intranasal introductions, an ointment for epicutaneous applications, intravenous solutions, injection solutions, and suppositories. Preferably, the glutathione is introduced parenterally or topically.

In one embodiment, the composite is administered in a dosage of between about 0.1 mg/kg to about 1.0 mg/kg by body weight. In another embodiment, the composite is administered in a dosage of between about 1 mg/m² to about 100 mg/m² by body surface. In another embodiment, the drugs can be applied one or more times a day, by one or more day pulses or continuous administration until a desired therapeutic effect is achieved.

In a preferred embodiment, the GSSG•Pt material pharmaceutically acceptable derivatives are introduced to the body at a dose from 0.01 to 1.0 mg of GSSG•Pt material per kg of body weight for the GSSG•Pt material or salt thereof; or at a dose from 1 to 100 mg per 1 m² of body surface and in case when applied epicutaneously/through instillations at a dose from I to 100 mg per 1 m² of body surface as well, at least, once during each 24 hour period. Also the drug can be continuously injected or otherwise introduced to the body to have a 24 hour total dosage from 0.1 to 1.0 mg per kg of body weight for GSSG•Pt base and salts thereof, and from I to 100 mg per 1 m² of body surface during each 24 hour period.

Where the composite is administered as a solution, preferably the solution has a concentration of between about 1 % to about 10% of the composite. Preferably, the pharmaceutically acceptable derivatives of the GSSG•Pt material for parenteral use is in a pharmaceutically acceptable solvent as, for example, an aqueous solution including water, glucose solution, isotonic solutions of sodium chloride, buffered salt solutions. Other physiological solvents or carriers can be used. Where the composite is administered as an injectable form, preferably the injectable form comprises the composite in a solution in a concentration of between about 0.01% to about 3.0%.

For topical application including application for different body cavities, organic solvents or carriers may be used in the form of ointments, pastes, creams or suppositories.

The examples of the invention embodiments given below demonstrate feasibility of the invention practical use and confirm its effectiveness, and also expediency of using the series of the developed medicinal drugs taking into account the wide range of presented diseases (Examples 9-18).

### Examples of the Invention Embodiments

### Example 1

### Synthesis of bis-(L-phenylalanyl-γ-L-glutamyl)-L-cystinyl-bis-glycine disodium salt

(I.) General drug characteristics.
1. Name: bis-(L-phenylalanyl-γ-L-glutamyl)-L-cystinyl-bis-glycine disodium salt, composite with cis-diamminedichlorplatinum.
2. Structural formula - see FIG. 7.
3. Gross-formula: C₃₈H₄₈N₈O₁₄Na₂S₂ • [Pt(NH₃)₂Cl₂]
4. Molecular weight: 950,94 on C₃₈H₄₈N₈O₁₄Na₂S₂ with Pt content 0,033 %.
5. Appearance: white odorless powder.
6. Solubility: soluble in water, 0.9% isotonic solution of sodium chloride for injections; insoluble in 95% alcohol, chloroform, ether and other organic dissolvents.
7. Solution transparency and color: 0.05 g of the drug solution in 10 ml of water is transparent and colorless.
8. pH of 0.1% solution: 5.75 (potentiometry).
9. Authenticity:
a) amino-acid analysis ( 6 n HCl, 110°C, 20 hrs.), (error margin 20%, for cysteine - 35%), in correspondence: glycine - 2.00; glutamic acid 1.92; cysteine - 1.81; phenylalanine - 2.04.
b) NMR(¹H)-spectroscopy, according to - "BRUKER" AM 500, 500 MHz, D₂O.

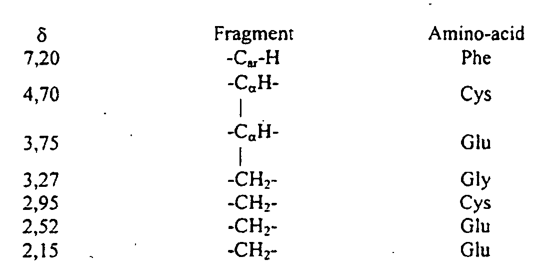

10. Purity (main substance content):
a) At HPLC: not less than 97%:
   Device: BECKMAN "Gold Nouveau Chromatography Data System" Version 6.0, Diod Array Detector Module 126. Assay - 20 µl of 0.1% drug solution in the mobile phase, chromatography on the column ULTRASPERE ODS 250x4.6 mm with a converted C₁₈ phase in isocratic conditions acetonitrile-0.1% trifluorideacetic acid (2:98); flow rate 1 ml/min., detecting at 220 nm, scanning 190-600 nm, PDA functions - Contour Plot, 3D.
b) At the amino-acid analysis: not less than 85 % (analysis according to Item 9a with an exact weight);
c) Thin-layer chromatography is homogenous, analysis is performed at introduction of 5 µl of the 1% drug solution in the band. There are plates Kieselgel 60_{f} (Merck) 10x5 cm, system: n.butanol - acetic acid - water (4:1:1). Development is performed according to the standard methods - ninhydrine and chlorine\benzidine. R_{f}= 0,15;
d) Sodium (Na) content according to the emission spectral method is: 4.8 %;
e) Platinum (Pt) content according to the mass spectrometric analysis is 0.033%.

11. Elements detected content, µg/g:

| | |
|---|---|
| Silver (Ag) | < 1.0 (less than 0.0001 %) |
| Aluminum (AI) | 2.0 |
| Arsenic (As) | < 1.0 |
| Barium (Ba) | < 0.50 |
| Beryllium (Be) | < 0.05 |
| Calcium (Ca) | 7.0 |
| Cadmium (Cd) | < 0.05 |
| Cobalt (Co) | < 0.5 |
| Chromium (Cr) | 1.7 |
| Copper (Cu) | < 0.5 |
| Iron (Fe) | < 1.0 |
| Potassium (K) | < 2.5 |
| Selenium (Se) | < 2.0 |
| Magnesium (Mg) | < 2.5 |
| Manganese (Mn) | < 0.2 |
| Molybdenum (Mo) | < 0.2 |
| Sodium (Na) | 48 mg/g |
| Nickel (Ni) | < 0.5 |
| Lead (Pb) | < 0.40 |
| Platinum (Pt) | 330 µg/g |
| Strontium (Sr) | 1.9 |
| Titanium (Ti) | < 0.5 |
| Vanadium (V) | < 0.5 |
| Zinc (Zn) | 0.65 |
| Antimony (Sb) | < 0.5 |

Determination method:
The exact assay weight (about 50 mg) is dissolved in 50 ml of double-distilled water and the solution is used for the analysis.
The platinum content is determined quantifiably by the method of mass spectrometric analysis with inductively bound plasma at a PQe device made by VG Elemental, England. The analysis relative precision is 5%.
Content of other elements is determined quantifiably by the method of the atomic-emission spectroscopy with inductive bound plasma on a TRACE 61 E device made by Thermo Jarell Ash, USA. The analysis relative precision is 5%.
12. Weight loss at drying: 10% at drying till the constant weight at 100°C in vacuum (1 mm Hg) above CaCl₂ and P₂O₅.
(II.) Synthesis method description.
13. Process chemical scheme - see FIG. 8.
14. Method description

(III). Product (I) γ-L-glutamyl-L-cysteinyl-glycine in amount of 3.07 g (10 mmol) and N-hydroximethylbenzamide (II) in amount of 5.89 g (13 mmol) is dissolved in 30 ml of anhydrous trifluoroacetic acid (TFA) mix at the room temperature during one hr. Then the solvent is distilled off in vacuum at 40° C, 30 ml of anhydrous ethyl alcohol is added to the remainder; the solvent is again distilled off in vacuum and the procedure is repeated two times more. The product is crystallized through grinding in 50 ml of anhydrous diethyl ether, filtered, washed on the filter with 2x20 ml of anhydrous ether and further it is dried in vacuum above KOH and P₂O₅. Recrystallization is done from 90% ethanol. Yield - 5.50 g (80%). R_{f} = 0,43, Kieselgel 60_{f} (Merck) 10x5 cm, system: n.butanol - acetic acid - water (4:1:1).
(V). Product (III) in amount of 4.40 g (10 mmol) is stirred in the mixture of 15 ml of distilled water and 25 ml of dioxane; then along with mixing, 10 ml (20 mmol) of 2 N NaOH solution is added.
Then 3.62 g (10 mmol) of phenylalanine N-hydroxysuccinimide ester (IV) is introduced in the reaction mixture and the stirring is continued during 12 hrs at room temperature.
Then the mixture is evaporated in vacuum at 40° C to dryness. The residue is dissolved in 200 ml of ethyl acetate and washed by 2x20 ml of 1 N sulphuric acid, water, sodium bicarbonate (2x50), water and the organic layer is above the anhydrous chloride calcium. Then ethyl acetate is distilled in vacuum at 40° C to dryness and the product is crystallized from ethyl acetate/ether.
The crystals are separated by filtration and dried in vacuum above phosphorus pentoxide to constant weight. The product yield (V) - 4.88 g (70 %). R_{f} = 0,80, Kieselgel 60_{f} (Merck) 10x5 cm, system: n.butanol - acetic acid - water (4:1:1).
(VI). Product (V) in amount of 6.87 g (10 mmol) is dissolved in 20 ml of distilled trifluoroacetic acid and the solution is kept at room temperature during two hrs. Then the product is precipitated by absolute ether (about 200 ml), filtered and dried in vacuum above KOH to the constant weight. The product yield - (V) 5.28 g (90 %), R_{f}=0.48, Kieselgel 60_{f} (Merck) 10x5 cm, system: n.butanol - acetic acid - water (4:1:1).
(VII). Product (IV) in amount of 5.87 g (10 mmol) is dissolved in 100 ml of a mixture methanol-water (1:1), then 200 ml (10 mmol) of mercury acetate solution are added and the mixture is stirred at room temperature during one hr. Then the hydrogen sulphur flow is sparged through the solution during 20 min. controlling precipitation efficiency at the assay. The mercury sulphide precipitate is filtered; the filtrate is evaporated in vacuum up to volume of 10 ml; then 200 ml of isopropyl alcohol is added and the product is crystallized at cooling to 0-4° C. The crystals are filtered, washed with isopropyl alcohol, acetone and dried in vacuum above C₂O₅. The product yield (VII) - 3.72 g (82%). R_{f} =0.30, Kieselgel 60_{f} (Merck) 10x5 cm, system: n.butanol - acetic acid - water (4:1:1).
(VIII). Product (VII) in amount of 4.54 g (10 mmol) is suspended in 20 ml of water and along with stirring, 5 ml (10 mmol) of 2 N NaOH solution and then 4.8 ml of 0.05% water solution of cis-diamminedichloroplatinum (cis-[Pt(NH₃)₂Cl₂]) is added. The solution is cooled to 18-20° C and in little portions during about two min., 5.1 ml of 3% solution is added at such a rate so that the temperature will not exceed 22-25° C. Thirty minutes after introduction of the whole hydrogen peroxide amount, the solution pH is measured and its value is brought to 5.6 - 5.8 by adding of the necessary amount of 4N NaOH solution, while the solution temperature is monitored (it is within 22-25° C). Then the stirring is continued without external cooling for 30 min. and then the control analysis of the reaction mixture is performed by HPLC. With that purpose 10 µl is taken out of the reaction solution and dissolved in 1 ml of the mobile phase. If, according to the HPLC data, the oxidized form content is equal or exceeds 95%, the reaction is deemed as finished. Otherwise, the stirring at the room temperature is continued 30 min. more and the HPLC assay is repeated.

Then the reaction solution is filtered through the filter with pore size not larger than 0.7 µm and the filtrate is lyophilized. The obtained dry product is dried out in vacuum at 40°C above anhydrous calcium chloride to the constant weight. Yield - 4.51 g (95%).

The ready substance is analyzed according to Items 5-12.

### Example 2

### Synthesis of bis-(γ-L-glutamyl)-L-cystinyl-bis-glycine lithium salt

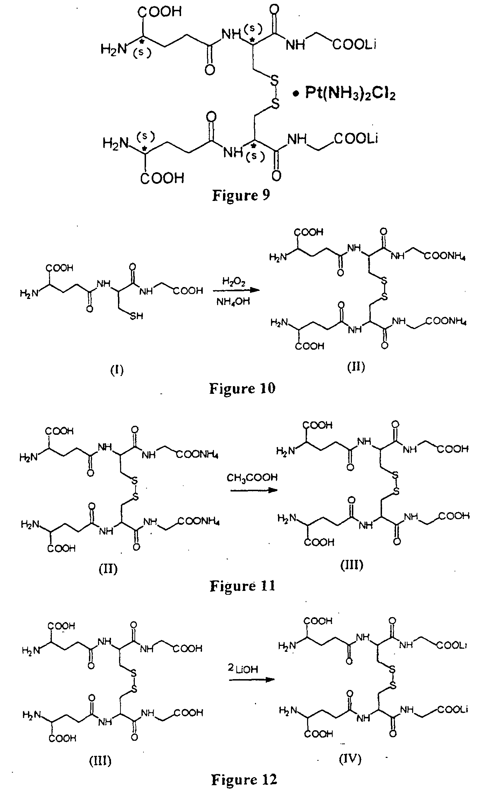

(I.) General drug characteristics.
   1. Name: bis-(γ-L-glutamyl)-L-cystinyl-bis-glycine dilithium salt with cis-diamminedichloroplatinum.
   2. Structural formula - see FIG. 9.
   3. Gross-formula: C₂₀H₃₀N₆O₁₂Li₂S₂ **•** [Pt(NH₃)₂Cl₂]
   4. Molecular weight: 624.49 on C₂₀H₃₀N₆O₁₂Li₂S₂ with Pt content 0,032%
   5. Appearance: white odorless powder.
   6. Solubility: soluble in water, 0.9% isotonic solution of sodium chloride for injections: insoluble in 95% alcohol, chloroform, ether and other organic dissolvents.
   7. Solution transparency and color: 0.05 g of the drug solution in 10 ml of water is transparent and colorless.
   8. pH of 0.1% solution: 5.0-6.0 (potentiometry).
   9. Authenticity:
      a) amino-acid analysis (6 n HCl, 110° C, 20 hrs.) in correspondence: glycine - 2.0 (2.0); glutamic acid - 1.9 (2.0); cysteine - 1.7 (2.0).
      b) NMR(¹H)-spectroscopy, in correspondence: CH₂ (δ 2.05, 2.40, 3.00, 3.80); CH (δ 3.72, 4.65).
      c) HPLC corresponds with the standard according to the yield time.
   10. Purity (main substance content):
      a) at HPLC > 95%;
      b) at the amino-acid analysis > 85 %;
      c) Thin-layer chromatography (TLC) homogenous;
      d) Lithium (Li) content according to the emission spectral method is 2.2 ± 0.1%
      e) Platinum (Pt) content according to the mass spectrometric analysis is 0.01 - 0.02%
(II.) Staged scheme for the product synthesis (A→B →C)
   A. Oxidation of reduced glutathione (γ-L-glutamyl-L-cysteinyl-glycine)
      A₁ - stage for the source reduced glutathione processing by hydrogen peroxide at pH=8 - see FIG.10.
         Source compound (I): γ-L-glutamyl-L-cysteinyl-glycine (GSH)
         H-γ-L-Glu-L-Cys-Gly-OH
         Reagents:
         1) hydrogen peroxide (35%) Fluka
         2) ammonia solution (25%)
         Reaction conditions: stirring of the water solution (I) and the reagents at 20° C (pH=8.0) during 20 min.
      A₂ - separation of free hexapeptide bis-(γ-L-glutamyl)-L-cysteinyl-bis-glycine (see FIG. 11) 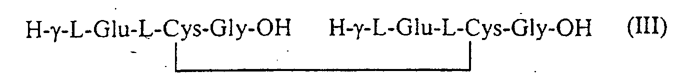 Source compound: reaction mixture of the A₁ stage.
         Reagents: glacial acetic acid
         Reaction conditions: acidification of the A₁ reaction mixture by glacial acetic acid up to pH=5.0, the solution filtration and lyophilic drying of the product.
         Control for the A oxidation stage processing: by HPLC at the Delta Pack C18 column (0.1% TFA-MeCN, 0-25%); one will check presence of the peak (>97%) for the compound (III) (7.4 ± 0.4 min) and the peak absence for the compound (I) (3.0 ± 0.3 min).
   B. Conversion of the oxidized form (III) into the lithium salt (IV) - see FIG. 12.
      Source compound: free hexapeptide (III).
      Reagents: 1 N LiOH solution.
      Reaction conditions:
      a) titration of the compound (III) water solution by two LiOH equivalents;
      b) water evaporation in vacuum at 35-40°C;
      c) product precipitation by isopropyl alcohol;
      d) precipitate filtration;
      e) precipitate washing by acetone;
      f) product drying in vacuum (1 mm Hg) at 35-40°C.
      Control of the B stage processing: the reagents quantities and technological conditions at drying is inspected.
   C. Finished product quality control.
      1. The main material content according to HPLC: >97%.
         The analysis is performed using 20 µl of 0.1 % drug solution in the mobile phase, on the column 250x4.6 mm with a converted C₁₈ phase in isocratic conditions acetonitrile-0.1% trifluorideacetic acid (2:98); flow rate 1 ml/min., detecting at 220 nm. The comparison is made with the standard peak obtained in the same conditions.
      2. The main material content according to the spectrophotometric analysis data on the non-oxidized thiol groups content: >95%.
         Analysis: 0.12 ml of 0.5% drug solution is placed into a 25-ml measuring flask, 1 ml of 0.1 % Tris-HCl buffer with pH=8, 0.01 M EDTA and 1 ml of 2% NaBH₄ solution. The reaction mixture is incubated at 20°C during 30 min. The reaction is terminated through introduction of 0.6 ml of 1 M HCl during two min. in portions of 0.05 ml along with agitation and following introduction of 2 ml of acetone during three min. with stirring. Then 0.25 ml of the Elman reagent is added and the volume is brought to the mark by 0.1 M the phosphate buffer solution (pH 8.0). An optical density is measured at 412 nm, water is a comparison solution. Simultaneously, the procedure with the drug standard is conducted and the obtained data is compared.
      3. Presence of foreign admixtures: according to TLC data the drug is homogenous.
         Analysis is performed at introduction of 5 µl of the 0.1% drug solution in the band. The plates are Kieselgel 60_{f} (Merck) 10x5 cm, system: n.butanol - pyridine - acetic acid - water (150:100:30:120). Development is performed according to the standard methods - ninhydrine and chlorine\benzidine.
      4. Lithium (Li) content according to the emission spectral method is 2.2 ± 0.1 %.

### Example 3

### Pharmacokinetics and metabolism of GSSG•Pt and GSSG in blood serum and tissues after intravenous introduction

The time-concentration GSSG curves and activity changes for enzymes participating at the GSSG metabolism after the GSSG•Pt and GSSG intravenous introduction in different doses were studied. The variation of the oxidized glutathione concentration was evaluated in animal blood serum, liver, kidneys, spleen and lymphocytes during 60 min. after the single GSSG•Pt and GSSG intravenous introduction in doses 2 mg/kg and 20 mg/kg of body weight. In addition, the activity variation of the enzymes participating in GSSG metabolism was evaluated (glutathione reductase, glutathione-peroxidase, glutathione-S-transferase, γ-glutamyl-transpeptidase).

The study was performed at male CBA mice (standard body weight - 180 to 200 g). Five groups of animals (with no less than 15 mice in each) were formed. The group description is represented below.

### Control groups:

#1 - intact animals receiving a single injection of the tested article vehicle (normal saline - (NS)) instead of the drug;

### Test groups:

#2 - animals receiving the GSSG injection (GSSG dissolved in normal saline) in a dose of 2 mg/kg;
#3 - animals receiving the GSSG injection (GSSG dissolved in normal saline) in a dose of 20 mg/kg;
#4 - animals receiving the GSSG•Pt injection (GSSG•Pt dissolved in normal saline) in a dose of 2 mg/kg;
#5 - animals receiving the GSSG•Pt injection (GSSG•Pt dissolved in normal saline) in a dose of 20 mg/kg.

The blood samples were taken at 1, 2, 5, 10, 20, 40 and 60 min., the serum was separated and the concentration analysis was performed according to a standard method where the main stages are protein precipitation, removal from the sample of non-polar and medium-polar compounds and the following chromatographic analysis with spectrophotometric detection in conditions of isocratic and linear gradient elution.

The GSSG content variation in the blood serum, different organs and the lymphocytes at the drug intravenous introduction are given in the Tables 1-4.

Activity of the enzymes participating in the GSSG metabolism (glutathione reductase: EC.1.6.4.2; glutathione-peroxidase: EC.1.11.1.9; glutathione-S-transferase: EC.2.5.1.18; γ-glutamyl-transpeptidase: EC.2.3.2.2) were determined by the standard reagent kits produced by Boehringer Mannheim GmbH. The enzyme activity variation values after the GSSG•Pt and GSSG intravenous introduction in dose of 2 mg/kg are given at the Tables 5, 6, 7.

Comparing the drug dynamic distribution of the drugs in the blood serum, liver, kidneys, spleen and lymphocytes a clear advantage for the GSSG•Pt pharmacokinetics regarding GSSG is evident. The GSSG concentration to the 10^{th} minute is almost equal to the initial one whereas, at the same time, the GSSG•Pt concentration exceeds 50 times the initial parameters and remains at the given high level till the end of the studied period. Besides, the maximal GSSG•Pt concentration in the blood serum and in the tissues exceeds 3 times the maximal GSSG concentration. These features determine higher effective duration of impact for GSSG•Pt comparing to GSSG.

As it follows out of the Tables 5, 6, 7 materials the GSSG drug increases approximately two times the activity for the enzymes participating in the thiol metabolism with large number of proved indices whereas the GSSG•Pt does not significantly alter activity of the thiol metabolism enzymes, mainly the HADP•H-glutathione-reductase. It indicates the greater GSSG•Pt drug stability as a substrate in regard to main enzymes participating in the glutathione metabolism and, therefore, calls out longer presence of the glutathione oxidized form in the blood serum and different organs. The GSSG•Pt effects on glutathione-S-reductase that is considered now as playing a crucial role in regard to tumor transformation mechanisms were found to be a very important exclusion from the aforesaid. Activity of the given enzyme was noted to be stimulated by GSSG•Pt and, a t that, in a larger extent comparing to GSSG (Tables 5, 6 and 7).

Thus, the obtained data analysis demonstrated the higher GSSG•Pt stability to selective impact of the main glutathione metabolism enzymes (first of all, glutathione-reductase) that determines new dynamics for the drug pharmacokinetics. It facilitates manifestation of new biological-pharmacological effects and, thereupon, new GSSG•Pt therapeutic effects.

### Example 4

### Comparative analysis of the GSSG•Pt and GSSG pharmacokinetics at experimental intravenous introduction.

### 1. GSSG pharmacokinetics.

### 1.1 Initial data

| | 0 | 1 | 2 | 5 | 10 | 20 | 40 | 60 |
|---|---|---|---|---|---|---|---|---|
| Assays | - | 1-4 | 5-8 | 9-12 | 13-16 | 17-20 | 21-24 | 25-28 |
| Drug concentrations, µg/mL | 0.2 | 46.7 | 43.15 | 17.6 | 7.0 | 1.5 | 1.9 | 5.6 |
| | 0.6 | 86.6 | 31.1 | | 7.0 | 3.2 | | 0.04 |
| | | | 73.6 | 23.7 | 19.1 | 5.6 | 3.8 | 0.1 |
| | | 71 | 41.3 | 10.6 | 7.7 | 4.8 | | |
| Ar. mean. | 0.3 | 68.1 | 47.3 | 17.3 | 10.2 | 3.8 | 2.9 | 1.9 |
| Mean error | 0,02 | 7.6 | 9.2 | 3.8 | 3.0 | 0.9 | 1.0 | 1.8 |

### 1.2 Graph on mean values

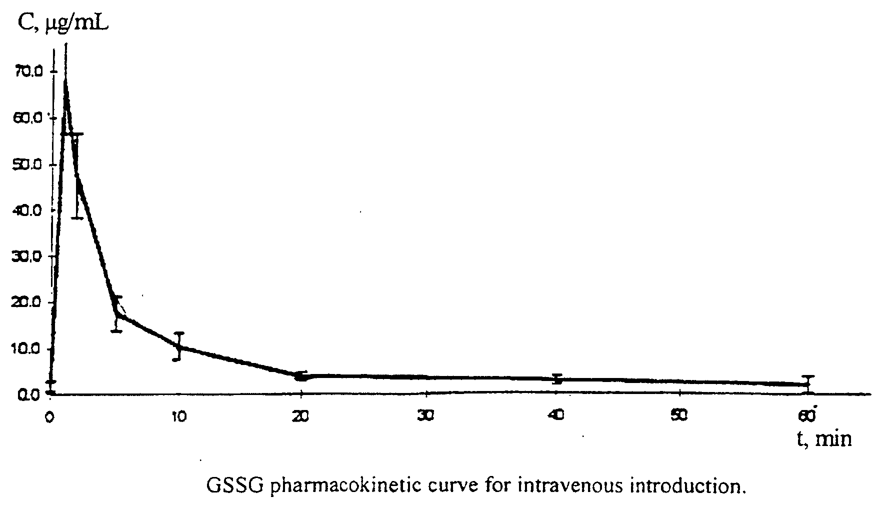

### 1.3. Pharmacokinetic parameters

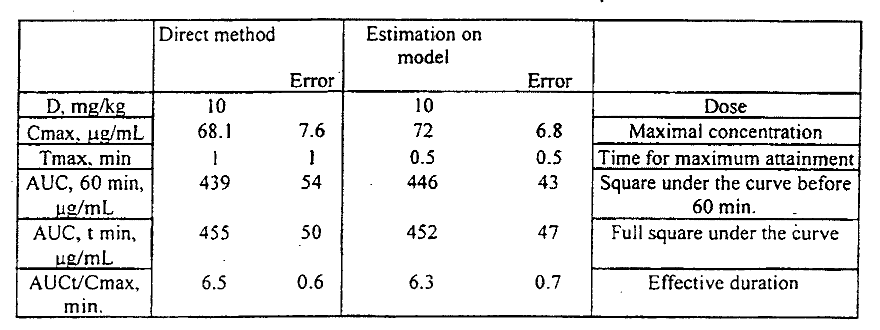

### 2. GSSG•Pt pharmacokinetics.

### 2.1 Initial data.

| Time, min. | 0 | 1 | 2 | 5 | 10 | 20 | 40 | 60 |
|---|---|---|---|---|---|---|---|---|
| Assays | | 1-4 | 5-8 | 9-12 | 13-16 | 17-20 | 21-24 | 26-28 |
| | 0.15 | 265.5 | 213.6 | 98 | 5 | 9 | 1.6 | 0.8 |
| | 0.3 | 186.2 | 11.95 | 56.7 | 19.8 | 6 | 1.1 | 0.3 |
| | | 112.5 | | 87.4 | 25.8 | 6.75 | 1.1 | 0.9 |
| | | | | 29 | 9.6 | 4.4 | 0.4 | 0.3 |
| Mean concentration, µg/mL Error | 0.23 | 187.4 | 112.8 | 67.8 | 15.1 | 5.7 | 1.1 | 0.6 |
| | 0.01 | 19 | 100.8 | 15.6 | 4.7 | 0.6 | 0.2 | 0.2 |

### 2.2. Graph on mean drug concentration values

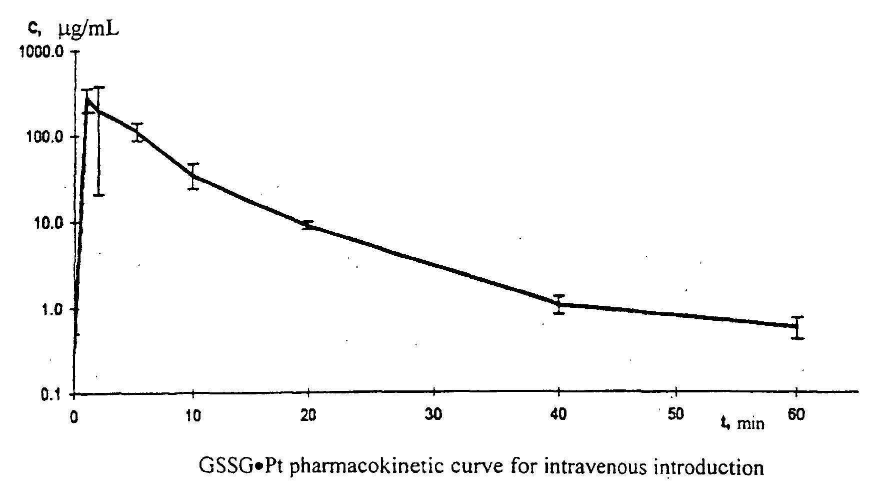

### 2.3. Pharmacokinetic parameters

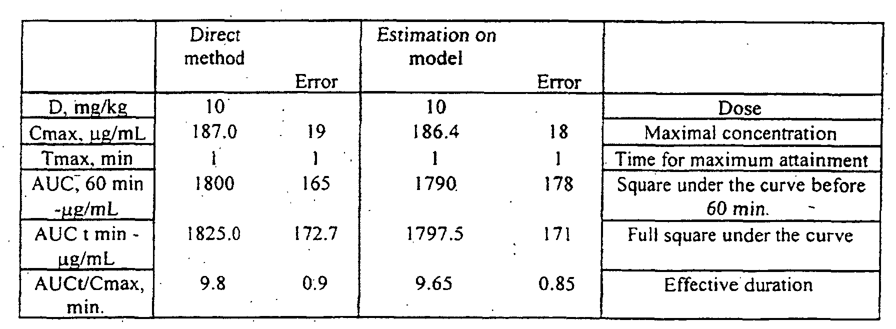

### Conclusion.

It was demonstrated at the comparative analysis for the GSSG•Pt and GSSG drugs that the main GSSG•Pt pharmacokinetic parameters (maximal blood concentration and effective drug influence duration) obtained both by the direct method and by the estimation on model exceed about 2-3 times the main GSSG pharmacokinetic parameters. The integral indices showing the drug blood presence duration determined by calculation of the square under the GSSG•Pt pharmacokinetic curve exceed 4 times the corresponding indices regarding to GSSG. Thus, due to more advantageous pharmacokinetic parameters the GSSG•Pt drug has higher pharmacological activity and biological availability comparing to the GSSG drug.

### Example 5

### Effect of GSSG•Pt and GSSG on cytokine production by human peripheral blood mononuclear leukocytes in vitro

Oxidized glutathione (GSSG) as well as a structural analog thereof, which is a hexapeptide with a stabilized disulfide bond, were evaluated for their effect on cytokine production by human peripheral blood mononuclear leukocytes in vitro.

The leukocytic cytokine production was triggered by adding a mitogen, concanavalin A (ConA) to the cell culture immediately after introducing the test substances. In 24 hours of the cellular exposure to ConA and the test articles, the culture supernatants were sampled and stored until cytokine determination at -70°C.

With the aim of evaluating the functional status of the cells and their capacity of responding to the mitogen in the presence of the test articles at each concentration level, the control cell cultures, containing the test articles in identical concentrations, were incubated for 72 hours following the initial concomitant introduction of ConA and the test substances. Sixteen hours prior to the incubation completion, ³H-thymidine was added, and the label rate of incorporation into DNA was interpreted as the criterion of the cellular test system functional state.

Venous blood from male healthy volunteers was collected into plastic heparinized tubes (endotoxin tested). PMNL fraction was isolated by centrifugation in density gradient of Ficoll and sodium diatrizoate (Histopaque-1077; Sigma).

Cell concentration was adjusted to 2x10⁶ per 1 mL of culture medium (RPMI 1640, Sigma) containing: HEPES (20 mM); L-glutamine (2 mM); Gentamicin (50 _g/mL); fetal calf serum (10%). All the reagents used were of cell culture tested grade, Sigma. Cell viability was estimated by the Trypan blue exclusion method and 100 _L of cell suspension (200,000 cells) were placed into each well of flat bottom 96-well sterile microtiter plates for tissue cultures. Cells from each subject were placed into no less than 39 wells.

The five following final concentrations of the test articles (GSSG, as well as GSSG•Pt) were evaluated: 5000 µg/mL; 500 µg/mL; 50 µg/mL; 5 µg/mL; and 0.5 µg/mL. Each concentration was established in no less than six wells by adding 50 mL of medium containing the appropriate quantity of the previously dissolved test articles. Another six wells were used for control cultures: only 50 µL of medium was added.

Immediately after the test articles had been introduced into the cultures, 50 µL of medium containing ConA (Sigma, cell culture tested) in a quantity required for a final concentration of 4.0 µg/mL, was added to all the wells excepting three additional ones which served for evaluation of spontaneous ³H-thymidine uptake (without ConA).

After a 24-hour incubation at 37°C and 5% of CO₂, contents of three wells (from each sextuplet of identical wells) were taken out, centrifuged, and the supernatants were frozen and kept at -70°C until the cytokine assay was to be performed. Cultures in the other three wells (of each sextuplet) were incubated further under the conditions described above.

Fifty-six hours after the incubation had begun, 1.0 µCi of ³H-thymidine was added into all the remaining cultures, the plates were incubated for another 16 hours, and then the contents of the wells were harvested and transferred onto glass-fiber filters which were consequently treated with 5% trichloroacetic acid and ethanol. The filters were dried and their radioactivity (counts per minute, cpm) was determined using liquid scintillation counter, Betaplate 1.205 (LKB).

Mean radioactivity values for triplicates of identical cultures were used to calculate the index of mitogenic stimulation: the ratio of averaged cpm values for ConA stimulated cultures to averaged cpm values for unstimulated ones (three wells without ConA). This stimulation index for wells, where the test articles were present in various concentrations, served as a criterion of cellular functional status, and ability of the cells to respond to mitogenic stimulation.

Supernatants of 24-hour culture triplicates were subsequently assayed for cytokine content only if their 72-hour matched control culture triplicates developed mitogenic response to ConA with value of the stimulation index in the range from 15 to 50.

Concentrations of interleukin-1b (IL-1b), interleukin-2 (IL-2); interleukin-3 (IL-3); interleukin-4 (IL-4); interleukin-6 (IL-6), interleukin-8 (IL-8); interleukin-10 (IL-10): interleukin-12 (IL-12); tumor necrosis factor-α, γ (TNF-α, γ), and interferon-α, γ (IFN-α, γ) were determined by ELISA using commercial reagent kits (Medgenix, Belgium) and were expressed in pg/mL of culture supernatants.

The salient findings given in Tables 8, 9. As one can see from Tables 8 and 9, the adding of GSSG and GSSG•Pt into the culture media resulted in statistically significant and dose-dependent stimulation of the cytokine production by human mononuclear leukocytes. However, GSSG•Pt stimulating influence was more significant (1.5-2 times as much) on the studied cytokine production with stimulation and regulation for production of the wider cytokine range in comparison with the GSSG effect. One can clearly see correlation of the interrelated cytokine changes (increasing of IL-1b, IL-2, TNF-α, γ along with decreasing of IL-4, IL-10) in the Tables 8 and 9.

Thus, the GSSG•Pt impact on the human peripheral mononuclear leukocytes *in vitro* was manifested with considerable stimulation of the wider cytokine range release into culture media considering their reciprocal regulative effect, and, thereby, it confirmed the GSSG•Pt stimulatory and regulatory effect on the natural cytokine-producing capacity of the human blood cells.

### Example 6

### Effect of GSSG and GSSG•Pt on cytokine and hemopoietic factor production as well as on hemopoiesis and immunity parameters in cyclophosphamide-induced hemo- and immunodepression.

1. The oxidized (GSSG) glutathione as well as the structural analog thereof, which is the hexapeptide with the stabilized disulfide bond, were evaluated in a murine model of hemo- and immunodepression induced by a single administration of cytostatic agent Cyclophosphamide (CP).

The study was designed to evaluate the effect of a five-day long administration of the test articles on the capability of the CP-treated murine splenocytes to produce interleukin-1 (IL-1 α, β); interleukin-2 (IL-2); interleukin-3 (IL-3); interleukin-4 (IL-4); interleukin-6 (IL-6), interleukin-8 (IL-8); interleukin-10 (IL-10); interleukin-12 (IL-12); tumor necrosis factor-α, γ (TNF-α, γ), interferon-α, γ (IFN-α, γ) and G-CSF, M-CSF, GM-CSF *in vitro.* In addition, the blood leukocyte and lymphocyte count and the bone marrow cellularity (karyocyte count) were determined at eight days after CP administration. Some animals receiving CP were then challenged with sheep red blood cells (SRBC), and the humoral immune response to the antigen was evaluated.

Male CBA mice (180 to 200 g body weight) were given a single intraperitoneal injection of CP in a dose of 50 mg/kg. Four groups of animals (with no less than 15 mice in each) were formed. The group description is represented below.

### Control groups:

- #1 - intact animals receiving a single injection of normal saline (NS) instead of CP injection, which further were treated with test article vehicle (normal saline);
- #2 - animals receiving a single CP injection, which further were treated with test article vehicle (normal saline);

### Test groups:

- #3 - animals receiving a single CP injection, which further were treated with the test article (GSSG dissolved in normal saline) in a dose of 5 mg/kg;
- #4 - animals receiving a single CP injection, which further were treated with a GSSG•Pt (dissolved in normal saline) in a dose of 5 mg/kg.

Twenty-four hours after the CP injection, five animals in each group were immunized with SRBC (10⁷ cells in 0.5 mL of NS, intra-peritoneally).

On day 3 after the CP injection (24 hours after the immunization) the intraperitoneal injections of the test or reference articles were started (as it has been described above). Injections were performed during five days: once a day, daily.

Twenty-four hours after the completion of five-day treatment course (on the 8^{th} day after the CP injection), mice were euthanized and splenocyte cultures were aseptically prepared for assessment of spontaneous cytokine and hemopoietic factor production by the spleen lymphocytes in vitro.

Simultaneously, blood and marrow samples were collected for blood leukocyte, lymphocyte, and marrow nucleated cell counts.

Serum samples from immunized animals were tested on level of SRBC agglutinins (day 8 after the CP injection, and day 7 after the immunization).

Table 10 shows the parameters of cytokine and hemopoietic factor production by splenocytes, bone marrow and blood count indices, and the immune response to sheep red blood cells in mice receiving the test articles against the background of cyclophosphamide induced hemo- and immunodepression.

According to the Table 10 data, the GSSG•Pt administration set to norm the cytokine and hemopoietic factor production while GSSG performed only exiguous stimulating effect. Besides, GSSG•Pt stimulated production for the wider cytokine and hemopoietic factor range as well as had significant regulatory influence on shift with respect of the cytokine status that was confirmed with the positive correlation of the interrelated cytokine changes at the corresponding pathologic process.

Thus, the GSSG•Pt use in CP-induced hemo- and immunocompromised animals results in a prominent stimulation of the cytokine and hemopoietic factor endogenous production along with restoration of the bone marrow and blood cellular indices as well as immune response development to sheep red blood cells.

2. The purpose of the present study was to explore the GSSG•Pt efficacy on the cyclophosphamide-induced cytopenia (myelopenia) model.

The study was conducted on the white male rats weighing 160.0 gr. Cyclophosphamide was introduced once subcutaneously at the back in doses 100 mg/kg (a vehicle was water for injections).

Four groups of animals (with no less than 15 mice in each) were formed. Group description is represented below.

### Control groups:

- #1 - intact animals receiving a single injection of normal saline (NS) instead of CP injection, which further were treated with test article vehicle (normal saline);
- #2 - animals receiving a single CP injection, which further were treated with test article vehicle (normal saline).

### Test group:

- #3 - animals receiving a single CP injection, which further were treated with GSSG•Pt (dissolved in normal saline) in a dose of 5 mg/kg.

On day 2 after the CP injection the intraperitoneal injections of the test or reference articles were started (once a day, during 10-15 days).

At the end of each series (10 and 15 days) the experimental groups were euthanized through ether overdose and peripheral blood (tail veins) and bone marrow (femora) were taken for the analysis. The hematological studies were conducted with the unitized standard methods. The peripheral blood analysis results are represented in the Table 11; myelogram analysis results are given in the Table 12.

Analyzing Table 11 results one can note that cyclophosphamide in the dose 100 mg/kg was found to perform the marked cytopenic effect at all of the formed blood elements with absolute and relative lymphopenia depending on the observation terms (maximally exhibited at the day 15).

It should be noted that four animals died: on the 9^{th}, 10^{th}, 12^{th}, and 14^{th} days. In fact, all these animals demonstrated flabbiness, hypodynamia, weight loss.

The GSSG•Pt introduction provided the significant stimulating effect. The general state improvement, positive weight changes were observed, and the immature cell form appearing in blood indicated the bone-marrow hemopoiesis activation. The death of the animals was not noted.

Analysis of the myelogram revealed that cyclophosphamide in the dose 100 mg/kg was found to perform the significant myelotoxic effect. Erythro-, trombocyto- and lymphopoiesis are especially suppressed. Myelosuppression is the most marked at the 15^{th} day.

The GSSG•Pt administration had the considerable myelostimulating effect.

The drug GSSG•Pt in the dose 10 mg/kg being introduced as the daily course during 15 days exerts the marked myelostimulating effect at the cyclophosphamide-induced cyto- and myelopenia. The drug administration provided 100% survival rate of the treated animals whereas the 30% mortality rate was noted in the control group.

### Example 7

### Effect of GSSG and (Li•GSSG•Pt) on cytokine and hemopoietic factor production as well as on hemopoiesis and immunity parameters in radiation-induced hemo- and immunodepression

Both oxidized (GSSG) and the structural analog thereof, which is the lithium salt of GSSG•Pt (Li•GSSG•Pt), were evaluated in a murine model of hemo- and immunodepression induced by a single irradiation in a total dose of 1 Gy [19, 22, 27-37].

The study was designed to evaluate efficacy of seven-day daily administration of the test articles (with the dosing started two hours post-exposure) on the capability of the splenocytes from mice exposed to radiation to produce interleukin-1 (IL-1α, β); interleukin-2 (IL-2); interleukin-3 (IL-3); interleukin-4 (IL-4); interleukin-6 (IL-6), interleukin-8 (IL-8); interleukin-10 (IL-10); interleukin-12 (IL-12); tumor necrosis factor-α, γ (TNF-α, γ), interferon-α, γ (IFN-α, γ) and G-CSF, M-CSF, GM-CSF *in vitro*. In addition, the blood leukocyte and lymphocyte counts and the spleen and bone marrow cellularity (karyocyte count), as we well as splenic and medullary colony-stimulating capacity, were determined at the 8^{th} day post-exposure.

Male CBA mice (18 to 20 g body weight) were irradiated with single dose of 180 kV X-rays filtered with 0.5 mm Cu (at 15 mA, distance - 70 cm, duration two min. and 28 sec.). The total absorbed dose comprised approximately 1 Gy.

Four groups of animals (with no less than 12 mice in each) were formed. Group description is represented below.

### Control groups;

- #1 - intact animals receiving a sham irradiation procedure to reproduce a stress impact, which further were treated with the test article vehicle (normal saline);
- #2 - control animals irradiated in a dose of 1 Gy, which further were treated with test article vehicle (normal saline).

### Test groups:

- #3 - animals irradiated in a dose of 1 Gy, which further were treated with the test article (GSSG dissolved in normal saline) in a dose of 5 mg/kg;
- #4 - animals irradiated in a dose of 1 Gy, which further were treated with the test article (Li•GSSG•Pt dissolved in normal saline) in a dose of 5 mg/kg.

Two hours after the irradiation the intraperitoneal injections of the test or reference articles were started (as it has been described above). Injections were performed during seven days: once a day, daily.

Twenty-four hours after the completion of seven-day treatment course (on the 8^{th} day after the irradiation), mice were euthanized and splenocyte cultures were aseptically prepared for assessment of spontaneous cytokine and hemopoietic factor production (interleukin-1 (IL-1 α, β); interleukin-2 (IL-2); interleukin-3 (IL-3); interleukin-4 (IL-4); interleukin-6 (IL-6), interleukin-8 (IL-8); interleukin-10 (IL-10); interleukin-12 (IL-12); tumor necrosis factor-α, γ (TNF-α, γ), interferon-α, γ (IFN-α, γ) and G-CSF, M-CSF, GM-CSF) by the spleen lymphocytes *in vitro.*

Simultaneously, blood, spleen and marrow samples were collected for blood leukocyte and lymphocyte, and spleen and marrow nucleated cell counting.

Additionally, hemopoietic colony-formation ability of spleen and bone marrow cells was assessed by the method of colony-forming unit (CFU) direct count in the spleens of irradiated singenic CBA mice receiving intravenously spleen or bone marrow cells obtained from animals of control or test groups.

Splenocytic levels for the cytokine and hemopoietic factor production, blood, bone marrow, and spleen cellular indices as well as the colony-forming parameters (colony-forming units, CFU) at the bone marrow and spleen of the irradiated animals at the 8^{th} day post-exposure, are summarized in Table 13.

As is evident from the table data, the Li•GSSG•Pt administration results in statistically significant recovery of the cytokine and hemopoietic factor production by splenocytes, whereas GSSG produces less significant effect. However, Li•GSSG•Pt influences endogenous production for the wider cytokine and hemopoietic factor range as well as regulates the cytokine status alterations with respect of the corresponding pathologic process.

Thus, the Li•GSSG•Pt usage as an applied method in animals with developed radiation-induced hemo- and immunodepression results in pronounced stimulation-regulation of the endogenous cytokine and hemopoietic factor production, and also leads to an effective recovery for the cellular compositions of the blood, lymphoid and hemopoietic organs as well as the bone marrow and spleen colony-forming activity.

### Example 8

### Effect of the GSSG•Pt composite and salts thereof on processes of phosphate modification as well as on content of lymphocytes bearing IL-2 receptors

The molecular mechanisms of the reproduction of the immuno-biochemical effects of the cytokines with the GSSG•Pt composite and the salts thereof were studied.

At the study action of the GSSG•Pt composite and the salts thereof: sodium (Na), lithium (Li) and magnesium (Mg) - was evaluated at the murine model of hemo- and immunodepression induced by single administration of cytostatic cyclophosphamide (CF).

At this study the effect of a five-day long administration of the test articles on the capability of the phosphorylating level of the lymphocyte cytosol proteins on tyrosine and content of the "active" lymphocytes-carriers of IL-2-receptors were evaluated.

Male CBA mice (18 to 20g body weight) were given a single intraperitoneal injection of CP in a dose of 50 mg/kg. After the CF injection the animals were introduced with the tested articles in dose of 5 mg/kg 24 hours later. The tested articles were introduced during five days (daily, once a day). Twenty-four hours after completion of the tested article introduction the mice were euthanized and blood samples were collected to conduct the study.

A fraction of mononuclear leukocyte was obtained by centrifugation in gradient of ficoll-metrizoat (Histopaque, Sigma). Cell concentration was adjusted to 2x10⁶ cells per 1 ml of cell culture medium (RPMI 1640), containing 20 mM HEPES, 2 mM glutamine, 50 mg/mL gentamicin and 10% fetal calf Cell viability was estimated by the Trypan blue exclusion method, then the cell suspension was placed into wells of 96-well microliter plates - 200,000 cells per well.

Content of the lymphocytes-carriers of the IL-2-receptors was determined according to Horgan A. F. (1994) on smears of mononuclear slip. Mononuclear antibodies to chains p55 and p75 of the IL-2-receptor were used as the first antibodies. To reveal the first antibodies the polyclonal rabbit antibodies against murine immunoglobulins marked with were used. Count of the lymphocytes-carriers of the IL-2-receptors was made in percentage to the number of total lymphocytes.

For metabolical marking lymphocytes were cultivated in the Igla medium with addition of 10% cattle serum. The metabolical marking with [32P]ortho-phosphoric acid was performed by cell incubation during 10-12 hrs. in the phosphorusless medium DME containing 100 µCi/ml of [32P]ortho-phosphate. On each sample 0.2 ml of the medium with isotope were added. After incubation cells were destroyed by pipetting and centrifuged at 6000g for 30 min. The obtained supernatant was used for immunoprecipitation with polyclonal antibodies to phosphotyrosine at Fu method (1992). Protein A-sefarose was used for the precipitation of immune complex. The precipitate was washed three times and the precipitate activity was counted on "Gamma" counter.

According to the results of the conducted study (Table 14), it was found that action of the GSSG•Pt composite on the isolated lymphocytes causes (see Table 14) at 10 minute the significant increase of the phosphorylating level on tyrosine of the lymphocyte cytosol proteins is observed, which is the integral indication of the activity of the signal-transducing systems. These changes due to the GSSG•Pt composite action, largely due to the GSSG•Pt composite derivative action determine the modulation of the redox-sensitive gene expression, first of all, immunologically important genes, responsible for the synthesis of the cytokines and hemopoietic factors.

In Table 15 the data is given on phosphorylating level on tyrosine of the lymphocyte cytosol proteins and percentage of the lymphocytes-carriers of IL-2-receptors in CBA-line mice received the tested articles having cyclophosphamide-induced hemo- and immunodepression.

Application of the GSSG•Pt composite salts results in the increase of the percentage of the lymphocytes-carriers of IL-2-receptors and almost normalizing their quantity (normal one is 18.3±1.6%). The similar regularity was found when phosphorylating level on tyrosine of the lymphocyte cytosol proteins was studied.

Application of the GSSG•Pt composite salts results in restoration of the percentage of the lymphocytes-carriers of IL-2-receptors in immunodeficiency conditions modeled by cyclophosphamide. There is the increase of the phosphorylating level on tyrosine of the lymphocyte cytosol proteins of the signal-transducing systems that can be one of the factors of the described immunostimulating actions of the articles tested.

Thus, the example provides evidence of GSSG•Pt to reproduce (imitate) the regulatory effects of the range of cytokines, first of all, IL-2. We are intending to mean the induction by the GSSG•Pt composite of the intracellular mechanisms performing regulatory cytokine signals on the system of immunocompetent and hemopoietic cells as the IL-2 effect reproduction. The conducted studies have shown that changing of the phosphorylating level on tyrosine of the lymphocyte cytosol proteins of the signal-transducing systems of the cells of the organs immunogenesis and hemopoiesis in conditions of cyclophosphamide modeled immunodeficiency causes the effect of the dynamic normalization of the active lymphocyte content.

Therefore, application of the GSSG•Pt composite and derivatives thereof in the form of therapeutically purposed medicinal drugs not only stimulates the cytokine and hemopoietic factor endogenous production but also provides the reproduction of the immune-biochemical cytokine effects, especially in case of receptors desensitization observed mainly in oncological and retro-virus pathology.

### Example 9

### Stimulation of endogenous cytokine production and the therapeutic effect of the GSSG•Pt application in a patient with a stomach cancer, peritoneal metastases, ascites, splenomegaly and cholestatic hepatitis

A 33-year old patient was diagnosed as having stomach neoplasm for more than two years (adenocarcinoma of moderate differentiation degree). In 1993 the patient was operated for malignant stomach ulcer and numerous dense lymph nodes were found in the *porte hepatis* which were considered to be metastases.

In January 1994 the course of chemotherapy (5-FU) was complicated by the severe cholestasis and percutaneous drainage of the left and right liver ducts was undertaken, that six months later was followed by the choledochoejunostomy with changeable transliver drains with Brown's anastomosis.

In November 1995 the patient's state worsened. According to the examinations the patient experienced an active secondary hepatitis. The liver was enlarged and painful and protruded from the costal arch up to 5-6 cm. Blood chemistry indices proved to be persistently abnormal and hardly corrected by the performed treatment: bilirubin - 40.0 due to indirect (up to 31.0); activity of amino-transferases - approximately six times higher than upper normal limit, hypoalbunemia was up to 26%; and there was also hypergammaglobulinemia; hypercholesterolemia was up to 10.2 µmol/l.

During fibrogastrocopy (November, 1995) a stomach cancer located in the middle area of the stomach body and extended about 8 cm was confirmed. The tumor was solid-like type. Stomach walls were rigid. Histology examination defined the tumor as adenocarcinoma of moderate degree differentiation. In December, 1995, the patient had an explorative laparotomy. Ascites was found with plural metastases all over the peritoneum, splenomegaly. The patient's case was identified as inoperable.

A decision was taken to apply GSSG drug form. The drug was injected parenterally (intramuscularly and intravenously), and additionally, the drug form was used via local injections around the tumor tissue through an endoscope. An average doses which were used for intramuscular and intravenous injections - 0.1-0.5 mg/kg, and for local injections - up to 50 mg *in situ.* Parenteral injections of the drug were applied every other day, b.i.d. (intravenous injections at the morning, and intramuscular ones - at the evening), during three weeks, and after that, two times a week during four weeks. The drug introduction through the endoscope was performed once in seven days. Two months after the beginning of the treatment with the drug form used the fibrogastroduodenoscopy showed: esophagus was passable, mucous membrane was pink, cardia rosette was partly closed. On empty stomach moderate amount of foamy secretion was in the stomach, which was intensively colored with bile. The tumor extent was 4.8 cm. At the same time, substantial improvement of hematology and blood chemistry indices was found and the liver size decreased up to 3 cm (below the costal arch).

Six months after the treatment completion (July, 1996) the patient's state worsened significantly. According to the examination the secondary hepatitis relapsed. The liver was increased in size and tender, protruded 4 cm beyond the costal arch. Blood chemistry indices were abnormal and hardly corrected by the performed treatment: bilirubin - 360 due to indirect (up to 28.0); activity of amino-transferases - approximately four times higher than upper normal limit, hypoalbunemia was up to 21%; and there was also hypergammaglobulinemia; hypercholesterolemia was up to 9.42 µmol/l.

At the fibrogastrocopy (July, 1996) a stomach cancer located in the middle area of the stomach body and extended about 6 cm was confirmed. The tumor was solid-like type. Stomach walls were rigid. Histology examination defined the tumor as a moderate degree differentiation adenocarcinoma. In August, 1996, the patient had an explorative laparotomy. Ascites was found with plural metastases all over the peritoneum, splenomegaly.

Considering the previously conducted therapy the decision was taken to apply the new drug GSSG•Pt that is the structural analog of the former administered GSSG drug. The drug was introduced according to the identical regime: parenterally (intramuscularly and intravenously), and additionally, the drug form was introduced via local injections around the tumor tissue through an endoscope. An average doses which were used for intramuscular and intravenous injections - 0.1-0.5 mg/kg, and for local injections - up to 50 mg *in situ*. Parenteral injections of the drug were applied every other day, b.i.d. (intravenous injections at the morning, and intramuscular ones at the evening), during three weeks, and after that, two times in a week during four weeks. The drug introduction through the endoscope was performed once in seven days.

Two months after the treatment initiation with the applied drug: the liver protruded 1 cm beyond the costal arch, tenderless at palpation. According to the ultrasound examination data: there is fibrous tissue at the place of previously determined cancer sites. At the fibrogastrocopy: the esophagus was passable, mucous membrane was pink, cardia rosette was partly closed. The gastric walls are elastic. There was moderate amount of foamy secretion in the stomach with saliva in empty stomach. The tumor extent was 1.5 cm. The duodenum was freely passable. At the same time, substantial improvement of hematology and blood chemistry indices was indicated.

Comparing the therapeutical efficacy of the drugs GSSG•Pt and GSSG using of the former one was found to be advantageous that was manifested by the positive changes at the clinical, biochemical, hematological and immunological indices, fibrogastrocopy data (the tumor size decrease at 75% while applying GSSG•Pt comparing to the 40% decrease after the GSSG administration) (Tables 16, 17). Moreover, at the Table 17 one can see that GSSG•Pt stimulates production of the wider cytokine and hemopoietic factor range having a regulatory influence on their content change.

Thus. the treatment according to the present invention resulted in considerable regress of tumor process with simultaneous obvious beneficial changes in hematology, blood chemistry and immunology parameters, and significant improvement of the life quality.

### Example 10

### Therapeutical efficacy of the GSSG•Pt application for treatment of lung cancer

Patient # 1: Resnikov Eugeniy Fridrihovich

Year of birth: 1938.

Diagnosis: Cancer of the right lung upper lobe.

Histological diagnosis: # 45760 (State Research Center on Pulmonology) - small cell cancer.

Case-history: # 4024.

Complaints on admission: coughing with hard discharged mucous sputum, dyspnea on little exertion.

Objective examination: The patient's state is satisfactory. Peripheral lymph nodes are not enlarged. There are coarse breath sounds weakened at the upper and medium regions of the right lung. There are rare dry rales, dyspnea on the slightest exertion.

Roentgenography (initial data): The upper mediastinum shadow is broadened due to enlarged right paratracheal lymph nodes. The indistinct shadow of the upper right root part is broadened. There is an additional shadow in the peripheral S₃ region of the right lung against the background of marked interstitial changes in both lungs. The right interlobar borders are thickened. Conclusion: there are signs of metastases into lymph nodes of the root and the mediastinum with lymphangoitis sings.

Treatment course: there were applied three immunochemotherapy courses using GSSG•Pt drug.

After the treatment: the patient's state has improved significantly: the mild weakness is still present, there is no dyspnea.

Objective examination: The state is satisfactory. The peripheral lymph nodes are not enlarged. There is a weakened breath sounds in the medium departments of the right lung. The breath sounds in other departments are vesicular. There are no rales.

Roentgenography (after the treatment performed): The lung fields are particularly clear. There are mild infiltrative signs at the S₃ department of the right lung. The roots are not enlarged. There are no additional formations at the right root projections. The upper mediastinum shadow is not enlarged. The solitary paratracheal lymph nodes can be determined.

**Patient # 2: Semyonov Petr Alexandrovich**

**Year of birth: 1945.**

Diagnosis: Right lung cancer, hepatic metastases.

Histologic diagnosis: # 45998, small cell cancer

Case-history: # 4076

Complaints on admission: coughing with hard discharged mucous sputum, dyspnea on mild exertion, weakness, constant pains in the lumbar region extending to stomach. During last six months the patient lost 6 kg.

Objective examination: The state is medium severe. Scleras are icteric. The breathing sounds are coarse, weakened in the right lung upper and medium departments. In the right supraclavicular zone one can palpate enlarged lymph nodes (solid consistency, hardly movable, size - 3.0 and 1.0 cm, painless). At auscultation there are coarse breathing sounds, weakened in the medium departments in the right. There are solitary dry rales, dyspnea on the slightest exertion. The liver protruded 3.5 cm over the costal arch.

At examination there were found: middle-lobe bronchus cancer, middle lobe hypoventilation, pneumonitis. At ultrasound examination - there are sings of metastatic liver impairment.

Roentgenography (initial data): The right lung middle lobe is decreased in size (state of hypoventilation). Against the background of the increased lung pattern there is intensive, almost homogenous infiltration with distinct margin along the horizontal interlobar pleura. The right root cannot be determined. The upper and lower lobes of the right lung and the left lung are without any particular features. The mediastinal organs are not noticeably displaced.

Treatment course: there were applied three immunochemotherapy courses using GSSG•Pt drug.

After the treatment: the patient's state has improved significantly: there are no weakness and dyspnea, appetite has appeared, he gained 5 kg. The blood indices have restored.

Objective examination: The state is satisfactory. The peripheral lymph nodes are not enlarged. There is a weakened breath sounds in the medium departments of the right lung. The breath sounds in other departments are vesicular. There are no rales.

According to the liver ultrasonography and CAT scan data: there is full mass process regression.

Roentgenography (after the treatment performed): There is the insignificant atelectasis of the middle lobe at the thoracic X-rays picture. The roots are structural and not enlarged, the right one is slightly displaced downward. The heart is not enlarged in size.

Comparing to the initial data there is considerable positive development: at the right the pulmonary tissue has become more transparent (reduction of the hypoventilation signs), there are no infiltrative shadows.

### Conclusion:

Evaluating therapeutical effectiveness of the GSSG•Pt drug the following was found:
1. Clinical restoration of indices (general state improvement, pathologic absence of symptoms, body weight gaining);
2. Roentgenologic picture changes (pulmonary tissue transparency increase, infiltrative absence of shadows, disappearing of atelectasis and hypoventilation);
3. Hematologic restoration of indices (increase of the erythrocyte count and hemoglobin content, restoration of white blood count);
4. Changes of the ultrasound and CAT scan data (full tumor process regression);
5. Immune indices restoration and increase of the CD16⁺, CD25⁺ counts indicating restitution of the antitumor surveillance system.
6. Induction of the wide cytokine range synthesis as well as modulation of their content mutual regulation (correlation of the content changes of IL-1β, IL-4, TNF-α).

Thus, in the given clinical Examples it has been demonstrated that the GSSG•Pt drug application has provided faster restoration of clinical, roentgenologic, hematologic and immune indices ensuring more effective restitution of the immunity and hemopoiesis systems. The aforementioned indicates the tumor process regression that, eventually, calls out significant increase of the patient's quality of life.

### Example 11

### Therapeutical efficacy the GSSG•Pt application for treatment of chronic viral hepatitis B (CHBV)

Patient No 1: Kupchenko Vladimir Mikhailovich

Date of birth: 1945

Diagnosis: CHBV, replicative phase (PCR HBV+) with moderate activity grade.

Case-history: # 1068.

Complaints at admission: weakness, discomfort under the right costal arch, nausea, no appetite.

Anamnesis morbi: during last six years the patient noted periodically appeared dull pains under the right costal arch, weakness, urine color changes. At examination there was found: hyperbilirubinemia up to 34 µmol/L, ALT increase to 5.4 mmol/hr.L. CHBV serologic markers and PCR HBV(+) were determined at the hospital examination.

Objective examination: The patient's state is satisfactory. The liver protruded 2 cm beyond the costal arch. The liver margin is firm and tender.

Previous treatment was not performed.

Treatment course: There was performed the treatment course with administration of the GSSG drugs according to the regime.

State after the performed treatment course: there were noted the following positive changes - significant general state improvement, no weakness and nausea, diminution of the discomfort sensation. The liver protruded 0.5 cm beyond the costal arch. The liver margin is soft and tenderless.

Patient No.2: Lukashenko Igor Matveyevich

Year of birth: 1964

Diagnosis: CHBV, replicative phase (PCR HBV+), moderate activity degree.

Case-history: # 1043.

Complaints on admission: considerable weakness, no appetite, sweating, urine darkening.

Anamnesis morbi: The patient feels sick beginning from January 1996 when for the first time there appeared dull pains under the right costal arch, temperature increase up to 38.7⁰ C, vomiting, urine darkening. Acute viral hepatitis B was diagnosed and confirmed serologically. The patient was administered with detoxicating and antibacterial therapy. However, afterwards there were observed increased Hbs Ag and ALT values, persistent viral replicative activity. Being examined at the last time there were found: hyperbilirubinemia to 78 µmol/L, ALT increase to 6.2 mmol/hr.L. CHBV serological markers and PCR HBV(+) were determined at the hospital examination.

Objective examination: The general state is satisfactory. Skin and scleras are icteric. The liver protruded 2.5 cm beyond the costal arch. The liver margin is firm and tender.

Previous treatment: from 17.09.97 the patient was administered with acyclovir during 21 days. After the course completion there were increased ALT - up to 2.1 mmol/hr.L, bilirubin to 32 µmol/L. The Hbs Ag degree did not change. The patient's .state was defined by the significant asthenic-vegetative syndrome.

Treatment course: There was performed the treatment course with administration of the GSSG•Pt drugs according to the regime.

State after the performed treatment course: There were noted the vivid following positive changes - significant general state improvement, no weakness, sweating and nausea. The skin and scleras were not icteric. The urine color became normal, diminution of the discomfort sensation. The liver protruded 0.5 cm beyond the costal arch. The liver margin became softer and tenderless.

Comparison (Tables 20, 21):

At the comparative analysis on the therapeutical efficacy of the GSSG•Pt and GSSG drugs the former was shown to be advantageous that was manifested by the following:
1. Biochemical indices normalization (ALT and bilirubin decrease);
2. Significant HBs Ag decrease and replication termination;
3. Immune indices normalization and increase of the CD95⁺ content indicating the apoptosis process activation in the virus-transformed cells;
4. Considerable regulation for the wider cytokine range.

### Example 12

### Therapeutical efficacy the GSSG•Pt application for treatment of acute viral hepatitis B (AHBV)

Patient's full name: Minina Oksana Alexandrovna.

Sex: female.

Age: 20.

In-patient card: 678

Diagnosis: Acute viral hepatitis B (HBs Ag "+"), replicative phase (PCR HBV "+"), prolonged form; chronic viral hepatitis D, replicative phase (PCR HDV "+").

Complaints during examination: weakness, appetite decrease.

Anamnesis morbi: The patient felt sick in August 1997, when she noticed sharp weakness, malaise, back bone aching, temperature raising up to 38.8° C. Dark urine and sclera icterus appeared 10 days later. The patient was admitted to the viral infectious clinic, where she received course of the detoxicating, spasmolytic, antibacterial therapy. However, replicative viral activity and increased GPT level were still present. The prolonged cytolytic syndrome gave foundation for administration of the drug GSSG•Pt.

Previous treatment was not performed.

Treatment with GSSG•Pt drugs: from 30.10.97 to 23.11.

Patient's state after the treatment course completion:

The patient's state is satisfactory. She noted the appetite increase, weakness reduction.

Conclusion (Tables 22-24):

The immunomodulating course with the GSSG•Pt drugs has provided the following positive changes: biochemical indices normalization; termination of HBV and HDV replication; termination of HBsAg persistency; virus-infected cell apoptosis induction; general state improvement; stable therapeutic effect.

### Example 13

### Therapeutical efficacy the GSSG•Pt application for treatment of chronic viral hepatitis C (CHCV)

Patient's full name: Zubov Vitaliy Valerievich.

Sex: male.

Age: 18.

Patient's case: No. 1043

Diagnosis: Chronic viral hepatitis C, replicative phase (PCR HCV "+"), moderately manifested activity; chronic viral hepatitis B, integrative phase (PCR HBV "-"); narcotic intoxication, narcomania.

Complaints during examination: weakness, pains at the right under the ribs, at knee-joints, the backbone and wrist joints.

Anamnesis morbi: The patient noticed pains in the knee-joints and the backbone at the beginning of August, 1997. On blood test an increase of the bilirubin level up to 34 µmol/L and GPT level up to 2.1 mmol/hr.L were found. During examination in the hospital from August 15, 1997, anti-HCV IgG and the replicative activity of the hepatitis C virus were found.

Anamnesis vitae: The patient started using drugs at 14. To the examination time he uses up to 2 g of heroin per day. He is at the state of the narcotic abstinence.

Previous treatment was not conducted.

Immunomodulating therapy course with GSSG•Pt drugs: from August 15, 1997, to September 7, 1997.

Patient's state after the treatment course completion: The patient's state is satisfactory. He noticed significant reduction of weakness, no pains in the right under the ribs and in the joints. As patient said the narcotic abstinence state diminished almost without pain and in less time. Biochemical indices normalization and absence of the viral replicative activity was marked.

Conclusion: The immunomodulating therapy course with the GSSG•Pt drug provided positive changes, which were indicated by: biochemical and serologic indices normalization; termination of HCV replication. Immune indices and cytokine status parameters correlate to the infectious process controlling and viral replication absence. Examination of the patient's peripheral blood lymphocytes by liquid chromatography with monoclonal antibodies to FasAg (CD95⁺) after the treatment revealed the CD95⁺ cell increase indicating activation of the programmed cell death process in virus-infected cells. At supervision at one and three months after the treatment stabilization of this state was noted.

Concomitant drug intoxication and an abstinence state at application of the GSSG•Pt drugs were corrected faster and were less excruciating for the patient.

The immunomodulating course with the GSSG•Pt drugs for chronic hepatitis C with replicative activity and concomitant drug intoxication has provided the following results:
- biochemical indices normalization in blood;
- hematological indices normalization;
- termination of HCV replication;
- normalization of the immune blood indices and cytokine status;
- apoptosis process induction in the peripheral blood lymphocytes;
- rapid correction for the drug abstinence state;
- stable therapeutic effect

### Example 14

### Comparative analysis of the GSSG and GSSG•Pt effects on growth development and apoptotic DNA degradation at normal and transformed cells

The GSSG and GSSG•Pt effects on growth development and apoptotic DNA degradation at normal and transformed (HL-60) cells were comparatively analyzed. To that end, GSSG and GSSG•Pt were incubated for 24 hours with HL-60 myeloid line cells and normal human lymphocytes obtained from healthy volunteers' blood.

Venous blood of healthy volunteers was collected into heparinized test-tubes, which had been tested for endotoxin. A mononuclear fraction of blood leukocytes was obtained by centrifugation in ficoll-metrizoat gradient (Histopaque, Sigma). Cell concentration was adjusted to 2x10⁶ cells per 1 ml of cell culture medium (RPMI 1640), containing 20 mM HEPES, 2 mM glutamine, 50 mg/mL gentamicin and 10% fetal calf serum. Cell viability was estimated by the Trypan blue exclusion method, then the cell suspension was placed into wells of 24- and 96-well microliter plates - 250,000 cells per well.

The HL-60 myeloid line cells were grown in RPMI-1640 medium with addition of 10% fetal calf serum. Cultivation was carried out in closed flasks, the medium volume was 12 mL, it was replaced every four days. Directly before testing the cell suspension in the fresh medium was brought into 24-well microliter plates (cell concentration for each well was 250.000 cells per well) and the tested articles - GSSG and GSSG•Pt - were added into the corresponding wells up to final concentration 100 µg/mL.

The effect testing for the testing articles was performed 24, 48 hours after addition into the culture.

The analysis procedure involved the following: after 24-48 hour incubation there were calculated the total cell count and the dead cell count by the Trypan blue exclusion method; afterwards the cell suspension was centrifuged (at 12.000 g, in Eppendorf test-tubes during 10 min). The cell pellet was frozen and kept at -70 °C before the DNA separation. The DNA separation was conducted by Kirbi-Georgiyev phenol method. To the cell pellet there were added 0.5 ml of 10% SDS and 0.5 ml of TE-buffer containing 0.1M EDTA and 0.01 M Tris-HCl with pH 8.0 ("A" buffer), and the pellet was resuspended by the tube shaking during 15 min. Then the equal phenol amount was added adjusted by 0.01 M Tris-HCl with pH 8.0, the product was mixed during two min. and centrifuged in the Eppendorf test-tubes at 12.000 g during 15 min. After the centrifugation completion the upper water phase was phenol-treated one more time. After the phenol treatment the upper water phase was twice treated with phenol-chloroform mixture (1:1) that was mixed with the water phase in equal amount. The water phase was separated by centrifugation at 12.000 g during 10 min., pumped out and once mixed with the equal chloroform amount. Afterwards it was centrifuged at 12.000 g, the upper phase was separated, mixed with double amount of distilled ethyl alcohol frozen to -20 °C and kept for one night at -20 °C. The DNA pellet was gathered by centrifugation at 12.000 g during 10 min., the supernatant was removed and the pellet was washed by 200 µL of 70% ethyl alcohol frozen to -20 °C during five min., centrifuged one more time at the same conditions and afterwards the pellet was air-dried during one hour. Then it was diluted in 10 µL of the A buffer; the DNA amount was determined by Dishe method and electrophoresis was performed in 2% agarose gel (agarose with NA grade made by "Pharmacia LKB, Biotechnology Inc" (Austria) was used). The DNA electrophoretic separation was made in a block of 2% agarose gel in a device made by "Pharmacia LKB, Biotechnology Inc" (Austria). As a buffer solution 0.04 M tris-HCl buffer, pH 7, containing 0.02 M of sodium acetate and 0.02 M EDTA was applied. Agarose (2%) was prepared at an electrode buffer. The DNA samples (2-5 µg) were placed into the gel slots. The electrophoresis was conducted with an electrical field intensity of 6 W/cm during three hours. The sample propulsion was observed due to bromine-phenol blue motion. On the electrophoresis completion the gel block was taken out of the device and introduced into a tray with etidium bromide solution (3 µg/mL H₂O) for 30 minutes in dark place. After the incubation completion the gel was rinsed with water and examined in the transmitted ultraviolet radiation with the wave-length 254 nm at a transilluminator made by "Pharmacia LKB, Biotechnology Inc" (Austria). The gel was photographed by Zenit E camera with a red colour filter.

The study results are given in the Tables 28 and 29 and Figs. 13 and 14. As one can see in the Tables 28 and 29 data, the GSSG and GSSG•Pt influence on normal and transformed cells is of an alternative character. GSSG and GSSG•Pt stimulated the normal cell proliferation (Table 28). The electrophoresis of DNA obtained from the normal cells (Table 28) revealed presence of only traceable quantities for apoptotic fragments at the background of homogenous high-molecular fraction characteristic for the viable cells.

In the contrary, in the myeloid origin (HL-60) cancer cell culture, the apoptosis activation and the cell division inhibition were observed due to the influence of both drugs (Table 29, FIG. 14). The diversities of the effects were of the quantitative nature.

Thus, the dead HL-60 cell quantity after the GSSG impact was reliably lower than the one after the GSSG•Pt effect (Table 29). An evident indication for stronger impact efficacy (GSSG•Pt comparing to GSSG) on the HL-60 cells is the apoptotic fragment's character obtained after the electrophoretic analysis. The DNA electrophoresis of the HL-60 cells non-incubated with the drugs demonstrated presence of highly-molecular, practically homogenous DNA characteristic for the viable cells (FIG. 14, band #2). The DNA electrophoresis of the cells incubated during 24 and 48 hours with the GSSG•Pt and GSSG drugs revealed the DNA oligonucleosomic degradation (FIG. 14, bands 1 and 3, respectively), i.e., an apoptotic ladder, that is indisputable sign for the programmed cell death. However, the apoptotic ladder for the HL-60 cells treated with GSSG contained considerable amount of the high molecular DNA characteristic for the viable HL-60 cells (FIG. 14, band #1), whereas the high molecular DNA for the cells treated with GSSG•Pt was virtually absent (FIG. 14, band #3).

The HL-60 cells are defective in the p53 gene (p53 gene deletion) and apoptosis induction through the GSSG and GSSG•Pt treatment can occur only without involving the p53 product. Therefore, one can state that the GSSG and GSSG•Pt drugs activate an inner contour of the programmed cell death irrespectively of the p53 gene product. The p53 defect is present approximately at half of cancer pathology cases. The experimental results appeared to denote efficacy of GSSG and, especially, GSSG•Pt for the tumor chemotherapy.

**Conclusion:** The performed studies allowed to obtain data indicating capacity of the GSSG and GSSG•Pt drugs to increase normal cell (lymphocytes) viability and, contrariwise, to induce apoptosis in transformed cells, i.e., exercise an antitumor activity. Besides, the GSSG•Pt activity regarding to the transformed cell apoptosis induction excelled significantly the one for the GSSG drug according to the objective viability criterion, i.e., presence of the high. molecular DNA that appeared considerably after the GSSG treatment and was almost absent after the GSSG•Pt treatment. Thus, the data obtained on cells of the myeloid line HL-60 defective in the p53 gene that is the crucial agent in apoptosis induction allows us to state that:
The GSSG and GSSG•Pt drugs induce the inner contour of the apoptosis development regardless of the p53 gene product;
The GSSG•Pt drug based on composite possesses higher chemotherapeutic activity (due to the apoptotic induction in the transformed cells) versus GSSG.

### Example 15

### Analysis of the GSSG•Pt effects on growth development and apoptotic DNA degradation at normal and transformed cells defective in the p53 antioncogene with increased ras-gene expression

The GSSG•Pt effects on growth development and apoptotic DNA degradation of different lineage of transformed cells (HL-60, C-8, A-4) were comparatively analyzed depending on the p53 defect that is the key factor for the apoptosis development and the ras-gene that is a multipotent factor for cell reaction. The HL-60 cell culture is human cells of myeloluekosis origin defective in the p53 gene. Cell cultures C-8 and A-4 are transformed murine fibroblasts having a plasmid with the ras gene and a gene of the Ela expression enhancement factor that is an adenovirus antigen fragment. At that, the A-4 cells are defective at the p53 gene and the C-8 ones contained the intact p53 gene. A donor blood lymphocyte slip was used as a control of the human cells with the intact p53 gene.

The HL-60 (p53--) myeloid line cells were grown in RPMI-1640 medium with addition of 10% fetal calf serum. The cell suspension cultivation was carried out in closed flasks, the medium volume was 12 mL, it was changed every four days. Directly before testing the cell suspension in the fresh medium was brought into 24-well microliter plates (cell concentration for each well was 250.000 cells per well) and the tested article - GSSG•Pt - was added into the corresponding wells up to final concentrations 10-100 µg/mL.

Venous blood of healthy volunteers was collected into heparinized test-tubes, which had been tested for endotoxin. A blood leukocyte mononuclear fraction was obtained by centrifugation in ficoll-metrizoat gradient (Histopaque, Sigma). Cell concentration was adjusted to 2x10⁶ cells per 1 ml of cell culture medium (RPMI 1640), containing 20 mM HEPES, 2 mM glutamine, 50 mg/mL gentamicin and 10% fetal calf serum. Cell viability was estimated by the Trypan blue exclusion method, then the cell suspension was placed into wells of 24- and 96-well microliter plates - 250,000 cells per well.

The murine transformed fibroblasts were grown in DMEM medium with addition of 10% fetal calf serum. The cell suspension cultivation was carried out in closed flasks, the medium volume was 12 mL, and it was changed every four days. Directly before testing the cell suspension in the fresh medium was brought into 24-well microliter plates (cell concentration for each well was 50.000 cells per well) and the tested article - GSSG•Pt - was added into the corresponding wells up to final concentrations 10-100 µg/mL.

The effects testing for the testing article was performed 24, 48 hours after introduction into the culture.

The analysis procedure involved the following: after 24-48 hour incubation, the total cell count and the dead cell count were calculated by the Trypan blue exclusion method; afterwards the cell suspension was centrifuged (at 3.000 g, in Eppendorf test-tubes during 10 min). The cell pellet was frozen and kept at -70 °C before the DNA separation. The DNA separation was conducted by Kirbi-Georgiyev phenol method. To the cell pellet there were added 0.5 ml of 10% SDS and 0.5 ml of TE-buffer containing 0.1M EDTA and 0.01 M Tris-HCl with pH 8.0 ("A" buffer), and the pellet was resuspended by the tube shaking during 15 min. Then the equal phenol amount was added adjusted by 0.01 M Tris-HCl with pH 8.0, the product was mixed during two min. and centrifuged in the Eppendorf's test-tubes at 12.000 g during 15 min. After the centrifugation completion the upper water phase was phenol-treated one more time. After the phenol treatment the upper water phase was twice treated with phenol-chloroform mixture (1:1) that was mixed with the water phase in equal amount. The water phase was separated by centrifugation at 12.000 g during 10 min., pumped out and once mixed with the equal chloroform amount. Afterwards it was centrifuged at 12.000 g, the upper phase was separated, mixed with double amount of distilled ethyl alcohol frozen to -20 °C and kept for one night at -20 °C. The DNA pellet was gathered by centrifugation at 12.000 g during 10 min., the supernatant was removed and the pellet was washed by 200 µL of 70% ethyl alcohol frozen to -20 °C during five min., centrifuged one more time at the same conditions and afterwards the sediment was air-dried during one hour. Then it was diluted in 10 µL of the A buffer; the DNA amount was determined by Dishe method and electrophoresis was performed in 2% agarose gel (agarose with NA grade made by "Pharmacia LKB, Biotechnology Inc" (Austria) was used). The DNA electrophoretic separation was made in a block of 2% agarose gel in a device made by "Pharmacia LKB, Biotechnology Inc" (Austria). As a buffer solution 0.04 M tris-HCl buffer, pH 7, containing 0.02 M of sodium acetate and 0.02 M EDTA was applied. Agarose (2%) was prepared at an electrode buffer. The DNA samples (2-5 µg) were placed into the gel slots. The electrophoresis was conducted with an electrical field intensity of 6 W/cm during three hours. The sample propulsion was observed due to bromine-phenol blue indicator motion. On the electrophoresis completion the gel block was taken out of the device and introduced into a tray with etidium bromide solution (3 µg/mL, H₂O) for 30 minutes in dark place. After the incubation completion the gel was rinsed with water and examined in the transmitted ultraviolet radiation with the wave-length 254 nm at a transilluminator made by "Pharmacia LKB, Biotechnology Inc" (Austria). The gel was photographed by Zenit E camera with a red color filter.

The study results are given in the Tables 30, 31, 32, 33. As is evident from the Table 31, GSSG•Pt induces apoptosis in the HL-60 cell culture defective at the p53 gene. The effect was rather developed at the concentration of 10, however, it was more evident at 100 µg/mL. Also there were observed conglomeration of DNA apoptotic fragments multiple of DNA nucleosome size that is the indisputable sign of programmed cell death.

In contrary, GSSG•Pt made stimulating effect on normal cells, i.e., there was certain proliferation observed (Table 30). Electrophoresis of DNA obtained from normal cells exhibited that it was represented by a homogenous high-molecular fraction characteristic for viable cells.

Thus, differences of the drug action on normal and transformed cells were basically divergent. Mechanism for the GSSG•Pt divergent action might be conditioned by activation of the p53-independent apoptotic pathway through the ras-signal-transducing system. The ras-system is capable to stimulate cell proliferation and differentiation through the mitogenic factor cascade and induce apoptosis (programmed cell death) through another cell signal cascade.

To check it there were compared the GSSG•Pt effects on murine fibroblasts with enhanced ras-gene expression but different in the intact p53 gene (wild type) presence - cells C-8(p53++), or the p53 gene absence (a genetic defect) - (cells A-4(p53--)). As one can see from the Tables 32 and 33, the GSSG•Pt effect appeared in both cell lineages. The apoptosis induction was significantly exhibited. It indicates activation of the p53-independent apoptotic pathway. Presence of the activated ras gene in both of the cell lineages can be an explanation for the apoptotic exhibition that was even more marked in the transformed fibroblasts than in the HL-60 cells. It confirmed the apoptosis induction through the ras-signal-transducing system. The GSSG•Pt effect on the A-4 and C-8 cells was not different at concentration of 10 µg/mL. The significantly superior GSSG•Pt effect on the A-4 cells comparing to the C-8 cells was noted at the concentration 100 µg/mL. The p53-protein absence appeared even to enhance the ras-signal-transducing pathway for apoptosis induction in tumor cells.

The p53 gene defect occurs in approximately half of the cancer disease cases. The results of these experiments can imply the GSSG•Pt effectiveness for chemotherapy of these tumors.

**Conclusion:** The data from these studies indicate the capacity of the GSSG•Pt drug to increase normal cell (lymphocytes) viability and, contrariwise, to induce apoptosis in transformed cells, i.e., exercise an antitumor activity. Besides, the GSSG•Pt activity regarding to the transformed cell (defective at the p53 gene) apoptosis induction even excelled the one for the cells with the intact p53 gene at the high drug concentration. According to the objective viability criterion, i.e., presence of the high molecular DNA that appeared in considerable amounts after the GSSG•Pt treatment of the normal donor cells (take from the lymphocyte slip), the cell death was practically absent whereas in case of the GSSG•Pt impact on transformed cells there was observed the DNA apoptotic degradation, i.e., the sign of the irreversible apoptotic death induction even in the cells defective at the p53 gene (with the stimulation especially). Previously, in the Example 9 there was shown activation of the cytokine range after the GSSG•Pt treatment. Considering that the cytokine action may be determined with the ras-signal-transducing pathway activation the cytokine stimulation cam also cause the antitumor effect through an interaction with the ras-protein.

### Example 16

### Analysis of the GSSG•Pt effects on growth development and apoptotic DNA degradation at murine transformed cells cultures defective at antioncogenes

The GSSG•Pt effects on growth development and apoptotic DNA degradation of transformed fibroblasts of a cell lineage with activated ras-gene but an intact p21 gene (p21++; C-8 cells) and murine cell lineage with knockout p21 gene (p21--).

The p21++ lineage cells are murine transformed fibroblasts having a plasmid with the ras gene arid a gene of the Ela expression enhancement factor that is an adenovirus antigens fragment but have intact p53 and p21 genes. The p21 (--) lineage has the intact p53 and ras genes but it is defective at the p21 gene. It allows evaluating the GSSG•Pt impact on the apoptosis induction in conditions of impaired regulation for the cell division G1 phase.

The cell cultures were grown in DMEM medium with addition of 10% fetal calf serum. The cell suspension cultivation was carried out in closed flasks, the medium volume was 12 mL, it was changed every four days. Directly before testing, the cell suspension in the fresh medium was brought into 24-well microliter plates (cell concentration for each well was 50.000 cells per well) and the tested article - GSSG•Pt - was added into the corresponding wells up to final concentrations 100 µg/mL.

The effect testing for the testing articles was performed 24, 48 hours after introduction into the culture.

The analysis procedure involved the following: after 24-48 hour incubation, the total cell count and the dead cell count were calculated by the Trypan blue exclusion method; afterwards the cell suspension was centrifuged (at 3.000 g, in Eppendorf test-tubes during 10 min). The cell pellet was frozen and kept at -70 °C before the DNA separation. The DNA separation was conducted by Kirbi-Georgiyev phenol method. The cell lysis was performed by addition of 0.5 ml of 10% SDS. The equal phenol amount was added there adjusted by 0.01 M Tris-HCl with pH 8.0, the product was mixed during two min. and centrifuged in the Eppendorf test-tubes at 13.000 g during 15 min. The phenol, deproteinization was repeated twice. Afterwards the water phase was twice treated with phenol-chloroform mixture (1:1) and once with chloroform. Nucleic acids were precipitated by the addition of two volumes of 96% ethyl alcohol at 20□C overnight. The DNA was gathered by centrifugation at 13.000 g during 30 min., decanted, washed with 70% ethyl alcohol and air-dried. After the drying the precipitate was dissolved in TE buffer. The DNA concentration was determined in the obtained solution (by Dishe method). Fractional content of the nucleic acids was determined by electrophoresis in 2% agarose gel (agarose with NA grade made by "Pharmacia LKB, Biotechnology Inc" (Austria) was used). The DNA electrophoretic separation was made in a block of 2% agarose gel in a device made by "Pharmacia LKB, Biotechnology Inc" (Austria). The electrophoresis was performed in TAE buffer pH 7.4 (0.04 M tris, 0.02 M of sodium acetate, 0.02 M of EDTA) at an electrical field intensity of 6 W/cm during three hours. The sample propulsion was observed due to bromine-phenol blue indicator motion. The electrophoresis results were examined in the transmitted ultraviolet radiation (λ=254 nm) at a transilluminator made by "Pharmacia LKB, Biotechnology Inc" (Austria) after the gel dying with etidium bromide solution (5 µg/ml).

The study results are given in the Tables 34, 35. As it is evident from the Table 34, GSSG•Pt induces apoptosis in the p21++ cell culture with activated ras-gene manifested by conglomeration of DNA apoptotic fragments multiple of DNA nucleosome size.

In the p21 (--) cells having non-active ras-signal-transducing system but defective at the control of the cell cycle delay in the G1 phase GSSG•Pt induced apoptosis to less extent that in p21++ cells (Tables 34, 35). It might be implicated by the fact that the ras-system is capable of stimulating cell proliferation and differentiation through the mitogenic factors' cascade and inducing apoptosis (programmed cell death) through another cell signals' cascade. Absence of the p21 gene expression can cause changes in interrelations of specific cascades of the ras-signal-transducing pathway and, thereupon, lessen the apoptotic stimulation by the drug in cases of p21 gene defects.

The p21 gene defect does not often occur in oncological diseases that considering the performed experiments' issues can imply the GSSG•Pt effectiveness for chemotherapy of these tumors except cases with lower effectiveness at tumors with mutations in p21 gene.

Conclusion: The performed studies allowed obtaining data indicating capacity of the GSSG•Pt drug to induce apoptosis actively in transformed cells, i.e., exercise an antitumor activity. The enhanced ras-gene expression facilitates the apoptosis induction in the transformed cells C-8 indicating implementation of the GSSG•Pt antitumor activity through the ras-signal-transducing system. The lesser drug effect in case of p21 gene expression absence can be determined by the redistribution of factors in mitogenic and apoptotic cascades of the ras-signal-transducing pathway.

Nevertheless, presence of the DNA apoptotic degradation, i.e., the sign of the irreversible apoptotic death induction, is found after the GSSG•Pt action even in p21-defective cells.

The aforesaid allows us to state that the GSSG•Pt composite-based drug:
- Realizes chemotherapeutic activity regarding the tumor-transformed cells through induction of the ras-dependent apoptotic pathway;
- Induces the apoptosis' development in the tumor-transformed cells including p21-defective cells.

### Example 17

### Therapeutical effect of the GSSG•Pt vanadium salt in patient with diabetes mellitus

Patient: Bronavetz Lidia Sergeyevna.

Gender: female

Age: 37

Out-patient card: # 63

Diagnosis: Diabetes mellitus. Insulin-independent type - type II. Diabetic angiopathy, grade IV.

Anamnesis morbi: Firstly the high blood sugar was revealed in the age of 31. In October 1994, the patient was admitted into the Endocrinologic Department of the Hospital # 16. There were diabetes cases in the patient's hereditary history on the mother's side. The disease developed gradually, the blood glucose level fluctuated from 12.1 to 15.7 mmol/L, glucosuria to 7 %.

Previous treatment: At the onset of the disease, the patient was frequently admitted to hospitals; in 1994 during six months she was administered with Insulin-lente S.N.C.-32 IU and peroral hypoglycemic agents of the sulfonylureas group; then Insulin was cancelled and the patient used antidiabetic agents - Bucarban, Diabeton. The blood glucose changed from 10.7 to 15.4 mmol/L, glucosuria 3-7%.

In January 1998 the patient was taken to the hospital and the following treatment regime was administered: Diabeton 0.08 g - 2 tab twice a day, Glucobay 0.1, 3 times per day.

The blood glucose content:

| | |
|---|---|
| 9.00 | 12.6 mmol/L |
| 12.00 | 12.0 mmol/L |
| 14.00 | 15.3 mmol/L |
| 17.00 | 19.1 mmol/L |
| 6.00 | 11.5 mmol/L |

Glucosuria - to 3%.

Because of the previous treatment inefficacy, it was decided to use the drug of the GSSG•Pt vanadium salt.

**Hospital treatment regime for the GSSG•Pt vanadium salt:** from 17.01.98.

During 10 days once a day - intravenous injections of the GSSG•Pt vanadium salt (daily dose - 0.01-0.5 mg/kg).

During the following 10 days - intravenous injections of the GSSG•Pt vanadium salt every other day (daily dose - 0.01-0.5 mg/kg).

During the following 10 days (the third decade) - intramuscular injections of the GSSG•Pt vanadium salt once a day (daily dose - 0.01-0.5 mg/kg).

The GSSG•Pt vanadium salt treatment was performed along with changed treatment regime by Diabeton and Glucobay. Diabeton was administered 0.08 twice a day, Glucobay 0.05, 3 times a day.

The blood glucose restored to normal values and did not exceed 8.2-10.6 mmol/L after meals. After the discharge the patient received the GSSG•Pt vanadium salt treatment as an out-patient during one month.

### Ambulant treatment regime for the GSSG•Pt vanadium salt:

For one month there were administered intramuscular injections of the GSSG•Pt vanadium salt once a day (daily dose - 0.01-0.5 mg/kg).

During two sequential months of treatment with the GSSG•Pt vanadium salt drug there were noted two episodes of the glucose content decrease to 1.5 - 2.5 mmol/L, thereat the antidiabetic drugs dosages were lowered. After two months of the treatment, Glucobay was cancelled.

**Patient's state after the treatment course completion:** After four months of the treatment with administration of the GSSG•Pt vanadium salt drug as a support for the basic therapy, the patient's general state improved significantly and was evaluated as satisfactory. Development of hematologic, biochemical, immunologic blood indices is provided in the Tables 36, 37.

Comments to the indices development of glucose, cAMP/cGMP, thiol-disulfide ratio and other analyzed parameters.

The glucose content development is an integrative criterion for the impact effectiveness of the antidiabetic drugs. The combined therapy by the peroral antidiabetic drugs (Diabeton and Glucobay) along with the GSSG•Pt vanadium salt drug called forth the blood glucose normalization, decrease of the Diabeton therapeutic dosage and the Glucobay cancellation. Character of the hematologic, biochemical and immune parameter changes indicated restoration of the metabolism and the systemic reactions in general that does not allow to determine the antidiabetic constituent for the GSSG•Pt vanadium salt drug. The following indices were used - cAMP/cGMP and thiol-disulfide ratio (TDR) - that allowed to characterize basically a molecular mechanism of the GSSG•Pt vanadium salt drug action on cellular reaction complex stabilizing the blood glucose level. In particular, the key intracellular messengers - cAMP and cGMP - determine intensity of the glucose flow into intracellular metabolic processes. At that, cGMP-dependent enzymatic cellular systems determine the cellular glucose intake intensity. In its turn, the cGMP level is determined by the cellular oxidative potential. In particular, oxidants increase the cGMP level, in the contrary, antioxidants perform depressive action on its content. Thus, the intracellular thiol-disulfide ratio (TDR) reflecting the balance of the anti- and pro-oxidants determines value of the intracellular cAMP/cGMP index. Taking into account an organism as a whole we can analyze these indices in blood as integrative internal medium interrelated with the glucose level fluctuations, because the glucose metabolism is an indefeasible constituent for functioning of all cell types at our organism along with thiol-disulfide metabolism and cyclic nucleotide systems.

The results obtained indicated the glucose-lowering influence of the GSSG•Pt vanadium salt drug. Besides, this effect follows the cGMP content increase development (lowering of the cAMP/cGMP index) and the TDR value decrease (increase of the thiol oxidized forms). Considering regulatory possibilities of the oxidized thiol forms and the cGMP in blood glucose regulation, one can state the basic mechanism of the regulatory hypoglycemic effect of the GSSG•Pt vanadium salt drug. Inter alia, the regulatory impact of the GSSG•Pt vanadium salt drug on the cellular redox contour, provides an increase of the oxidant constituent level that, in its turn, redistributes balance in the cyclic nucleotide system in favor of cGMP (guanylat-cyclase induction and inhibition of phosphodiesterase, cGMP synthesis and destruction enzymes, respectively). The cGMP regulatory impact stimulates the glucose transport processes into insulin-dependent tissues calling forth the blood glucose decrease.

**Conclusion:** Application of the GSSG•Pt vanadium salt drug in the scheme for the combined diabetes treatment allowed to obtain the following therapeutic effects:
- the quality of life improvement and the blood glucose level stabilization;
- dosage decrease for the administered glucose-lowering drugs;
- restoration to normal values of the hematologic, biochemical and immunologic indices.

### Example Nº 18

### Comparative study of adjuvant activity of GSSG•Pt and GSSG•Pd on models of typhoid fever and live tularemia vaccines

Adjuvant properties of GSSG•Pt and GSSG•Pd were assessed in experiments on white mice immunized with chemical typhoid fever and live tularemia vaccines.

In experiments there were used adult random-bred male mice with body weight of 18-21 g obtained from factory "Rappolovo" (Russian Academy of Medical Sciences). Immunization was performed by commercial drugs. The tularemia vaccine was introduced intraperitoneally in dose of 250 microbe cells, the typhoid fever one was introduced subcutaneously in the previously chosen optimal dosage equivalent to 0.2 of the human one.

GSSG•Pt and GSSG•Pd were introduced in amount of- 0.5 ml through intraperitoneal route once a day during 2 days before immunization and at the Day of immunization 40-60 min before the antigen injection in the dose of 5 mg/kg per a single introduction. At the similar terms control animals were introduced with 0.5 ml of isotonic solution of sodium chloride.

The adjuvant efficacy of GSSG•Pt and GSSG•Pd was assessed through their impact on cellular and humoral immunity by means of delayed-type hypersensitivity reaction (DTHR) and determination of specific antibodies. Testing was performed at the 14^{th} day after the immunization. To obtain DTHR the mice were introduced subcutaneosuly in right back leg with the corresponding antigen in amount of 0.05 ml (tularine or O-antigen of S. typhi), and in the left one - similar amount of isotonic sodium chloride solution. Twenty-four hours later the mice were euthanized by ether overdosage, both back legs were cut at the level of an ankle joint and were weighed, and then the reaction index was calculated. Antibodies to somatic antigens of tularemia and typhoid fever agents were determined by a non-direct agglutination assay applying corresponding antigen erythrocyte diagnostic assay; impact of GSSG•Pt and GSSG•Pd on antibody development was assessed with 2 criteria - seroconversion frequency and antibody titre values.

The study results are given in the Tables 38-41.

The obtained data indicated that triple intraperitoneal introduction of GSSG•Pt and GSSG•Pd in dose of 5 mg/kg before the immunization brought forth enhanced immune response against chemical typhoid fever and live tularemia vaccines that exhibited (comparing with the control) more intensive antibody development and higher degree of DTHR on somatic antigens of these microbes. Along with that one should note that the adjuvant effect of both studied drugs was, actually, similar because inconsiderable differences of the immune response values were statistically insignificant.

Thus, the new derivatives of the oxidized glutathione containing platinum and palladium are active biological-pharmacological compounds with the adjuvant effect in regard to antigens of tularemia and typhoid fever agents.

Those skilled in the art would readily appreciate that all parameters listed herein are meant to be examples and that actual parameters will depend upon the specific application for which the methods and apparatus of the present invention are used. It is, therefore, to be understood that the foregoing embodiments are presented by way of example only and that, within the scope of the appended claims and equivalents thereto, the invention may be practiced otherwise than as specifically described.

### References:

1. International application WO 97/21444, MKI A61K 38/02, published 19/06/97.
2. RF Patent Nº 2089179, MKI A61K 38/02, 1997.
3. R. Douson, D. Elliot, W. Elliot, K. Jones. Biochemist's manual, Moscow, "Mir", 1991.
4. Tam J.P. et.al// Int.J.Pept. Prot. Res.vol.29.p.421-431, 1987.
5. Ahmed A.K. et.al// J/ Biol. Chem. 250, p/ 8477-8482, 1975.
6. Kamber B. et.al// Helv. Chim.Acta. vol. 63, p. 899-915. 1980.
7. Hope D.B. et.al// J. Biol. Chem., vol. 237, p. 1563-1566, 1962.
8. William A., Kato et. al. //Chem. Pharm. Bull., vol.34 (2), p. 486-495, 1986.
9. A. Meister et. al. // Ann. Rev. Biochem., p/711-718, 1983.
10. Chambers R.W., J.Am.Chem.Soc., 80, 3752, 1958.
11. Szent-Gyorgyi A., Proc. Natl. Acad. Sci. U.S., 58, 2012 (1967); Introduction to a Submolecular Biology, Academic Press, New York (1960).
12. Robert C Bohinski "Modern Concepts in Biochemistry", 4^{th} edition, 1987.
13. Stedman's Medical Dictionary, 26^{th} edition, Moscow, 1995; p. 519.
14. Miller-Keane "Encyclopedia & Dictionary of Medicine, Nursing, & Allied Health", 5^{th} edition, W.B.Saunders Company, 1992, p. 1221.
15. A.Horst "Molecular Basis for Disease Pathogenesis", Moscow, 1982, p. 125.
16. Brian WJ Mahy "A Dictionary of Virology", 2-e 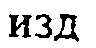 , Academic Press, 1997, c. 87.
17. Stedman's Medical Dictionary, 26^{th} edition, Moscow, 1995; p. 179.
18. Miller-Keane "Encyclopedia & Dictionary of Medicine, Nursing, & Allied Health", 5^{th} edition, W.B.Saunders Company, 1992, p. 424.
19. Harrison's Principles of Internal Medicine, 14^{th} edition, p. 511, 1998.
20. Apoptosis: a role in neoplasia, C.D. Gregory, 1996.
21. The Molecular Biology of Apoptosis, D.L. Vaux, A.Strasser; Proc. Natl. Acad. Sci. USA 93 (1996).
22. Cytokines in oncohematology, L.A.Grachyova, Moscow. 1996.
23. Textbook of biochemistry: with clinical correlations. Ed. Devlin T.M., 3rd ed. 1992, Wiley-Liss, Inc., NY. p. 522-525.
24. Kehrer J.P., Lund L.G. Cellular reducing equivalents and oxidative stress. Free Radic Biol Med. 1994 Jul. 17(1). P 65-75.
25. Beckett G.J., Hayes J.D. Glutathine S-transferase: biomedical applications. Advan. Clin. Chem. 1993, vol. 30, P. 281-380.
26. E. Busse, G.Zimmer, B. Schopohl, and Komhuber. Influence of α-Lipoic Acid on Intracellular glutathione in vitro and in vivo. // Arzneim. -Forch. / Drug Res. 42 (1), Nr. 6 (1992).
27. Holmlund J. T. Cytokines. Cancer Chemother Biol Response Modif. 1993. 14P 150-206.
28. Hansson M., Soderstrom T. The colony stimulating factors. Med Oncol Tumor Pharmacother. 1993. 10(1-2). P 5-12.
29. Dillman R. O. The clinical experience with interleukin-2 in cancer therapy. Cancer Biother. 1994 Fall. 9(3). P 183-209.
30. Whittington R., Faulds D. Interleukin-2. A review of its pharmacological properties and therapeutic use in patients with cancer. Drugs. 1993 Sep. 46(3). P 446-514.
31. Hieber U., Heim M. E. Tumor necrosis factor for the treatment of malignancies. Oncology. 1994 Mar-Apr. 51(2). P 142-53.
32. Morstyn G., Sheridan W. P. Hematopoietic growth factors in cancer chemotherapy. Cancer Chemother Biol Response Modif. 1993. 14P 353-70.
33. Neidhart J. A. Hematopoietic cytokines. Current use in cancer therapy. Cancer. 1993 Dec 1. 72(11 Suppl). P 3381-6.
34. Meister A. Anderson M.E. Glutathione. Annu. Rev. Biochem., 1983, 52:711-60.
35. Sokolovsky M., Wilchek M., Patchomik A. On the synthesis of cysteine peptides. J. Amer. Chem. Soc. 1964, Mar. 86(6), P 1202-6.
36. John J. Reiners, Jr, Effat Kodari, Roseann E.Cappel fnd Hiram F.Gilbert. Assessment of antioxidant/prooxidant status of murine skin. Part 2$5%: Quantitation of glutathione and glutathione disulfide. // Carcinogenesis Vol.12. pp. 2345-2352.
37. A.Stacchini, L. Fubini. E. Gatti, L. Mutti, W.Piacibello, F. Sanavio, G. Scagliotti, E. Pozzi and M. Aglietta. In vivo effect of human granulocyte-macrofage colony-stimulating factor (GM-CSF) on neutrophil GM-CSF receptors. // Leucemia, 1995. Vol.9. p.1-6.
38. S. W. Lowe; H. E. Ruby; T. Jacks and D.E. Hoksman "P53-dependent apoptosis modulates the Cytotoxicity of Anticancer Agents"// Cell - 1993, -74, pp. 703-711.
39. Y Xu; E.M.Yang; J.Brugalos; T. Jacks; D. Baltimor "Involvement of p53 and p21 in cellular defects and tumorogenesis in Atm -/- mice"//Mol.Cell. Biol. - 1998, - 18(7) - pp. 4385-4390.

**Table 1.**

| **Variation of the GSSG content (µg/mL) in the blood serum of CBA mice after the GSSG intravenous introduction in different doses, M±m.** | | |
|---|---|---|
| Time intervals, min | GSSG dose | |
| | 2 mg/kg | 20 mg/kg |
| GSSG content, µg/mL | | |
| 0 | 0.3±0.02 | 0.42±0.03 |
| 1 | 12.4±1.3* | 130±12.2* |
| 2 | 9.4±0.87 | 89.5±9.1* |
| 5 | 3.3±0.28* | 35.6±4.2* |
| 10 | 0.6±0.05* | 4.2±0.37* |
| 20 | 0.44±0.4 | 3.9±0.31* |
| 40 | 0.32±0.05* | 3.4±0.32* |
| 60 | 0.3±0.05 | 3.3±0.27* |

| | | |
|---|---|---|
| *p<0.01 - the p values were calculated relatively to content at 0 minute. | | |

**Table 2.**

| **Variation of the GSSG content (µg/mL) in the blood serum of CBA mice after the GSSG•Pt intravenous introduction in different doses, M±m.** | | |
|---|---|---|
| Time intervals, min | GSSG•Pt dose | |
| | 2 mg/kg | 20 mg/kg |
| GSSG content, µg/mL | | |
| 0 | 0.2±0.01 | 0.37±0.04 |
| 1 | 56.4±5.3 | 572±56.3* |
| 2 | 33.2±0.24* | 345±32.1* |
| 5 | 23.3±1.4 | 227±20.3* |
| 10 | 13.2±1.5* | 129±13.5* |
| 20 | 10.6±1.2* | 107±10.2* |
| 40 | 9.4±0.91* | 98±9.3* |
| 60 | 9.1±0.93 | 94±9.3* |

| | | |
|---|---|---|
| *p<0.01 - the p values were calculated relatively to content at 0 minute. | | |

**Table 3.**

| **The GSSG content in the blood serum and different organs of the CBA mice after the GSSG intravenous introduction in dose of 2 µg/mL, M±m.** | | | | | |
|---|---|---|---|---|---|
| Time interval, min. | Blood serum, µg/mL | Liver | Kidneys | Spleen | Lymphocytes, f×10⁵ of cells |
| | | µg/gram of tissue | | | |
| 0 | 0.3±0.02 | 9.1±0.9 | 5.3±0.6 | 3.3±0.4 | 8.9±0.96 |
| 1 | 12.4±1.3* | 402±41* | 212±22.3* | 137±0.34* | 356±32.1 |
| 2 | 9.4±0.87 | 292±33.2* | 105±11.4 | 98.6±9.5 | 157±16.2* |
| 5 | 3.3±0.28* | 94.3±9.7 | 74.3±7.6* | 67.5±6.4* | 89.3±8.4* |
| 10 | 0.6±0.05* | 17.2±1.4* | 16.3±1.4 | 12.8±3.5* | 21.8±2.6 |
| 20 | 0.44±0.4 | 9.5±0.9* | 6.1±0.6* | 4.1±0.56 | 10.3±1.1* |
| 40 | 0.32±0.05* | 9.3±0.9 | 6.7±0.5* | 3.7±0.32 | 9.6±0.9 |
| 60 | 0.3±0.05 | 8.9-±0.9* | 5.5±0.47* | 3.5±0.4* | 8.7±0.8* |

| | | | | | |
|---|---|---|---|---|---|
| * - P<0.01 - the values were calculated relatively to the analogous parameters of animals that were not introduced with the drug. ** - f (fitogram)- 10⁻¹⁵ gram. | | | | | |

**Table 4.**

| **The GSSG content in the blood serum and different organs of the CBA mice after the GSSG•Pt intravenous introduction in dose of 2 µg/mL, M±m.** | | | | | |
|---|---|---|---|---|---|
| Time interval, min. | Blood serum, µg/mL | Liver | Kidneys | Spleen | Lymphocytes f× 10⁵ of cells |
| | | µg/gram of tissue | | | |
| 0 | 0.2±0.01 | 9.1±0.9 | 5.3±0.6 | 3.3±0.4 | 8.9±0.96 |
| 1 | 56.4±5.3 | 2613±220.3* | 1842±194.1* | 936±92.7* | 2247±229.3 |
| 2 | 33.2±0.24* | 1315±132.4* | 832±87.7* | 447±42.7 | 1216±123.3* |
| 5 | 23.3±1.4 | 754±76.8 | 449±45.6* | 219±23.6* | 715±82.4* |
| 10 | 13.2±1.5* | 382±33.7* | 227±23.2* | 107±10.3* | 306±33.3* |
| 20 | 10.6±1.2* | 378±31.2* | 216±22.3 | 105±10.1* | 296±28.2* |
| 40 | 9.4±0.91* | 369±29.3* | 213±21* | 103±10.1* | 289±24.1 |
| 60 | 9.1±0.93 | 367±32.9* | 215±21.2* | 102±10.4 | 287±20.8* |

| | | | | | |
|---|---|---|---|---|---|
| * - P<0.01 - the values were calculated relatively to the analogous parameters of animals that were not introduced with the drug. ** - f (fitogram) - 10⁻¹⁵ gram. | | | | | |

**Table 5.**

| **Activity of the enzymes participating in the thiol metabolism at the intact CBA mice, M±m.** | | | | |
|---|---|---|---|---|
| Enzyme activity | Liver | Kidneys | Spleen | Thymus |
| glutathione-reductase ⁽¹⁾ | 65±3 | 89±5 | 31±4 | 11±3 |
| glutathione-peroxidase ⁽²⁾ | 32±4 | 29±6 | 12±2 | 8±2 |
| glutathione-S-transferase ⁽²⁾ | 242±14 | 310±18 | 87±6 | 41±4 |
| γ-glutamyl-transpeptidase ⁽³⁾ | 46±8 | 96±6 | 12±3 | 14±2 |

| | | | | |
|---|---|---|---|---|
| ⁽¹⁾ - activity in amount of utilized HADP, µmol/min for 1 mg of protein | | | | |
| ⁽²⁾ - activity in amount of reduced glutathione, µmol for 1 mg of protein | | | | |
| ⁽³⁾ - activity in µkat/L | | | | |

**Table 6.**

| **Activity changes for the enzymes participating in the thiol metabolism at the CBA mice after the GSSG intravenous introduction in dose of 20 µg/mL, M±m.** | | | | |
|---|---|---|---|---|
| Enzyme activity | Liver | Kidneys | Spleen | Thymus |
| glutathione-reductase ⁽¹⁾ | 128±13* | 169±12* | 74±6* | 24±4* |
| glutathione-peroxidase ⁽²⁾ | 54±6* | 46±8* | 18±3* | 14±2* |
| glutathione-S-transferase ⁽²⁾ | 351±20.8* | 510±14* | 162±8* | 81±6* |
| γ-glutamyl-transpeptidase ⁽³⁾ | 96±7* | 189±20* | 23±4* | 28±3* |

| | | | | |
|---|---|---|---|---|
| ⁽¹⁾ - activity in amount of utilized HADP, µmol/min for 1 mg of protein | | | | |
| ⁽²⁾ - activity in amount of reduced glutathione, µmol for 1 mg of protein | | | | |
| ⁽³⁾ - activity in µkat/L | | | | |
| * - P<0.01 - the p values were calculated relatively to the analogous parameters of animals that were not introduced with the drug. | | | | |

**Table 7.**

| **Activity changes for the enzymes participating in the thiol metabolism at the CBA mice after the GSSG•Pt intravenous introduction in dose of 20 µg/mL, M±m.** | | | | |
|---|---|---|---|---|
| Enzyme activity | Liver | Kidneys | Spleen | Thymus |
| glutathione-reductase ⁽¹⁾ | 67±3* | 91±4 | 37±4 | 13±2* |
| glutathione-peroxidase ⁽²⁾ | 33±4 | 31±4* | 14±2 | 9±1 |
| glutathione-S-transferase ⁽²⁾ | 513±28 | 324±22 | 93±7 | 44±5 |
| γ-glutamyl-transpeptidase ⁽³⁾ | 49±5* | 99±8 | 15±2* | 16±1 |

| | | | | |
|---|---|---|---|---|
| ⁽¹⁾ - activity in amount of utilized HADP, µmol/min for 1 mg of protein | | | | |
| ⁽²⁾ - activity in amount of reduced glutathione, µmol for 1 mg of protein | | | | |
| ⁽³⁾ - activity in µkat/L | | | | |
| * - P<0.01 - the p values were calculated relatively to the analogous parameters of animals that were not introduced with the drug. | | | | |

**Table 8.**

| **GSSG effect on in vitro cytokine production by human mononuclear leukocytes. (M±m)** | | | | | | |
|---|---|---|---|---|---|---|
| Cytokine production (pg/mL) | GSSG (µg/mL) | | | | | |
| | 0.5 | 5.0 | 50 | 500 | 5000 | Control (RPMI) |
| IL-1β | 56.0±9.1 | 88.5±13.5* | 202±24.9* | 275±39.3* | 259±36.8 | 46.0±6.8 |
| IL-2 | 87.2±7.5 | 123±10.6* | 234±21.5* | 310±32.1* | 348±29.4* | 51±5.4 |
| IL-6 | 430±55.6 | 550±61.3* | 1810±205* | 2103±132* | 2518±264* | 129±12.4 |
| INF-α | 130±14.9 | 109±12.1* | 407±51.4* | 514±56.2* | 811±64.1 | 98.3±14.0 |
| TNF-α | 99.1±11.6 | 314±44.7 | 813±90.8* | 1525±163* | 1900±206* | 88.7±9.3 |

| | | | | | | |
|---|---|---|---|---|---|---|
| Note: (*) ― values' differences are statistically significant (p < 0.01) as compared to the control. | | | | | | |

**Table 9.**

| **GSSG•Pt effect on in vitro cytokine production by human mononuclear leukocytes. (M±m)** | | | | | | |
|---|---|---|---|---|---|---|
| Cytokine production (pg/mL) | GSSG•Pt (µg/mL) | | | | | |
| | 0.5 | 5.0 | 50 | 500 | 5000 | Control (RPMI) |
| IL-1β | 83.0±7.8 | 212±31.7* | 511±55.1* | 650±67.1* | 620±61.3* | 46.0±6.8 |
| IL-2 | 117±11.5 | 263±20.6* | 532±53.5* | 703±72.0* | 848±89.4 | 51±5.4 |
| IL-3 | 436±43 | 543±55.2* | 754±74.5* | 965±87.4* | 1024±108* | 206±22.4 |
| IL-4 | 160±14.9 | 143±14.1* | 124±13.3* | 107±10.1* | 84.9±7.1 | 175.3±16.0 |
| IL-6 | 851±111 | 1680±207* | 3859±425* | 4007±419* | 4035±518* | 129±12.4 |
| IL-8 | 123±13.7 | 134±13.2* | 138±13.5* | 140±13.6 | 143±13.9* | 114±11.3 |
| IL-10 | 174±16.4 | 153±14.9* | 126±11.4* | 109±9.7* | 94±9.2 | 206±19.2 |
| IL-12 | 146±13.2 | 164±15.6* | 186±17.9* | 208±19.5 | 227±21.4* | 115±10.3 |
| INF-α | 150±14.9 | 176±17.1* | 468±69.3* | 905±141 | 849±1121 | 98.3±14.0 |
| IFN-γ | 156±14.8 | 175±16.9* | 194±18.5* | 205±21.4* | 267±25.1* | 132±11.4 |
| TNF-α | 318±47.8 | 502±86.4* | 1308±164* | 2100±294* | 2640±355 | 88.7±9.3 |
| TNF-γ | 167±15.8 | 386±37.5* | 584±57.7* | 796±78.4* | 1063±10* | 109±9.5 |

**Table 10.**

| **Effect of the GSSG and GSSG•Pt on cytokines' and hemopoietic factors' production by splenocytes, bone marrow and blood cellular indices, and immune response to SRBC in cyclophosphamide treated mice. (M±m)** | | | | | |
|---|---|---|---|---|---|
| Parameter | N | Intact animals | Cyclophosphamide-treated animals | | |
| | | Normal saline | Normal saline | GSSG | GSSG•Pt |
| Blood leukocyte count. 10⁹/L | 10 | 11.9±1.81 | 4.7±1.25* | 8.5±0.81◆ | 12.4±1.2◆ |
| Blood lymphocyte count, 10⁹/L | 10 | 7.4±0.85 | 3.1±0.56* | 6.0±1.28◆ | 6.9±1.04◆ |
| Bone marrow nucleated cell number, 10⁶/L | 10 | 53.7±8.7 | 23.8±5.0* | 45.4±3.9◆ | 62.3±4.7◆ |
| SRBC agglutinin titer (log₂) | 10 | 5.33±0.74 | 1.47±0.35* | 3.08±0.59◆ | 5.42±0.54◆ |
| IL-1β | 10 | 54±3.7 | 10.2±1.6* | 23.4±2.5◆ | 49.7±5.2◆ |
| IL-2 | 10 | 76±6.8 | 14.6±1.3* | 35±4.0◆ | 74.1±7.2◆ |
| IL-3 | 10 | 178±16.5 | 34.5±3.7* | 72.4±6.9◆ | 174±16.8◆ |
| IL-4 | 10 | 89±18.9 | 113.8±4.6* | 105.8±7.6◆ | 87±17.4◆ |
| IL-6 | 10 | 98±8.7 | 19.6±1.8* | 42.7±4.1◆ | 95.7±9.4◆ |
| IL-8 | 10 | 102±0.5 | 23.4±2.4* | 52.6±4.9◆ | 97.8±8.9◆ |
| IL-10 | 10 | 86.8±17.3 | 137.8±14.9* | 126.9±15.4◆ | 84.0±8.9◆ |
| IL-12 | 10 | 96±8.5 | 18.7±1.7* | 38.3±3.6◆ | 92.5±8.5◆ |
| INF-α | 10 | 113±11.2 | 26.4±2.5* | 57.2±5.2◆ | 109.5±9.7◆ |
| IFN-γ | 10 | 126±11.9 | 24.8±2.2* | 49.6±4.1◆ | 118.6±12.0◆ |
| TNF-α | 10 | 93±8.5 | 17.4±1.6* | 36.1±3.2◆ | 89.7±9.1◆ |
| TNF-γ | 10 | 115±10.6 | 22.7±2.5* | 47.4±5.1◆ | 111.3±12.3◆ |
| GM-CSF | 10 | 180±14.2 | 48.2±7.2* | 119.5±13.4◆ | 178.2±18.1◆ |
| G-CSF | 10 | 150±13.7 | 26.7±3.1* | 67±6.1◆ | 148.4±14.2◆ |
| M-CSF | 10 | 130±10.03 | 34.2±2.7* | 71±6.9◆ | 125.7±11.4◆ |

| | | | | | |
|---|---|---|---|---|---|
| Differences are statistically significant (*p*<0.05) as compared: (*) - to the group of intact animals; (◆) - to the control group (CP + normal saline). | | | | | |

**Table 11.**

| **General blood analysis of the male rats with cytopenia after the precursory cyclophosphamide introduction in the dose 100 mg/kg and the treatment with GSSG•Pt in the dose 10 mg/kg, M ± m** | | | |
|---|---|---|---|
| Parameter | Intact animals | Cyclophosphamide-treated animals | |
| | Normal saline | Normal saline | GSSG•Pt |
| Day 10 | | | |
| Hemoglobin, g/dL | 13.3±0.4 | 10.3±0.3* | 12.7±0.3* |
| Hematocrit, g% | 48.4±0.9 | 36.2±0.8* | 45.3±1.1* |
| Erythrocytes, 10¹²/L | 6.1±0.2 | 3.5±0.3* | 5.5±0.3* |
| Colour index (MCH), pg | 17.8±0.3 | 15.5±0.3* | 16.5±0.5* |
| ESR. mm/hr. | 5.3±0.2 | 6.6±0.3* | 5.5±0.2* |
| Platelets, 10⁹/L | 808±36 | 342±52* | 735±42* |
| Leukocytes, 10⁹/L | 7.8±0.1 | 1.2±0.1* | 5.6±0.2* |
| Stab neutrophils, % | 0 | 0 | 0 |
| Segmented neutrophils, % | 14.4±1.0 | 58.2±4.6* | 19.2±2.5* |
| Basophils. % | 0 | 0 | 0 |
| Lymphocytes, % | 82.0±1.5 | 34.7±0.9* | 50.0±0.8* |
| Eosinophils. % | 1.3±0.3 | 0 | 2.0±0.3* |
| Monocytes, % | 2.1±0.2 | 7.9±0.7* | 2.8±0.3* |
| Plasma cells, % | 0 | 0 | 2.0±0.2* |
| Reticulocytes, % | 2 | 1 | 2 |
| Normocytes, % | 0 | 0 | 1 |
| Polychromatic cells, % | 0 | 2 | 1 |

| Day 15 | | | |
|---|---|---|---|
| Hemoglobin. g/dL | 14.3±0.3 | 10.0±0.3* | 13.0±0.3* |
| Hematocrit, g% | 49.7±1.0 | 30.4±1.0* | 46.7±0.7* |
| Erythrocytes, 10¹²/L | 6.2±0.1 | 3.4±1.0* | 5.7±0.2* |
| Colour index (MCH), pg | 18.2±0.2 | 14.7±0.2* | 17.0±0.3* |
| ESR, mm/hr. | 5.3±0.2 | 7.6±0.2* | 5.6±0.3* |
| Platelets, 10⁹/L | 824±41 | 225±49* | 773±40* |
| Leukocytes, 10⁹/L | 8.0±0.1 | 1.2±0.21* | 5.5±0.3* |
| Stab neutrophils, % | 0 | 0 | 0 |
| Segmented neutrophils, % | 13.7±1.7 | 42.2±2.5* | 18.0±2.2* |
| Basophils. % | 0 | 0 | 0 |
| Lymphocytes, % | 77.3±2.0 | 35.2±3.5* | 71.0±1.8* |
| Eosinophils, % | 1.0±0.3 | 0 | 0 |
| Monocytes, % | 2.1±0.2 | 4.5±0.5* | 2.2±0.3* |
| Plasma cells, % | 0 | 0 | 2.0±0.2* |
| Reticulocytes, % | 2 | 1 | 1 |
| Normocytes. % | 0 | 0 | 0 |
| Polychromatic cells, % | 0 | 3 | 1 |

| | | | |
|---|---|---|---|
| Note: with a sign *) the significant distinctions from the intact group at the confidence level P>0.95 are marked. | | | |

**Table 12.**

| **Myelogram of the male rats with cytopenia after the precursory cyclophosphamide introduction in the dose 100 mg/kg and the treatment with GSSG•Pt in the dose 10 mg/kg, M ± m** | | | |
|---|---|---|---|
| Parameter | Intact animals | Cyclophosphamide-treated animals | |
| | Normal saline | Normal saline | GSSG•Pt |
| Day 10 | | | |
| Cells' quantity, 10¹¹/L | 71,4±5.5 | 33.4±4.2* | 63.0±7.4* |
| Non-differentiated blasts, % | 0.4 ± 0.1 | 0* | 1.0 ± 0.2* |
| Proerythroblasts, % | 0 | 0 | 0 |
| Erythroblasts. % | 0.8 ± 0.2 | 0.3 ± 0.1* | 1.8 ± 0.4* |
| Pronormoblasts, % | 0.6±0.1 | 0.2±0.1* | 0.5±0.1* |
| Basophilic normoblasts, % | 7.5 ± 1.0 | 3.2 ± 0.4* | 6.2 ± 1.08 |
| Polychromatic normoblasts, % | 8.4 ± 1.2 | 3. 1 ± 0.8* | 16.5 ± 2.2* |
| Eosinophilic normoblasts, % | 5.8 ± 1.2 | 2.3 ± 0.6* | 8.3 ± 2.0* |
| Red blood mitosis count, % | 0.56 ± 0.09 | 0.18 ± 0.08* | 0.75 ± 0.22 |
| Myeloblasts, % | 2.1±0.5 | 1.2±0.02 | 3.6 ± 0.6* |
| Promyelocytes, % | 4.0±0.5 | 1.8±0.5 | 5.6±0.8* |
| Myelocytes, % | 6.1±0.7 | 3.4 ± 1.1* | 7.5 ± 0.88 |
| Metamyelocytes. % | 8.5 ± 1.2 | 4.5 ± 0.8* | 6.7 ± 1.1* |
| Stab neutrophils. % | 11.6±1.5 | 4.2 ± 1.0* | 9.9±1.4 |
| Segmented neutrophils. % | 16.7 ± 3.5 | 37.4 ± 5.2* | 44.3 ± 5.0* |
| Eosinophils, % | 7.2 ± 1.2 | 3.1 ± 1.0* | 7.0 ± 1.2* |
| Basophils, % | 0 | 0 | 0.1 ± 0.05* |
| White blood mitosis count, % | 0.44 ± 0.10 | 0.15 ± 0.04* | 0.58 ± 0.14 |
| Prolymphocytes, % | 0 | 0 | 0.5 ± 0.2* |
| Lymphocytes, % | 8.1±0.7 | 2.2 ± 0.5* | 16.4±2.1*8 |
| Plasma cells. % | 1.3 ± 0.3 | 0 | 2.4 ± 0.2* |
| Promonocytes, % | 0 | 0 | 0.1 |
| Monocytes, % | 0.8 ± 0.2 | 0.1±0.05* | 0.5 ± 0.2* |
| Reticulocytes, % | 2.6±0.7 | 0.9±0.3 | 3.5±1.2* |
| Megakaryocytes and megakaryoblasts, % | 0.45 ± 0.08 | 0.12 ± 0.08* | 0.62 ± 0.11* |

| Day 15 | | | |
|---|---|---|---|
| Cells' quantity, 10¹¹/L | 70.8±6.0 | 36.8±5.0* | 66.1±6.5* |
| Non-differentiated blasts. % | 0.5 ± 0.1 | 0* | 1.2 ± 0.2 |
| Proerythroblasts, % | 0 | 0 | 0.1 ± 0.05 |
| Erythroblasts, % | 0.8 ± 0.2 | 0.4 ± 0.1 | 2.2 ± 0.5* |
| Pronormoblasts, % | 0.6 ± 0.1 | 0.3 ± 0.1 | 0.5 ± 0.1 |
| Basophilic normoblasts, % | 7.6 ± 0.8 | 2.4 ± 0.5* | 6.8 ± 1.1 |
| Polychromatic normoblasts, % | 8.3 ± 1.1 | 3.3 ± 0.9* | 15.7 ± 2.6* |
| Eosinophilic normoblasts, % | 5.7 ± 1.1 | 3.2 ± 0.9* | 7.7±2.2* |
| Red blood mitosis count, % | 0.58 ± 0.11 | 0.22 ± 0.12* | 0.71 ± 0.14* |
| Myeloblasts, % | 2.0±0.4 | 1.5±0.4 | 4.0±0.8** |
| Promyelocytes, % | 4.1±0.6 | 1.5 ± 0.3* | 5.0 ± 0.8* |
| Myelocytes, % | 5.9 ± 0.8 | 3.1±0.6* | 6.8 ± 0.7* |
| Metamyelocytes, % | 8.4±1.2 | 3.3±0.9 | 6.5±0.8** |
| Stab neutrophils, % | 11.5 ± 1.5 | 3.5 ± 0.6* | 11.3 ± 1.3* |
| Segmented neutrophils, % | 16.2±2.2 | 42.1±5.8 | 33.1±4.7* |
| Eosinophils, % | 7.4 ± 1.3 | 3.2 ± 0.8* | 4.0 ± 1.5* |
| Basophils, % | 0 | 0 | 0 |
| White blood mitosis count. % | 0.42 ± 0.10 | 0.17 ± 0.05* | 0.57 ± 0.13* |
| Prolymphocytes, % | 0 | 0 | 0.4 ± 0.2* |
| Lymphocytes, % | 8.0±0.6 | 2.0±0.4 | 14.3 ± 1.2* |
| Plasma cells, % | 1.4 ± 0.3 | 0 | 2.6 ± 0.6* |
| Promonocytes, % | 0 | 0 | 0.1 |
| Monocytes, % | 0.8 ± 0.2 | 0.1 ± 0.05* | 0.5 ± 0.2* |
| Reticulocytes. % | 2.4 ± 0.6 | 1.6±0.4 | 4.1±0.8* |
| Megakaryocytes and megakaryoblasts, % | 0.50 ± 0.10 | 0.15±0.05* | 0.54 ± 0.1* |

| | | | |
|---|---|---|---|
| Note: with a sign *) the significant distinctions from the intact group at the confidence level P > 0.95 are marked. | | | |

**Table 13.**

| **Effect of GSSG and Li•GSSG•Pt on cytokines' and hemopoietic factors' production by splenocytes, bone marrow, spleen and blood cellular indices, and bone marrow and spleen hematopoietic colony formation capability in irradiated mice. (M±m)** | | | | | |
|---|---|---|---|---|---|
| Parameter | N | Sham-irradiated animals | Irradiated animals | | |
| | | Normal saline | Normal saline | GSSG | Li•GSSG•Pt |
| Blood leukocyte count, 10⁹/L | 12 | 12.7±1.3 | 3.4±0.9* | 6.7±1.3◆ | 10.7±2.0◆ |
| Blood lymphocyte count, 10⁹/L | 12 | 7.9±0.7 | 2.2±1.3* | 5.2±0.8◆ | 7.4±0.8◆ |
| Bone marrow nucleated cell number, 10⁶/L | 12 | 45.1±3.2 | 14.0±1.0* | 23.3±5.2◆ | 42.0±4.0◆ |
| Splenocytes' count, 10⁷/organ | 12 | 9.8±1.5 | 4.8±1.3* | 6.3±1.2◆ | 8.9±2.0◆ |
| Bone marrow CFU | 12 | 59.4±3.2 | 11.6±2.2* | 34.3±3.9◆ | 56.3±3.9◆ |
| Spleen CFU | 12 | 93.2±4.1 | 40.0±5.4* | 56.3±6.8◆ | 89.6±4.7◆ |
| IL-1β | 12 | 56±3.2 | 8.3±1.5* | 1.8±2.5◆ | 52.7±5.4◆ |
| IL-2 | 12 | 73±6.2 | 10.6±1.4* | 28±4.0 | 70.6±7.1◆ |
| IL-3 | 12 | 169±16.7 | 41.7±4,7* | 82.4±7.9◆ | 167±16.1◆ |
| IL-4 | 12 | 86±10.4 | 136.3±12.9* | 126.8±6.4 | 84±8.2◆ |
| IL-6 | 12 | 98±8.7 | 19.6±1.8* | 42.7±4.1◆ | 95.7±9.4◆ |
| IL-8 | 12 | 103±10.5 | 25.4±2.4* | 57.6±5.9 | 99.8±9.9◆ |
| IL-10 | 12 | 96±10.3 | 154.8±14.9* | 132.9±8.4◆ | 98.0±9.9◆ |
| IL-12 | 12 | 97±8.7 | 28.7±2.7* | 48.3±4.6 | 95.5±9.5◆ |
| INF-α | 12 | 118±11.4 | 29.4±3.7* | 56.2±5.9◆ | 105.6±9.1◆ |
| IFN-γ | 12 | 116±12.9 | 35.8±3.2* | 47.6±4.3 | 113.8±11.0◆ |
| TNF-α | 12 | 95±9.5 | 21.4±2.6* | 34.5±3.8 | 91.7±9.3◆ |
| TNF-γ | 12 | 115±10.6 | 22.7±2.5* | 47.4±5.1◆ | 111.3±12.3◆ |
| GM-CSF | 12 | 173±17.2 | 39.2±3.6* | 127.5±12.4 | 169.2±16.7◆ |
| G-CSF | 12 | 156±15.3 | 28.7±2.7* | 59±5.6◆ | 139.4±13.5◆ |
| M-CSF | 12 | 121±12.6 | 45.2±4.8* | 78±7.5◆ | 118.7±11.1◆ |

| | | | | | |
|---|---|---|---|---|---|
| Differences are statistically significant (*p* < 0.05) as compared: (*) - to the group of intact animals; (◆) - to the control group of irradiated mice administered with the normal saline. | | | | | |

**Table 14.**

| **Molecular mechanisms of the immunomodulating effects of the GSSG•Pt composite indicating reproduction of cytokine effects** | | | | | | |
|---|---|---|---|---|---|---|
| Study conditions | Cytokine content (µg/ml) | | | | CD25+ content, (%) | Phosphorilating level on tyrosine of the lymphocyte cytosol proteins (impulse/min) |
| | IL-1 | IL-6 | TNF-α | IFN-γ | | |
| 1 | 2 | 3 | 4 | 5 | 6 | 7 |
| 0 (zero point as control ) | 45±4 | 120±7 | 90±7 | 90±6 | 3.7±0.9 | 6640±270 |
| 10 | 50±6 | 126±9 | 89±4 | 101±9 | 3.6±0.5 | 19240±360* |
| 30 | 48±7 | 128±6 | 93±6 | 102±8 | 3.8±0.4 | 46980±620* |
| 1 hr. | 52±6 | 142±11 | 108±6 | 134±6 | 3.9±0.5 | 22350±370* |
| 6 hrs. | 174±17* | 392±12 | 402±8* | 214±22* | 4.1±0.4 | 11210±260* |
| 12 hrs. | 275±39* | 2132±132* | 1525±163* | 514±56* | 5.9±0.5* | 8420±170 |
| 24 hrs. | 251±23* | 1621±36* | 1021±56* | 496±36* | 17.6±2.3* | 6780±420 |
| 48 hrs. | 189±17* | 1241±12* | 893±43* | 247±21 | 20.5±4.3* | 6320±210 |

| | | | | | | |
|---|---|---|---|---|---|---|
| **Note:** 1) Temporary points characterize intervals from the moment of the GSSG•Pt composite introduction in concentration 100 µg/ml of the cultural medium. 2) Sign * means reliability of the changes (p<0.01) in regard to the zero point considered as the control level. | | | | | | |

**Table 15.**

| **Influence of the GSSG•Pt composite salts on phosphorilating level on tyrosine and percentage of lymphocytes bearing IL-2-receptors to the total lymphocyte count of the CBA mice in conditions of cyclophosphamide-induced immunodepression** | | |
|---|---|---|
| The tested articles | Content of the lymphocytes bearing IL-2-receptors (%) | Phosphorilating level on tyrosine (impulse/min.) |
| Na-GSSG•Pt | 16.8±1.1* | 28980±420* |
| Li-GSSG•Pt | 20.2±1.9* | 34550±790* |
| Mg-GSSG•Pt | 18.6±0.8* | 29800±880* |
| Normal saline (control) | 4.3±1.3 | 11710±340 |

| | | |
|---|---|---|
| * p<0.05 Values of *p* were calculated relatively to the data obtained from normal saline usage. | | |

**Table 16.**

| **Effect of GSSG on blood and immunology indices and cytokine levels in patient with stomach cancer, peritoneal metastases, ascites and splenomegaly** | | |
|---|---|---|
| Parameter | Prior to the treatment | 2 months after the treatment onset |
| Hematology | | |
| Erythrocytes, 10¹²/L | 3.2 | 3.7 |
| Hemoglobin, g/L | 112 | 121 |
| Platelets, 10⁹/L | 205 | 195 |
| Leukocytes, 10⁹/L | 12.4 | 8.9 |
| Neutrophils (stab),% | 12 | 8 |
| Neutrophils (segm.), % | 54 | 44 |
| Eosinophils, % | 5 | 4 |
| Lymphocytes, % | 21 | 36 |
| Monocytes, % | 8 | 7 |
| ESR, mm/hr | 54 | 15 |

| Biochemistry | | |
|---|---|---|
| Total protein, g/L | 62 | 76 |
| Albumin, % | 26 | 42 |
| α₁-globulin, % | 3 | 7 |
| α₂- globulin, % | 14 | 12 |
| β-globulin, % | 7 | 10 |
| γ- globulin, % | 50 | 26 |
| A/G ratio | 0.35 | 0.72 |
| Urea, mmol/L | 6.6 | 6.1 |
| Creatinine, mmol/L | 0.11 | 0.09 |
| Bilirubin, mcmol/L | 40,0 | 32.4 |
| Bilirubin conjugated, µmol/L | 31.0 | 21.4 |
| Prothrombin index, % | 75 | 79 |
| Glucose, mmol/L | 5.9 | 5.3 |
| SGOT, mmol/hr/L | 4.8 | 1.21 |
| SGPT, mmol/hr/L | 3.8 | 1.21 |

| Immunology | | |
|---|---|---|
| Lymphocytes, 10⁶/L | 260.4 | 1204 |
| T-helpers (CD4⁺), 10⁶/L | 132.8 | 524 |
| T-suppressors (CD8⁺),10⁶/L | 13 | 374 |
| (CD4⁺)/(CD8⁺) | 10.2 | 1.4 |
| NK-cells(CD16⁺), 10⁶/L | 26 | 224 |
| B-lymphocytes (CD20⁺) | 26 | 152 |
| IL2-receptor bearing cells (CD25⁺), 10⁶/L, | 26.8 | 398 |
| HLA 11-receptor bearing cells, 10⁶/L | 13 | 158 |
| IgA, g/L | 3.2 | 2.38 |
| IgG, g/L | 21.82 | 14.34 |
| IgM, g/L | 3.6 | 0.58 |

| Cytokine status | | |
|---|---|---|
| IL-2, pg/mL | 145 | 367 |
| IL-1β, pg/mL | 92 | 527 |
| IL-6, pg/mL | 118 | 506 |
| IFN-γ, pg/mL | 105 | 624 |
| TNF-α, pg/mL | 183 | 507 |
| GM-CSF, colonies/10⁵ cells | 43.5 | 108 |

**Table 17.**

| **Effect of the GSSG•Pt on blood and immunology indices and cytokine levels in patient with stomach cancer, peritoneal metastases, ascites and splenomegaly** | | |
|---|---|---|
| Parameter | Prior to the treatment | 2 months after the treatment onset |
| Hematology | | |
| Erythrocytes, 10¹²/L | 3.1 | 4.4 |
| Hemoglobin, g/L | 110 | 135 |
| Platelets, 10⁹/L | 215 | 275 |
| Leukocytes, 10⁹/L | 12.2 | 8.1 |
| Neutrophils (stab).% | 11 | 2 |
| Neutrophils (segm.), % | 57 | 47 |
| Eosinophils, % | 4 | 3 |
| Lymphocytes, % | 22 | 39 |
| Monocytes, % | 6 | 9 |
| ESR, mm/hr | 54 | 15 |

| Biochemistry | | |
|---|---|---|
| Total protein, g/L | 64 | 82 |
| Albumin, % | 21 | 50 |
| α₁-globulin, % | 3 | 11 |
| α₂- globulin, % | 15 | 7 |
| β- globulin, % | 6 | 13 |
| γ- globulin, % | 50 | 19 |
| A/G ratio | 0.26 | 1.0 |
| Urea, mmol/L | 6.5 | 7.4 |
| Creatinine, mmol/L | 0.10 | 0.82 |
| Bilirubin. mcmol/L | 36.0 | 20.1 |
| Bilirubin conjugated, µmol/L | 28.7 | 14.4 |
| Prothrombin index, % | 73 | 95 |
| Glucose, mmol/L | 6.1 | 4.2 |
| SGOT, mmol/hr/L | 3.8 | 0.21 |
| SGPT, mmol/hr/L | 3.2 | 0.17 |

| Immunology | | |
|---|---|---|
| Lymphocytes, 10⁶/L | 476.2 | 3320 |
| T-helpers (CD4⁺), 10⁶/L | 157.2 | 1454 |
| T-suppressors (CD8⁺), 10⁶/L | 15.1 | 908 |
| (CD4⁺)/(CD8⁺) | 10.4 | 1.6 |
| NK-cells (CD16⁺), 10⁶/L | 39 | 776 |
| B-lymphocytes (CD20⁺) | 44 | 398 |
| IL2-receptor bearing cells (CD25⁺), 10⁶/L | 42 | 2000 |
| HLA11-receptor bearing cells, 10⁶/L | 45 | 754 |
| IgA, g/L | 3.0 | 2.42 |

| | | |
|---|---|---|
| IgG, g/L | 24,7 | 13.2 |
| IgM, g/L | 2.8 | 0.4 |

| Cytokine status | | |
|---|---|---|
| IL-2, pg/mL | 214 | 1237 |
| IL-1β, pg/mL | 115 | 1113 |
| IL-3, pg/mL | 87 | 589 |
| IL-4, pg/mL | 230 | 108 |
| IL-6, pg/mL | 215 | 1553 |
| IL-8, pg/mL | 136 | 157 |
| IL-10, pg/mL | 432 | 116 |
| IL-12, pg/mL | 89 | 626 |
| IFN-α, pg/mL | 86.4 | 962 |
| IFN-γ, pg/mL | 129 | 919 |
| TNF-α, pg/mL | 202 | 1080 |
| TNF-γ, pg/mL | 163 | 745 |
| GM-CSF, colonies/10⁵ cells | 45.3 | 213 |
| G-CSF, colonies/10⁵ cells | 32.7 | 174 |
| M-CSF, coionies/10⁵ cells | 25.6 | 146 |

**Table 18.**

| **GSSG influence on development of hematologic, biochemical, immunologic indices and cytokine content of the patient having lung cancer complicated with liver metastases** | | |
|---|---|---|
| Indices | Before treatment | 3 months later |
| Hematology | | |
| Erythrocytes (x 10¹²/L) | 3.2 | 3.9 |
| Hemoglobin, g/L | 91 | 118 |
| Stab neutrophils. % | 19.6 | 6 |
| Segmented neutrophils, % | 39 | 47 |
| Leukocytes (x 10⁹/L) | 12.9 | 9.1 |
| Lymphocytes. % | 15 | 39 |
| ESR, mm/hr | 65 | 27 |

| Biochemistry | | |
|---|---|---|
| ALT (mmol/hr.L.) | 0.7 | 0.59 |
| AST (mmol/hr.L.) | 1.8 | 0.5 |
| Bilirubin - total (µm/L) | 17.4 | 7.0 |
| Urea, mmol/L | 8.4 | 4.4 |
| Creatinine, mmol/L | 0.11 | 0.066 |

| Immunology | | |
|---|---|---|
| T-helpers, (CD4⁺), 10⁶/L | 325 | 692 |
| T-suppressors, (CD8⁺),10⁶/L | 112 | 320 |
| NK-cells(CD16⁺), 10⁶/L | 138 | 194 |
| B-lymphocytes (CD20⁺⁾ | 192 | 260 |
| Cells bearing IL-2 receptors (CD25⁺), 10⁶/L | 141 | 236 |

| Cytokine status | | |
|---|---|---|
| IL-2, pg/mL | 159 | 360 |
| IL-1β, pg/mL | 120 | 375 |
| IFN-γ, pg/mL | 198 | 213 |
| TNF-α pg/mL | 535 | 822 |

**Table 19.**

| **GSSG•Pt influence on development of hematologic, biochemical, immunologic indices and cytokine content of the patient having lung cancer complicated with liver metastases** | | |
|---|---|---|
| Indexes | Before treatment | 3 months later |
| Hematology | | |
| Erythrocytes (x10¹²/L) | 2.7 | 4.2 |
| Hemoglobin, g/L | 62 | 141 |
| Platelets, 10⁹/L | 336 | 302 |
| Leukocytes (x10⁹/L) | 14.8 | 8.9 |
| Stab neutrophils, % | 20.5 | 2 |
| Segmented neutrophils, % | 42 | 59 |
| Lymphocytes, % | 18.5 | 36 |
| ESR, mm/hr | 43 | 13 |

| Biochemistry | | |
|---|---|---|
| ALT (mmol/hr.L.) | 1.4 | 0.16 |
| AST (mmol/hr.L.) | 1.0 | 0.1 |
| Bilirubin - total (µm/L) | 24.0 | 6.2 |
| Urea. mmol/L | 5.0 | 4.0 |
| Creatinine, mmol/L | 110 | 100 |

| Immunology | | |
|---|---|---|
| T-lymphocytes (CD3⁺), % | 41 | 57 |
| T-lymphocytes (CD3⁺), 10⁶/L | 1148 | 1824 |
| T-helpers (CD4⁺), % | 13 | 26 |
| T-helpers (CD4⁺), 10⁶/L | 364 | 532 |
| T-suppressors (CD8⁺), % | 14 | 16 |
| T-suppressors (CD8⁺), 10⁶/L | 292 | 412 |
| CD4⁺ / CD8⁺ | 0.93 | 1.6 |
| NK-cells (CD 16⁺), 10⁶/L | 98 | 454 |
| Cells bearing IL-2 receptors, (CD25⁺), 10⁶/L | 120 | 460 |
| B-lymphocytes (CD72⁺), % | 7 | 13 |
| B-lymphocytes (CD72⁺), 10⁶/L | 196 | 416 |

| Cytokine status | | |
|---|---|---|
| IL-1β, pg/mL | 220 | 413 |
| IL-2, pg/mL | 150 | 492 |
| IL-4, pg/mL | 230 | 184 |
| IL-6, pg/mL | 173 | 354 |
| IL-10, pg/mL | 918 | 626 |
| IFN-α. pg/mL | 284 | 383 |
| IFN-γ, pg/mL | 217 | 584 |
| TNF-α | 168 | 835 |

**Table 20.**

| **Influence of GSSG on changes of hematologic, biochemical, serologic, immune indices and cytokine content at the patient with chronic HBV (viral hepatitis B)** | | |
|---|---|---|
| Parameter | Prior to the treatment | 1 month after the treatment |
| Hematology | | |
| Erythrocytes (x10¹²/L) | 4.1 | 4.3 |
| Hemoglobin, g/L | 100 | 115 |
| Leukocytes (x10⁹/L) | 9.9 | 6.2 |
| Lymphocytes, % | 16 | 25 |
| Stab neutrophils, % | 8 | 3 |
| Segmented neutrophils, % | 65 | 62 |
| Monocytes, % | 9 | 7 |
| Eosinophils, % | 2 | 3 |
| ESR, mm/hr | 35 | 17 |

| Blood Biochemistry | | |
|---|---|---|
| ALT (mmol/hr.L.) | 5.4 | 2.2 |
| Bilirubin - total (µm/L) | 34.6 | 26.0 |

| Serology | | |
|---|---|---|
| HBs Ag (ng/mL) | 183 | 178 |
| Anti HBcor IgG | +++ | +++ |
| Anti HBcor IgM | - | - |
| PCR HBV | + | - |
| Anti HBs Ag | <10 U/mL | <10 U/mL |

| Immunology | | |
|---|---|---|
| CD4⁺, 10⁶/L | 325 | 692 |
| CD8⁺, 10⁶/L | 112 | 320 |
| CD16⁺, 10⁶/L | 138 | 94 |
| CD72⁺, 10⁶/L | 192 | 160 |
| CD 95⁺, (Fas Ag), % | 10 | 21 |

| Cytokine status | | |
|---|---|---|
| IL-1β, pg/mL | 187 | 97 |
| IL-2, pg/mL | 138 | 79 |
| INF-γ, pg/mL | 283 | 252 |

**Table 21.**

| **Influence of GSSG•Pt on changes of hematologic, biochemical, serologic, immune indices and cytokine content at the patient with chronic HBV (viral hepatitis B)** | | |
|---|---|---|
| Parameter | Prior to the treatment | 1 month after the treatment |
| Hematology | | |
| Erythrocytes (x 10¹²/L) | 3.8 | 4.5 |
| Hemoglobin, g/L | 105 | 130 |
| Leukocytes (x 10⁹/L) | 10.5 | 5.6 |
| Lymphocytes, % | 19 | 28 |
| Stab neutrophils, % | 10 | 3 |
| Segmented neutrophils, % | 42 | 64 |
| Monocytes, % | 22 | 3 |
| Eosinophils, % | 7 | 2 |
| ESR, mm/hr | 42 | 11 |

| Blood biochemistry | | |
|---|---|---|
| ALT (mmol/hr.L.) | 6.2 | 0.8 |
| Bilirubin - total (µm/L) | 78.3 | 12.0 |

| Serology | | |
|---|---|---|
| HBs Ag (ng/mL) | 198 | 69 |
| Anti HBcor IgG | +++ | +++ |
| Anti HBcor IgM | - | - |
| PCR HBV | + | - |
| Anti HBs Ag | <10 U/mL | <10 U/mL |

| Immunology | | |
|---|---|---|
| CD4⁺ ,10⁶/π, | 306 | 785 |
| CD8⁺ ,10⁶/π | 121 | 528 |
| CD16⁺, 10⁶/π | 143 | 85 |
| CD20⁺, 10⁶/π | 260 | 144 |
| CD95⁺ (Fas Ag)% | 7 | 75 |

| Cytokine status | | |
|---|---|---|
| IL-1β, pg/mL | 195 | 76 |
| IL-2, pg/mL | 156 | 59 |
| IL-6, pg/mL | 124 | 323 |
| IL-4, pg/mL | 1550 | 300 |
| IL-10, pg/mL | 1362 | 686 |
| INF-γ, pg/mL | 275 | 192 |
| TNF-α, pg/mL | 720 | 184 |

**Table 22.**

| **Changes in hematological, serological and biochemical parameters before and after the treatment with the GSSG•Pt drugs use at the patient with acute viral hepatitis B** | | | |
|---|---|---|---|
| Parameter | Prior to the treatment | After the treatment | 1 month after the treatment |
| Hematology | | | |
| Erythrocytes (x 10¹²/L) | 3,9 | 4.1 | 4,2 |
| Hemoglobin, g/L | 116 | 125 | 134 |
| Leukocytes (x 10⁹/L) | 4,5 | 4.7 | 4,6 |
| Lymphocytes, % | 46 | 38 | 35 |
| Stab neutrophils, % | 6 | 6 | 5 |
| Segmented neutrophils % | 42 | 51 | 58 |
| Monocytes, % | 3 | 2 | 1 |
| Platelets, (thousand x 10⁹/L) | 120 | 240 | 236 |
| Eosinophils, % | 3 | 3 | 1 |

| Serology | | | |
|---|---|---|---|
| HBs Ag (ng/mL) | 129 | 117 | - |
| HBcor IgG | +++ | +++ | +++ |
| HBcor IgM | + | - | - |
| PCR HBV | + | + | - |
| Anti HBs Ag | 10 U/ml | 10 U/ml | 10 U/ml |
| PCR HDV | + | + | - |

| Blood biochemistry | | | |
|---|---|---|---|
| Bilirubin (µm/L) | 19.0 | 13.0 | 12.0 |
| ALT (mmol/hr.L.) | 2.8 | 0.09 | 0.38 |

**Table 23.**

| **Patient immunologic status at the treatment with the GSSG•Pt drugs at the patient with acute viral hepatitis B** | | |
|---|---|---|
| Index | Before the treatment | After the treatment |
| CD4⁺ | 680 | 504.5 |
| CD8⁺ | 467 | 560.7 |
| CD4⁺/CD8⁺ | 1. | 0.93 |
| CD4⁺ CD8⁺ | 363 | 256 |
| CD16⁺ | 595.7 | 378.4 |
| CD72⁺ | 483.4 | 485.9 |
| CIC | 112.85 | 93.2 |
| HLADR | 513 | 467 |
| FasAg (CD95+), % | 1.3 | .23 |

**Table 24.**

| **Patient cytokine status at the treatment with the GSSG•Pt drugs at the patient with acute viral hepatitis B** | | |
|---|---|---|
| Index | Before the treatment (pg/mL) | After the treatment (pg/mL) |
| IL-1β | 296.5 | 98.5 |
| IL-2 | 121 | 92 |
| IL-6 | 189 | 260 |
| IL-10 | 1001 | 226 |
| IFN-γ | 350.9 | 108 |
| IL-4 | 1650 | 450 |
| TNF-α | 1073 | 158 |

**Table 25.**

| **Changes in hematological, serological and biochemical parameters before and after the treatment with the GSSG•Pt drug use at the patient with chronic viral hepatitis C** | | | | |
|---|---|---|---|---|
| Parameter | Prior to the treatment | After the treatment | 1 month after the treatment | 3 months after the treatment |
| Hematology | | | | |
| Erythrocytes (x 10¹²/L) | 4.1 | 4.0 | 4.4 | 4.6 |
| Hemoglobin, g/L | 120 | 140 | 136 | 148 |
| Leukocytes (x10⁹/L) | 8.9 | 6.7 | 5.7 | 5.8 |
| Lymphocytes, % | 30 | 47 | 46 | 17 |
| Stab neutrophils, % | 5 | 2 | 1 | 2 |
| Segmented neutrophils, % | 52 | 41 | 38 | 68 |
| Monocytes, % | 10 | 8 | 11 | 12 |
| Platelets (thousandx10⁹/L) | 240 | 280 | 215 | 265 |
| Eosinophils, % | 3 | 2 | 4 | 1 |

| Serology | | | | |
|---|---|---|---|---|
| HBs Ag (ng/mL) | - | - | - | - |
| Anti HBcor IgG | +++ | +++ | +++ | +++ |
| Anti HBcor IgM | - | - | - | - |
| PCR HCV | + | - | - | - |
| Anti HBs Ag | 10 U/ml | 10 U/ml | 75 U/ml | 75 U/ml |
| Anti HCV IgG | +++cor | +++cor | +++cor++ns | +++cor++ns |

| Blood biochemistry | | | | |
|---|---|---|---|---|
| Bilirubin (µmol/L) | 34.0 | 22.0 | 24.0 | 18.0 |
| ALT (mmol/hr.L.) | 1.8 | 0.52 | 0.18 | 0.3 |

**Table 26.**

| **Patient immunologic status before and after the treatment with the GSSG•Pt drugs at the patient with chronic viral hepatitis C** | | |
|---|---|---|
| Index | Before the treatment | After the treatment |
| CD4⁺ | 519 | 679 |
| CD8⁺ | 541 | 450 |
| CD4⁺/CD8⁺ | 1 | 1.23 |
| CD4⁺ CD8⁺ | 363 | 2568 |
| CD16⁺ | 573.7 | 358 |
| CD72⁺ | 676 | 459 |
| CIC | 180,7 | 92 |
| HLA-DR | 715 | 424 |
| CD95⁺(FasAg), % | 5 | 45 |

**Table 27.**

| **Patient cytokine status at the treatment with the GSSG•Pt drugs at the patient with chronic viral hepatitis C** | | |
|---|---|---|
| Index | Before the treatment (pg/mL) | After the treatment (pg/mL) |
| IL-1β | 239,5 | 108,3 |
| IL-2 | 128 | 88 |
| IL-6 | 156 | 250 |
| IL-10 | 1133 | 887 |
| IFN-γ | 307,9 | 280 |
| IL-4 | 1800 | 600 |
| TNF-α | 976 | 358 |

**Table 28.**

| **Results of the GSSG and GSSG•Pt action on normal lymphocyte count in vitro during 48-hour incubation* (M ± m) (p<0.05)** | | | |
|---|---|---|---|
| Tested articles (concentration 100 µg/mL) | Cell state | 24 hours, x10³ | 48 hours, x10³ |
| Control^{xx} | Cells - total | 264 ±30 | 285 ±36 |
| | Dead cells - % | 7.0 ± 1.2 | 12.0 ±1.4 |
| GSSG | Cells - total | 265 ± 34 | 280 ± 38 |
| | Dead cells - % | 8.0±1.5 | 13.0 ± 1.3 |
| GSSG•Pt | Cells - total | 269 ± 32 | 287 ± 35 |
| | Dead cells - % | 6.0 ±1.1 | 12.0 ± 1.6 |

| | | | |
|---|---|---|---|
| * - initial cell count - 250 thousand/mL. | | | |
| ^{xx} - incubation in RPMI 1640 medium without the drugs adding 10% fetal calf serum. | | | |

**Table 29.**

| **HL-60 cell growth development during 48-hour incubation after the GSSG and GSSG•Pt treatment* (M ± m) (p<0.05).** | | | |
|---|---|---|---|
| Tested articles (concentration 100 µg/mL) | Cells' state | 24 hours, x10³ | 48 hours, x10³ |
| Control^{xx} | Cells - total | 800 ± 30 | 2785 ±36 1 |
| | Dead cells - % | 3.0 ± 1.2 | 6.0 ± 1.4 |
| GSSG | Cells - total | 515 ± 54 | 780 ± 38 |
| | Dead cells - % | 27.0 ± 3.3** | 53 ± 6.3** |
| GSSG•Pt | Cells - total | 360 ± 32 | 283 ± 35 |
| | Dead cells - % | 87.0 ± 8.5^{**x} | 100^{**x} |

| | | | |
|---|---|---|---|
| * - initial cell count - 250 thousand/mL. | | | |
| ** - significant differences comparing to the control, ^{x} - GSSG (p<0.05). | | | |
| ^{xx} - incubation in RPMI 1640 medium without the drugs adding 10% fetal calf serum. | | | |

**Table 30.**

| **GSSG•Pt effects on normal lymphocyte count in vitro during 48-hour incubation* (M ± m) (p<0.05).** | | | |
|---|---|---|---|
| GSSG•Pt, µg/mL | Cells' state | 24 hours, x10³ | 48 hours, x10³ |
| Control^{xx} | Cells - total | 264 ± 30 | 285 ±36 |
| | Dead cells - % | 7.0 ± 1.2 | 12.0 ± 1.4 |
| | DNA apoptotic degradation | - | - |
| 10 | Cells - total | 266 ± 28 | 285 ± 34 |
| | Dead cells - % | 7.5 ± 1.5 | 11.4 ± 2.0 |
| | DNA apoptotic degradation | - | - |
| 100 | Cells - total | 269 ± 32 | 287 ± 35 |
| | Dead cells - % | 6.0 ± 1.1 | 12.0 ± 1.6 |
| | DNA apoptotic degradation | - | - |

| | | | |
|---|---|---|---|
| * - initial cell count - 250 thousand/mL. | | | |
| ^{xx} - incubation in RPMI 1640 medium without the drugs with addition of 10% fetal calf serum. | | | |

**Table 31.**

| **HL-60 cell growth and apoptosis development during 48-hour incubation after the GSSG•Pt treatment * (M ± m) (p<0.05)** | | | |
|---|---|---|---|
| GSSG•Pt (concentration, µg/mL) | Cells' state | 24 hours, x10³ | 48 hours, x10³ |
| Control^{xx} | Cells - total | 800 ± 30 | 2785 ±36 1 |
| | Dead cells - % | 3.0 ± 1.2 | 6.0 ± 1.4 |
| | DNA apoptotic degradation | - | - + |
| 10 | Cells - total | 611 ± 52 | 778 ± 35 |
| | Dead cells - % | 26.0 ± 4.2** | 49 ± 5.0** |
| | DNA apoptotic degradation | ⁺ | ⁺ |
| 100 | Cells - total | 360 ± 32 | 283 ± 35 |
| | Dead cells - % | 87.0 ± 8.5^{**x} | 100^{**x} |
| | DNA apoptotic degradation | ⁺ | ⁺ |

| | | | |
|---|---|---|---|
| * - initial cell count - 250 thousand/mL. | | | |
| ** - significant differences comparing to the control (p<0.05). | | | |
| ^{xx} - incubation in RPMI 1640 medium without the drugs with addition of 10% fetal calf serum. | | | |

**Table 32.**

| **C-8 cell growth and apoptosis development during 48-hour incubation after the GSSG•Pt treatment* (M ± m) (p<0.05)** | | | |
|---|---|---|---|
| GSSG•Pt (concentration, µg/mL) | Cell state | 24 hours, x10³ | 48 hours, x10³ |
| Control^{xx} | Cells - total | 109.0 ± 4.0 | 188 ±5.5 |
| | Dead cells - % | 1.5 ± 0.5 | 6.0 ± 1.5 |
| | DNA apoptotic degradation | - | |
| 10 | Cells - total | 54.4±3.8 | 59.7 ± 3.5 |
| | Dead cells - % | 32.0 ± 4.0** | 52 ± 6.0** |
| | DNA apoptotic degradation | ⁺ | ⁺ |
| 100 | Cells - total | 32.6 ± 3.3 | 22.1 ± 2.8 |
| | Dead cells - % | 76.3 ± 7.8^{**x} | 100^{**x} |
| | DNA apoptotic degradation | ⁺ | ⁺ |

| | | | |
|---|---|---|---|
| * - initial cell count - 50 thousand/mL. | | | |
| ** - significant differences comparing to the control (p<0.05). | | | |
| ^{xx} - incubation in DMEM medium without the drugs with addition of 10% fetal calf serum. | | | |

**Table 33.**

| **A-4 cell growth and apoptosis development during 48-hour incubation after the GSSG•Pt treatment* (M ± m) (p<0.05).** | | | |
|---|---|---|---|
| GSSG•Pt (concentration. µg/mL) | Cell state | 24 hours, x10³ | 48 hours, x10³ |
| Control^{xx} | Cells - total | 98.1 ± 5.2 | 167 ± 5.8 |
| | Dead cells - % | 2.0 ± 0.5 | 4.5 ± 1.5 |
| | DNA apoptotic degradation | - | - |
| 10 | Cells - total | 53.7 ± 4.1 | 59.7 ± 3.5 |
| | Dead cells - % | 33.0 ± 3.0** | 55 ± 5.0** |
| | DNA apoptotic degradation | ⁺ | ⁺ |
| 100 | Cells - total | 24.7 ± 2.5 | 13.7 ± 2.4 |
| | Dead cells - % | 61.6 ± 6.5^{**x} | 100^{**x} |
| | DNA apoptotic degradation | ⁺ | ⁺ |

| | | | |
|---|---|---|---|
| * - initial cell count - 50 thousand/mL. | | | |
| ** - significant differences comparing to the control (p<0.05). | | | |
| ^{xx} - incubation in DMEM medium without the drugs with addition of 10% fetal calf serum. | | | |

**Table 34.**

| **C-8 cell growth and apoptosis development during 48-hour incubation after the GSSG•Pt treatment * (M±m) (p<0.05).** | | | |
|---|---|---|---|
| GSSG•Pt (concentration, µg/mL) | Cell state | 24 hours, x10³ | 48 hours, x10³ |
| Control^{xx} | Cells - total | 109.0 ± 4.0 | 188 ±5.5 |
| | Dead cells - % | 1.5 ± 0.5 | 6.0 ± 1.5 |
| | DNA apoptotic degradation | - | |
| | | | |
| 100 | Cells - total | 32.6 ± 3.3 | 22.1 ± 2.8 |
| | Dead cells - % | 76.3 ± 7.8^{**x} | 100^{**x} |
| | DNA apoptotic degradation | ⁺ | ⁺ |

| | | | |
|---|---|---|---|
| * - initial cell count - 50 thousand/mL. | | | |
| ** - significant differences comparing to the control (p<0.05). | | | |
| ^{xx} - incubation in DMEM medium without the drugs with addition of 10% fetal calf serum. | | | |

**Table 35.**

| **p21(--) cell growth and apoptosis development during 48-hour incubation after the GSSG•Pt treatment * (M ± m) (p<0.05).** | | | |
|---|---|---|---|
| GSSG•Pt (concentration, µg/mL) | Cell state | 24 hours, x10³ | 48 hours, x10³ |
| Control^{xx} | Cells - total | 109 ± 4.3 | 191 ± 6.4 |
| | Dead cells - % | 0.5 ± 0.5 | 1.5 ± 0.5 |
| | DNA apoptotic degradation | - | - |
| | | | |
| 100 | Cells - total | 46.7 ± 5.3 | 31.2 ± 2.5 |
| | Dead cells - % | 64.5 ± 6.5^{**x} | 78.0 ± 7.3 ^{**x} |
| | DNA apoptotic degradation | ⁺ | ⁺ |

| | | | |
|---|---|---|---|
| * - initial cell count - 50 thousand/mL. | | | |
| ** - significant differences comparing to the control (p<0.05). | | | |
| ^{xx} - incubation in DMEM medium without the drugs with addition of 10% fetal calf serum. | | | |

**Table 36.**

| **Blood glucose and biochemical blood indices highly correlated with it (r**_{**1**} **and r**_{**2**}**>0.85)* in the patient with diabetes mellitus** | | | |
|---|---|---|---|
| Indices | Before treatment | 1 month after the treatment | 4 months after the treatment |
| Blood glucose, mmol/L | 11.5 - 19.1 | 8.2 - 10.6 | 4.8 - 7.6 |
| cAMP/cGMP | 7.8 | 6.5 | 4.1 |
| TDR (thiol-disulfide ratio) | 1.6 | 1.3 | 0.9 |

| | | | |
|---|---|---|---|
| *-r₁- correlation ratio of the blood glucose development and changing of the cAMP/cGMP ratio; r₂-correlation ratio of the blood glucose development and changing of the thiol-disulfide ratio | | | |

**Table 37.**

| **Hematologic, biochemical and immunologic indices in the patient with diabetes mellitus** | | | |
|---|---|---|---|
| Index | Prior to the treatment | 1 month after the treatment | 4 months after the treatment |
| Hematology | | | |
| Erythrocytes. 10¹²/L | 3.9 | 4.1 | 4.4 |
| Hemoglobin, g/L | 120 | 131 | 143 |
| Platelets, 10⁹/L | 199 | 211 | 263 |
| Leukocytes, 10⁹/L | 8.1 | 7.3 | 6.1 |
| Stab neutrophils, % | 5 | 4 | 4 |
| Segmented neutrophils, % | 38 | 53 | 57 |
| Lymphocytes. 10⁹/L | 52 | 37 | 34 |
| Monocytes, % | 3 | 3 | 4 |
| Eosinophils, % | 2 | 2 | 1 |
| ESR, mm/hour | 17 | 13 | 10 |

| Blood biochemistry | | | |
|---|---|---|---|
| ALT, µmol/hr.L | 0.49 | 0.37 | 0.28 |
| AST, µmol/hr.L | 0.32 | 0.36 | 0.31 |
| Total protein g/L | 75 | 71 | 72 |
| Total bilirubin. µmol/L | 11.2 | 9.4 | 8.1 |
| Total cholesterol, µmol/L | 8.60 | 7.52 | 6.1 |
| Triglycerides , µmol/L | 4.8 | 3.9 | 2.7 |
| Urea, mmol/l | 4.7 | 4.3 | 3.8 |
| Creatinine, mmol/l | 0.146 | 0.104 | 0.097 |

| Immunity | | | |
|---|---|---|---|
| B-lymphocytes (CD20⁺), 10⁶/L | 486 | 409 | 391 |
| T-helpers (CD4⁺), 10⁶/L | 1432 | 1109 | 932 |
| T-suppressors (CD8⁺), 10⁶/L | 1104 | 969 | 710 |
| CD25⁺, 10⁶/L | 463 | 537 | 499 |
| Circulating immune complexes. U | 221 | 156 | 102 |

**Table 38.**

| **Influence of GSSG•Pt and GSSG•Pd in dose of 5 mg/kg on development of antibodies against O-antigen of F.tularensis** | | | | |
|---|---|---|---|---|
| Group | Number of animals per group | Seroconversion frequency (%) | Antibody titre (IU) | P |
| GSSG•Pt | 12 | 80 | 1:80 (1:20-1:80) | <0,05 |
| GSSG•Pd | 15 | 93 | 1:80 (1:20-1:160) | < 0,05 |
| Control | 15 | 66 | 1:20 (1:5-1:20) | |

**Table 39.**

| **Influence of GSSG•Pt and GSSG•Pd in dose of 5 mg/kg on DTHR (delayed-type hypersensitivity reaction) against O-antigen of F.tularensis** | | | |
|---|---|---|---|
| Group | Number of animals per group | Positive reaction frequency, % | DTH index |
| GSSG•Pt | 11 | 73 | 4,18* |
| GSSG•Pd | 10 | 80 | 6,5* |
| Control | 10 | 50 | 0.7 |

| | | | |
|---|---|---|---|
| Note: * P<0,05 | | | |

**Table 40.**

| **Influence of GSSG•Pt and GSSG•Pd in dose of 5 mg/kg on development of antibodies against O-antigen of S. typhi** | | | | |
|---|---|---|---|---|
| Group | Number of animals per group | Seroconversion frequency (%) | Antibody titre (IU) | P |
| GSSG•Pt | 14 | 100 | 1:160 (1:160-1:320) | < 0,05 |
| GSSG•Pd | 15 | 93 | 1:80 (1:40-1:160) | < 0,05 |
| Control | 15 | 66 | 1:20 (1:10-1:40) | |

**Table 41.**

| **Influence of GSSG•Pt and GSSG•Pd in dose of 5 mg/kg on DTHR (delayed-type hypersensitivity reaction) against O-antigen of S. typhi.** | | | |
|---|---|---|---|
| Group | Number of animals per group | Positive reaction frequency, % | DTH index |
| GSSG•Pt | 12 | 100 | 21,17* |
| GSSG•Pd | 10 | 90 | 18,42* |
| Control | 12 | 50 | 12,8 |

| | | | |
|---|---|---|---|
| Note: * P<0,05 | | | |

## Claims

1. A composite comprising an oxidized glutathione-based compound and a metal material in a ratio of between about 3000:1 to about 1:1, wherein the metal material comprises a metal selected from the group consisting of platinum and palladium.

2. The composite of claim 1, wherein the composite comprises the oxidized glutathione-based compound and the metal material in a ratio of between about 1000:1 to about 1:1, 10:1 or 100:1.

3. The composite of claim 1, wherein the metal is platinum.

4. The composite of claim 3, wherein the platinum material is selected from the group consisting of a platinum salt, a coordination compound and an organometallic compound.

5. The composite of claim 4, wherein the platinum material is a platinum coordination compound and, optionally, cis-platin.

6. The composite of claim 1, wherein the oxidized-based glutathione compound has the formula: 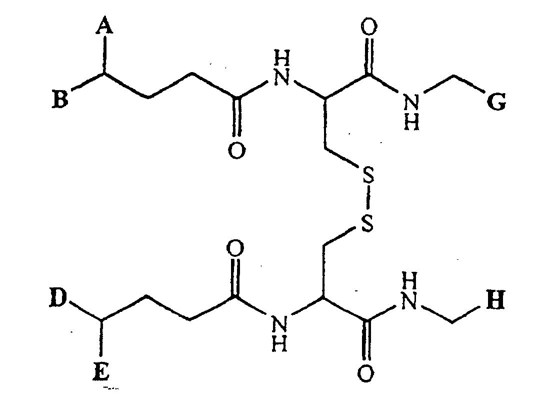 wherein A, B, D, E, G and H can be the same or different and each is selected from the group consisting of an organic unit and salts of the organic unit.

7. The composite of claim 6, wherein A, B, D, E, G and H can be the same or different and each includes a unit selected from the group consisting of amine groups, carboxyl groups and amides.

8. The composite of claim 7, wherein:-
(a) any two of A, B, D, E, G and H are linked to each other by at least one covalent bond;
(b) any two of A, B, D, E, G and H are linked to each other by an amide bond;
(c) A, B, D, E, G and H can be the same or different and each includes an amino acid; or
(d) the oxidized glutathione-based compound is oxidized glutathione and both A and E are -CO₂H, both B and D are -NH₂ and both G and H are -CO₂M, M being a counterion.

9. The composite of claim 7, wherein the oxidized glutathione-based compound is S-thioethylamine glutathione disulfide, *bis*-[DL-6,8-thioetic acid] glutathione disulfide, [β-alanyl-L-histidyl] glutathione disulfide, [9-β-D-ribofuranosyladenyl] glutathione disulfide, *bis*-[L-2-amino-4-[methylthio]butanoic acid] glutathione disulfide, *bis*-[L-phenylalanyl] glutathione disulfide, tetra-dopamine glutathione disulfide or theophylline glutathione disulfide.

10. The composite of claim 7, wherein the oxidized glutathione-based compound has an acylated primary glutamic acid amino group of oxidized glutathione and, optionally, is selected from the group consisting of *bis*-[methionyl] glutathione disulfide, *bis*-[aspartyl] glutathione disulfide, *bis*-[histidyl] glutathione disulfide, *bis*-[3-iodine-tyrosyl] glutathione disulfide, [γ-aminobutanoyl] glutathione disulfide, *bis*-[γ-*h*ydroxybutanoyl] glutathione disulfide, *bis*-[lipoyl] glutathione disulfide, and *bis-*[3,4-dihydroxyphenylalaninyl] glutathione disulfide.

11. The composite of claim 7, wherein the platinum material is cis-platin and the oxidized glutathione-based compound has an amide or phosphoramide bond to a unit selected from the group consisting of heterocyclic carbonic acids and nucleotides and, optionally, is selected from the group consisting of *bis*-[pyridine-3-carbonyl] glutathione disulfide, uridine-5'-monophosphatoyl glutathione disulfide, inosine-5'-monophosphatoyl glutathione disulfide, folliculylsuccinyl glutathione disulfide and glycerol-1,3-disphosphatyl glutathione disulfide.

12. The composite of claim 1, wherein the oxidized glutathione-based compound is chemically interacted with the metal material and, optionally, has a formula:

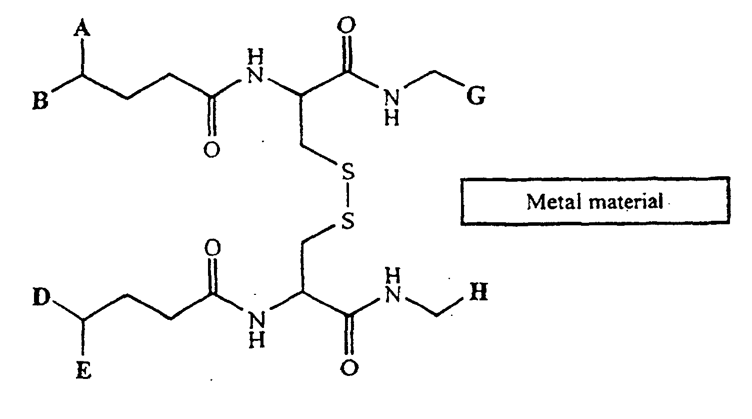

13. A method for stabilizing a disulfide bond of an oxidized glutathione-based compound, comprising interacting the oxidized glutathione-based compound with a metal material comprising a metal selected from the group consisting of platinum and palladium.

14. The method of claim 13, wherein the metal is platinum.

15. The method of claim 14, wherein the platinum material is present in an amount of between about 0.0003 equivalent to about 1 equivalent relative to the oxidized glutathione-based compound,
about 0.001 equivalent to about 0.01 equivalent relative to the oxidized glutathione-based compound,
about 0.001 equivalent to about 0.1 equivalent relative to the oxidized glutathione-based compound, or
about 0.001 equivalent to about 1 equivalent relative to the oxidized glutathione-based compound.

16. The method of claim 14, wherein the platinum material is selected from the group consisting of platinum metal, a salt, a coordination and an organometallic compound, and, optionally, is cis-platin.

17. The method of claim 13, wherein the oxidized glutathione-based compound has the formula: 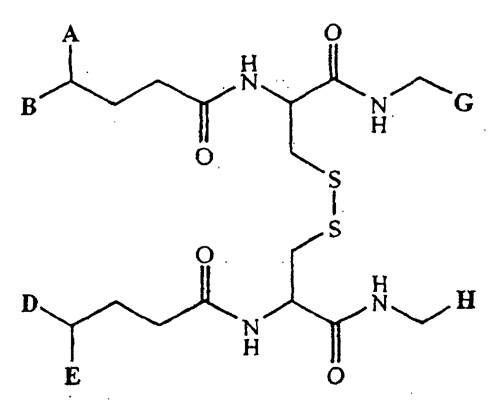 wherein A, B, D, E, G and H can be the same or different and each is selected from the group consisting of an organic unit and salts of the organic unit.

18. The method of claim 13, wherein the interacting comprises:
providing a glutathione tripeptide-based compound; and
reaction of interacting of the glutathione tripeptide sodium salt with an oxidant and a metal material selected from the group comprising platinum and palladium to obtain a tripeptide dimer, that is a compound being a structural analogue of an oxidized glutathione, videlicet, hexapeptide having a stabilized disulfide bond.

19. The method of claim 18, wherein the oxidant is oxygen or hydrogen peroxide.

20. The method of claim 19, wherein the reacting step comprises reacting one equivalent of the glutathione-based compound with less than about 1, or about 0.9, equivalent of hydrogen peroxide and between about 0.0003 equivalent and about 1 equivalent, about 0.001 equivalent and about 0.1 equivalent, about 0.001 equivalent and about 0.01 equivalent, or about 0.001 equivalent and about 1 equivalent of the platinum material.

21. The method of claim 13, wherein the interacting comprises adding between about 0.0003 equivalent to about 1 equivalent of the platinum material to 1 about equivalent of the oxidized glutathione-based compound.

22. The method of claim 13, wherein the oxidized glutathione-based compound is a salt selected from the group consisting of:
a) alkali metal salts, alkaline earth metal salts and transition metal salts, or
b) lithium salts, sodium salts, potassium salts, magnesium salts, calcium salts, vanadium salts, maganese salts, iron salts, molybdenum salts, zinc salts and silver salts.

23. The method of claim 18 or 22, wherein the oxidized glutathione-based salts selected from the group comprising salts of alkali metals, salts of alkali-earth metals and salts of transistion metals are obtained, i.e. synthesized.

24. The method of claim 13, wherein the oxidized glutathione-based compound is a fluoride-containing salt.

25. A composite as claimed in any of claims 1-12, for use in a method of stimulating endogenous production of cytokines and/or hemopoietic factors to obtain a therapeutic effect in a mammalian body.

26. A composite as claimed in claim 25, for oral administration to the mammalian body.

27. A composite as claimed in claim 25, for use in treating a disease selected from the group consisting of oncological, infectious, immunological, ischemic, neurodegenerative, metabolic, endocrinal and other diseases.

28. The composite of claim 27, wherein:-
(a) the oncological disease is selected from the group consisting of lung cancer, melanoma, cerebral tumours, colorectal cancer, breast cancer, prostate cancer, ovarian cancer, acute lymphoblastic leukosis and acute myeloblastic leukosis;
(b) the infectious disease is selected from the group consisting of tuberculosis, viral hepatitis B, viral hepatitis C, mixed infections (HBV and HCV), herpes, meningitis (sepsis), peritonitis, acute pancreatitis and supprative post-surgery sequalae;
(c) the immunological disease is selected from the group consisting of AIDS, immuosuppressions of infectious origin, immunosuppressions of radiation origin, immunosupressions of toxic origin, glomerulonephritis, rheumatoid arthritis, collagenosis, systemic lupus erythematois and allergic conditions;
(d) the ischemic disease is selected from the group consisting of ischemic cerebral conditions and ischemic heart disease;
(e) the neurodegenerative disease is selected from the group consisting of Alzheimer's disease, Parkinson's disease, hereditary (Huntington's) chorea, amyotrophic lateral sclerosis, neuro-AIDS and demyelinating diseases;
(f) the neurodegenerative disease is a neurobehavioral disease selected from the group consisting of narcotic abstinence, cerebral hypoxia, manic-depressive psychosis and schizophrenia;
(g) the metabolic disease is atherosclerosis; and/or
(h) the endocrinal disease is associated with hypothalamic-hypophysial-ovarian function disorder.

29. The composite of claim 25, wherein the therapeutic effect comprises a process selected from the group consisting of regulating proliferation in normal cells, regulating differentiation in normal cells and inducing apoptosis of transformed cells.

30. A composite of claim 25, wherein said composite is for administration to the mammalian body in a solution form selected from the group consisting of inhalation solutions, local instillations, eye drops, intranasal introductions, ointments for epicutaneous applications, intravenous solutions, injection solutions and suppositories.

31. The composite of claim 30, wherein the solution has a concentration of between about 1% to about 10% of the composite.

32. A composite as claimed in claim 25, for administration to the mammalian body in an injectable form.

33. The composite of claim 32, wherein the injectable form comprises the composite in a solution in a concentration of between about 0.01% to about 3.0%.

34. A composite as claimed in claim 25 or 32, for administration to the mammalian body in a dosage of between about 0.01 mg/kg to about 1.0 mg/kg by body weight, or between about 1 mg/m² to about 100 mg/m² by body surface.

35. A method of enhancing and prolonging the ability of an oxidized glutathione-based compound to stimulate endogenous production of cytokine and hemopoietic factors, said method comprising interacting the oxidized glutathione-based compound with a metal material in a ratio of between about 3000:1 to about 1:1 wherein the metal material comprises a metal selected from the group consisting of platinum and palladium.

36. The composite of claim 25, wherein said cells are in a mammalian body and said composite is introduced into said body at a rate of from about 0.01 mg/kg to about 1.0 mg/kg of body weight at least one time a day for at least one day, or said composite is introduced topically to a topical area at a dose of from about 1.0 mg/m² to about 100 mg/m² of topical area.

37. A composite as claimed in any of claims 1-12, for treating a subject having a disease by administration of said composite to the subject in an amount effective to stimulate endogenous production of cytokines and/or hemopoietic factors and to obtain a therapeutic effect.

38. A medicinal agent for regulating endogenous production of cytokines and hemopoietic factors and/or reproducing cytokine induced effects, to regulate metabolism, proliferation, differentiation and apoptosis induction in normal, tumour- and/or virus-transformed cells, said agent containing an effective amount of a composite in accordance with any of claims 1-12 as an active principle.

39. The medicinal agent of claim 38 for treating an oncological, infectious, immunological, hematological, ischemical, neurodegenerative, and/or metabolic disorder and/or an endocrine disease.

40. The agent of claim 38 or 39, wherein the composite comprises GSSG•Pt and is for treating lung cancer,
comprises bis-[3-iodine-tyrosyl]-GSSG•Pt and is for treating melanoma,
comprises bis-[dopamine]-GSSG•Pt and is for treating cerebral tumours,
comprises bis-[cysteamine]-GSSG•Pt and is for treating colorectal cancer,
comprises cysteamine-GSSG•Pt and is for treating breast cancer,
comprises a dizinc salts of GSSG•Pt and is for treating prostate cancer,
comprises theophylline-GSSG•Pt and is for treating ovarian cancer,
comprises lithium salt-GSSG•Pt and is for treating acute lymphoblastic leukosis,
is selected from the group consisting of dilithium salt-GSSG•Pt and cystamine-GSSG•Pt and combinations thereof and is for treating acute myeloblastic leukosis,
comprises bis-[histidyl]-GSSG•Pt and is for treating tuberculosis,
is selected from the group consisting of GSSG•Pt and inosine-5-monophosphatyl-GSSG•Pt and is for treating diseases selected from the group consisting of viral hepatitis B, viral hepatitis C, and mixed-infections thereof,
is selected from the group consisting of GSSG•Pt and inosine-5-monophosphatyl-GSSG•Pt and is for treating herpes,
comprises tetra-dopamine- GSSG•Pt and is for treating diseases selected from the group consisting of meningitis, sepsis,
is selected from the group consisting of GSSG•Pt and tetra-dopamine-GSSG•Pt and combinations thereof and is for treating peritonitis,
is selected from the group consisting of antigen-GSSG•Pt and/or antibody-GSSG•Pt and is for treating of extra harzardous infetions of viral origin, in particular, Rift Valley fever and bacterial origin, in particular, tularemia,
is selected from the group consisting of GSSG•Pt and inosine-5-monophospharyl-GSSG•Pt and combinations thereof and is for treating acute pancreatitis,
is selected from the group consisting of GSSG•Pt and inosine-5-monophosphatyl-GSSG•Pt and combinations thereof and is for treating suppurative post-surgery sequalae,
is selected from the group consisting of GSSG•Pt and uridine-[5-monophosphatyl]-GSSG•Pt and combinations thereof and is for treating AIDS,
is selected from the group consisting of GSSG•Pt and uridine -[5-monophsphatyl]-GSSG•Pt and combinations thereof and is for treating immunosuppressions of infectious origin,
is selected from the group consisting of GSSG•Pt and a lithium salt of GSSG•Pt and combinations thereof and is for treating glomerulonephritis, rhuematoid arthritis, collagenosis, or systemic lupus erythematosus,
is selected from the group consisting of GSSG•Pt and dihydrofluoride-GSSG•Pt and combinations thereof and is for treating an atopic form of an allergic condition,
consists of the vanadium salt of the GSSG•Pt composite and is for treating diabetes-type I,
comprises bis-[lipoyl]-GSSG•Pt and is for treating diabetes-type II,
comprises bis-[phenylalany]-GSSG•Pt and is for treating an ischemic cerebral condition,
comprises [β-alanyl-L-histidy]-GSSG•Pt and is for treating an ischemic heart disease,
comprises glycerol-[1,3-diphosphaty-GSSG•Pt and is for treating an ischemic heart disease manifested mainly as a syndrome of functional myocardial failure,
comprises bis-[3,4-dihydroxyphenylallanyl]-GSSG•Pt and is for treating a neurodegenerative disease,
comprises bis-[3,4-duhydroxyphenylalanyl]-GSSG•Pt and is for treating a demyelinating disease,
comprises gamma-hydroxy-[butanoyl]-GSSG•Pt and is for treating cerebral hypoxia,
comprises gamma-amino-[butanoyl]-GSSG•Pt and is for treating manic-depressive psycosis,
comprises bis-[nicotinoyl]-GSSG•Pt and is for treating a disease manifested with metabolic disorders in balance of vessel atheroscerosis-forming lipoproteins, or
comprises folliculyl-[succinyl]-GSSG•Pt and is for treating an endocrinal disease.

## Patentansprüche

1. Gemisch, umfassend eine oxidierte glutathionbasierte Verbindung und ein Metallmaterial in einem Verhältnis von zwischen etwa 3000:1 bis etwa 1:1, wobei das Metallmaterial ein Metall umfaßt, das ausgewählt ist aus der Gruppe, bestehend aus Platin und Palladium.

2. Gemisch nach Anspruch 1, wobei das Gemisch die oxidierte glutathionbasierte Verbindung und das Metallmaterial in einem Verhältnis von zwischen etwa 1000:1 bis etwa 1:1, 10:1 oder 100:1 umfaßt.

3. Gemisch nach Anspruch 1, wobei das Metall Platin ist.

4. Gemisch nach Anspruch 3, wobei das Platinmaterial ausgewählt ist aus der Gruppe, bestehend aus einem Platinsalz, einer Koordinationsverbindung und einer Organometallverbindung.

5. Gemisch nach Anspruch 4, wobei das Platinmaterial eine Platin-Koordinationsverbindung und, optional, cis-Platin ist.

6. Gemisch nach Anspruch 1, wobei die oxidierte glutathionbasierte Verbindung die folgende Formel aufweist: 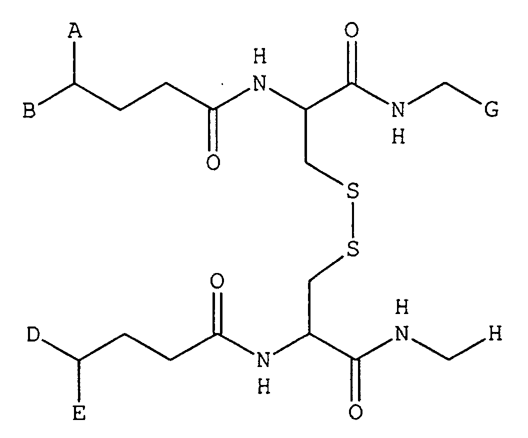 wobei A, B, D, E, G und H gleich oder verschieden sein können und jeweils ausgewählt sind aus der Gruppe, bestehend aus einer organischen Einheit und Salzen der organischen Einheit.

7. Mischung nach Anspruch 6, wobei A, B, D, E, G und H gleich oder verschieden sein können und jeweils eine Einheit beinhalten, die ausgewählt ist aus der Gruppe, bestehend aus Aminogruppen, Carboxylgruppen und Amiden.

8. Mischung nach Anspruch 7, wobei:
(a) beliebige zwei A, B, D, E, G und H miteinander durch mindestens eine kovalente Bindung verbunden sind;
(b) beliebige zwei A, B, D, E, G und H miteinander durch eine Amidbindung verbunden sind;
(c) A, B, D, E, G und H gleich oder verschieden sein können und jeweils eine Aminosäure beinhalten; oder
(d) die oxidierte glutathionbasierte Verbindung oxidiertes Glutathion ist und sowohl A als auch E -CO₂H sind, sowohl B als auch D -NH₂ sind und sowohl G als auch H -CO₂M, sind, wobei M ein Gegenion ist.

9. Mischung nach Anspruch 7, wobei die oxidierte glutathionbasierte Verbindung S-Thioethylaminglutathiondisulfid, bis-[DL-6,8-Thioctansäure]glutathiondisulfid, [β-Alanyl-L-histidyl]glutathiondisulfid, [9,8-D-Ribofuranosyladenyl]glutathiondisulfid, bis-[L-2-Amino-4-[methylthio]-buttersäure]glutathiondisulfid, bis-[L-Phenylalanyl]-glutathiondisulfid, Tetra-Dopaminglutathiondisulfid oder Theophyllinglutathiondisulfid ist.

10. Mischung nach Anspruch 7, wobei die oxidierte glutathionbasierte Verbindung eine acylierte primäre Glutaminsäure-Aminogruppe von oxidiertem Glutathion aufweist und optional ausgewählt ist aus der Gruppe, bestehend aus bis-[Methionyl]glutathiondisulfide, bis-[Aspartyl]glutathiondisulfid, bis-[Histidyl]glutathiondisulfid, bis-[3-Iodtyrosyl]glutathiondisulfid, [γ-Aminobutanoyl]glutathiondisulfid, bis-[γ-Hydroxybutanoyl]-glutathiondisulfid, bis-[Lipoyl]glutathiondisulfid und bis-[3,4-Dihydroxyphenylalanyl]glutathiondisulfid.

11. Mischung nach Anspruch 7, wobei das Platinmaterial cis-Platin ist und die oxidierte glutathionbasierte Verbindung eine Amid- oder Phosphoramidbindung an eine Einheit aufweist, die ausgewählt ist aus der Gruppe, bestehend aus bis-[Pyridin-3-carbonyl]glutathiondisulfid, Uridin-5'-monophosphatoylglutathiondisulfid, Inosin-5'monophysphatoylglutathiondisulfid, Folliculylsuccinylgluthationdisulfid und Glycerin-1,3-diphosphatylglutathiondisulfid.

12. Mischung nach Anspruch 1, wobei die oxidierte glutathionbasierte Verbindung chemisch mit dem Metallmaterial in Wechselwirkung gebracht ist und, optional, die folgende Formel aufweist:

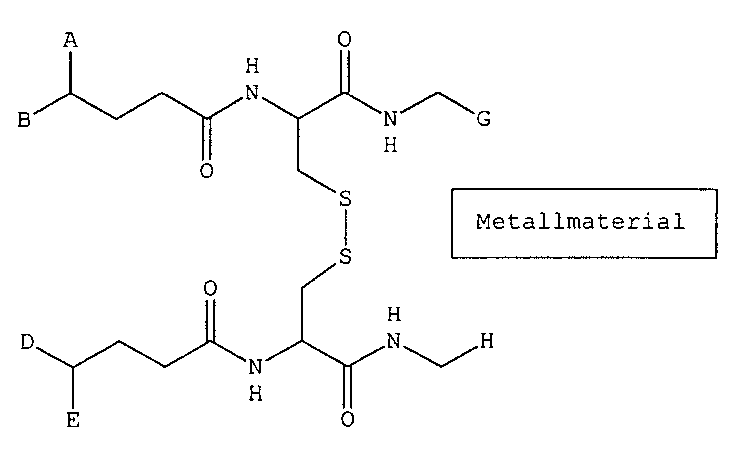

13. Verfahren zur Stabilisierung einer Dusulfidbindung einer oxidierten glutathionbasierten Verbindung, umfassend das In-Wechselwirkung-bringen der oxidierten glutathionbasierten Verbindung mit einem Metallmaterial, umfassend ein Metall, das ausgewählt ist aus der Gruppe, bestehend aus Platin und Palladium.

14. Verfahren nach Anspruch 13, wobei das Metall Platin ist.

15. Verfahren nach Anspruch 14, wobei das Platinmaterial in einer Menge von zwischen etwa 0,0003 Äquivalenten bis etwa 1 Äquivalent im Verhältnis zu der oxidierten glutathionbasierten Verbindung,
etwa 0,001 Äquivalenten bis etwa 0,01 Äquivalenten im Verhältnis zu der oxidierten glutathionbasierten Verbindung,
etwa 0,001 Äquivalenten bis etwa 0,1 Äquivalenten im Verhältnis zu der oxidierten glutathionbasierten Verbindung, oder
etwa 0,001 Äquivalenten bis etwa 1 Äquivalent im Verhältnis zu der oxidierten glutathionbasierten Verbindung vorliegt.

16. Verfahren nach Anspruch 14, wobei das Platinmaterial ausgewählt ist aus der Gruppe bestehend aus Platinmetall, einem Salz, einer Koordinations- und einer Organometallverbindung und, optional, cis-Platin ist.

17. Verfahren nach Anspruch 13, wobei die oxidierte glutathionbasierte Verbindung die folgende Formel aufweist: 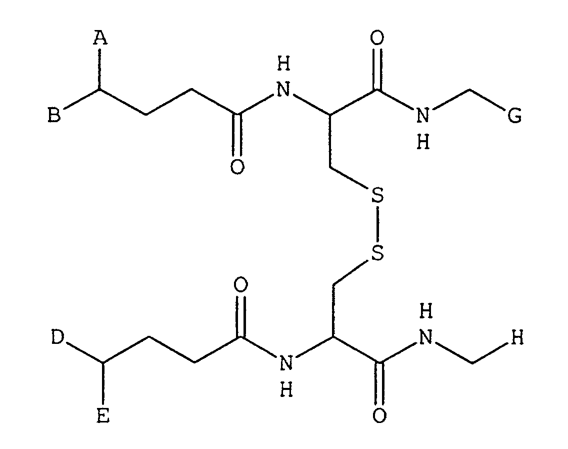 wobei A, B, D, E, G und H gleich oder verschieden sein können und jeweils ausgewählt sind aus der Gruppe, bestehend aus einer organischen Einheit und Salzen der organischen Einheit.

18. Verfahren nach Anspruch 13, wobei die Wechselwirkung umfaßt:
das Bereitstellen einer glutathiontripeptidbasierten Verbindung; und
das Inreaktionbringen das In-Wechselwirkung-bringen des Glutathiontripeptidnatriumsalzes mit einem Oxidationsmittel und einem Metallmaterial, das ausgewählt ist aus der Gruppe, umfassend Platin und Palladium, um ein Tripeptiddimer zu erhalten, das eine Verbindung ist, die ein Strukturanalogon eines oxidierten Glutathions, nämlich ein Hexapeptid mit einer stabilisierten Disulfidbindung, ist.

19. Verfahren nach Anspruch 18, wobei das Oxidationsmittel Sauerstoff oder Wasserstoffperoxid ist.

20. Verfahren nach Anspruch 19, wobei der Reaktionsschritt das Inreaktionbringen eines Äquivalents der glutathionbasierten Verbindung mit weniger als etwa 1 oder etwa 0,9 Äquivalenten Wasserstoffperoxid und zwischen etwa 0,0003 Äquivalenten und etwa 1 Äquivalent, etwa 0,001 Äquivalenten und etwa 0,1 Äquivalenten, 0,001 Äquivalenten und etwa 0,01 Äquivalenten oder etwa 0,001 Äquivalenten und etwa 1 Äquivalenten des Platinmaterials umfaßt.

21. Verfahren nach Anspruch 13, wobei das In-Wechselwirkung-bringen die Zugabe von etwa 0,0003 Äquivalenten bis etwa 1 Äquivalent des Platinmaterials zu etwa 1 Äquivalent der oxidierten glutathionbasierten Verbindung umfaßt.

22. Verfahren nach Anspruch 13, wobei die oxidierte glutathionbasierte Verbindung ein Salz ist, das ausgewählt ist aus der Gruppe, bestehend aus:
a) Alkalimetallsalzen, Erdalkalimetallsalzen und Übergangsmetallsalzen oder
b) Lithiumsalzen, Natriumsalzen, Kaliumsalzen, Magnesiumsalzen, Kalziumsalzen, Vanadiumsalzen, Mangansalzen, Eisensalzen, Molybdänsalzen, Zinksalzen und Silbersalzen.

23. Verfahren nach Anspruch 18 oder 22, wobei die aus der Gruppe, umfassend Salze von Alkalimetallen, Erdalkalimetallsalzen und Salzen von Übergangsmetallen ausgewählten oxidierten glutathionbasierten Salze erhalten, d.h. synthetisiert, sind.

24. Verfahren nach Anspruch 13, wobei die oxidierte glutathionbasierte Verbindung ein fluoridhaltiges Salz ist.

25. Mischung nach einem der Ansprüche 1-12 zur Verwendung in einem Verfahren zur Stimulierung der endogenen Bildung von Zytokinen und/oder hämopoetischen Faktoren, um eine therapeutische Wirkung in einem Säugetierkörper zu erhalten.

26. Mischung nach Anspruch 25 zur oralen Verabreichung an den Säugetierkörper.

27. Mischung nach Anspruch 25 zur Verwendung bei der Behandlung einer Krankheit, die ausgewählt ist aus der Gruppe bestehend aus onkologischen, Infektions-, Immun-, ischämischen, neurodegenerativen, Stoffwechsel-, endokrinen und anderen Krankheiten.

28. Zusammensetzung nach Anspruch 27, wobei
(a) die onkologische Krankheit ausgewählt ist der Gruppe, bestehend aus Lungenkrebs, Melanom, Hirntumoren, kolorektalem Krebs, Brustkrebs, Prostatakrebs, Eierstockkrebs, akuter lymphoblastischer Leukose und akuter myeloblastischer Leukose;
(b) die Infektionskrankheit ausgewählt ist aus der Gruppe, bestehend aus Tuberkulose, viraler Hepatitis B, viraler Hepatitis C, Mischinfektionen (HBV und HCV), Herpes, Meningitis (Sepsis), Peritonitis, akuter Pankreatitis und eitrigen post-operativen Folgeerkrankungen;
(c) die Immunkrankheit ausgewählt ist aus der Gruppe, bestehend aus AIDS, Immunsuppressionen infektiöser Herkunft, strahlungsbedingter Immunsuppressionen, Immunsuppressionen toxischer Herkunft, Glomerulonephritis, rheumatoider Arthritis, Kollagenose, systemischem Lupus erythematosus und allergischen Zuständen;
(d) die ischämische Krankheit ausgewählt ist aus der Gruppe, bestehend aus ischämischen zerebralen Zuständen und ischämischer Herzkrankheit;
(e) die neurodegenerative Krankheit ausgewählt ist aus der Gruppe, bestehend aus Alzheimer-Krankheit, Parkinson-Krankheit, erblicher Chorea (Chorea Huntington), amyotropher Lateralsklerose, Neuro-AIDS und entmyelinisierenden Krankheiten;
(f) die neurodegenerative Krankheit eine neurobehaviorale Krankheit ist, die ausgewählt ist aus der Gruppe, bestehend aus Betäubungsmittelentzug, zerebraler Hypoxie, manisch-depressiver Psychose und Schizophrenie;
(g) die Stoffwechselkrankheit Atherosklerose ist; und/oder
(h) die endokrine Krankheit mit hypothalamischhypophysärer Eierstockfunktionsstörung verbunden ist.

29. Mischung nach Anspruch 25, wobei die therapeutische Wirkung ein Verfahren umfaßt, das ausgewählt ist aus der Gruppe, bestehend aus dem Regulieren der Proliferation bei normalen Zellen, dem Regulieren der Differenzierung bei normalen Zellen und dem Induzieren der Apoptose von transformierten Zellen.

30. Mischung nach Anspruch 25, wobei die Mischung zur Verabreichung an den Säugetierkörper in einer Lösungsform vorgesehen ist, die ausgewählt ist aus der Gruppe, bestehend aus Inhalationslösungen, lokalen Instillationen, Augentropfen, intranasalen Einführmitteln, Salben für epikutane Anwendungen, intravenösen Lösungen, Injektionslösungen und Suppositorien.

31. Mischung nach Anspruch 30, wobei die Lösung eine Konzentration von zwischen etwa 1% bis etwa 10% der Mischung aufweist.

32. Mischung nach Anspruch 25 zur Verabreichung an den Säugetierkörper in einer injizierbaren Form.

33. Mischung nach Anspruch 32, wobei die injizierbare Form die Mischung in einer Lösung in einer Konzentration von zwischen etwa 0,01% bis etwa 3,0% umfaßt.

34. Mischung nach Anspruch 25 oder 32 zur Verabreichung an den Säugetierkörper in einer Dosierung von 0,01 mg/kg bis etwa 1,0 mg/kg Körpergewicht, oder zwischen etwa 1 mg/m² bis etwa 100 mg/m² Körperfläche.

35. Verfahren zur Verbesserung und Verlängerung der Fähigkeit einer oxidierten glutathionbasierten Verbindung, die endogene Bildung von Zytokin und hämopoetischen Faktoren anzuregen, wobei das Verfahren das In-Wechselwirkung-bringen der oxidierten glutathionbasierten Verbindung mit einem Metallmaterial in einem Verhältnis von zwischen etwa 3000:1 bis etwa 1:1 umfaßt, wobei das Metallmaterial ein Metall umfaßt, das ausgewählt ist aus der Gruppe, bestehend aus Platin und Palladium.

36. Mischung nach Anspruch 25, wobei sich die Zellen in einem Säugetierkörper befinden und die Mischung in einer Rate von etwa 0,01 mg/kg bis etwa 1,0 mg/kg Körpergewicht mindestens einmal täglich für mindestens einen Tag in den Körper eingeführt wird, oder die Mischung topisch auf eine topische Fläche in einer Dosis von etwa 1,0 mg/m² bis etwa 100 mg/m² topische Fläche eingeführt wird.

37. Mischung nach einem der Ansprüche 1-12 zur Behandlung einer Person mit einer Krankheit durch Verabreichung der Mischung an die Person in einer Menge, die wirksam ist, um die endogene Bildung von Zytokinen und/oder hämopoetischen Faktoren anzuregen und eine therapeutische Wirkung zu erzielen.

38. Arzneimittel zur Regulierung der endogenen Bildung von Zytokinen und hämopoetischen Faktoren und/oder Reproduktion von zytokininduzierten Wirkungen zur Regulation des Stoffwechsels, der Proliferation, der Differenzierung und der Apoptose-Induktion bei normalen, tumor- und/oder virustransformierten Zellen, wobei das Mittel eine wirksame Menge einer Mischung nach einem der Ansprüche 1-12 als wirksames Prinzip enthält.

39. Arzneimittel nach Anspruch 38 zur Behandlung einer onkologischen, Infektions-, Immun-, hematologischen, ischämischen, neurodegenerativen und/oder Stoffwechselfunktionsstörung und/oder einer endokrinen Krankheit.

40. Mittel nach Anspruch 38 oder 39, wobei die Mischung
GSSG•Pt umfaßt und zur Behandlung von Lungenkrebs vorgesehen ist,
bis-[3-Iodtyrosyl]-GSSG•Pt umfaßt und zur Behandlung von Melanom vorgesehen ist,
bis-[Dopamin]-GSSG•Pt umfaßt und zur Behandlung von Hirntumoren vorgesehen ist,
bis-[Cysteamin]-GSSG•Pt umfaßt und zur Behandlung von kolorektalem Krebs vorgesehen ist,
Cysteamin-GSSG•Pt umfaßt und zur Behandlung von Brustkrebs vorgesehen ist,
ein Di-Zinksalz von GSSG•Pt umfaßt und zur Behandlung von Prostatakrebs vorgesehen ist,
Theophyllin-GSSG•Pt umfaßt und zur Behandlung von Eierstockkrebs vorgesehen ist,
Lithiumsalz-GSSG•Pt umfaßt und zur Behandlung von akuter lymphoblastischer Leukose vorgesehen ist,
ausgewählt ist aus der Gruppe, bestehend aus Di-Lithiumsalz-GSSG•Pt und Cystamin-GSSG•Pt und Kombinationen davon, und zur Behandlung von akuter myeloblastischer Leukose vorgesehen ist,
bis-[Histidyl]-GSSG•Pt umfaßt und zur Behandlung von Tuberkulose vorgesehen ist,
ausgewählt ist aus der Gruppe, bestehend aus GSSG•Pt und Inosin-5-monophosphatyl-GSSG•Pt, und zur Behandlung von Krankheiten vorgesehen ist, die ausgewählt sind aus der Gruppe, bestehend aus viraler Hepatitis B, viraler Hepatitis C und Mischinfektionen davon,
ausgewählt ist aus der Gruppe, bestehend aus GSSG•Pt und Inosin-5-monophosphatyl-GSSG•Pt, und zur Behandlung von Herpes vorgesehen ist,
Tetra-Dopamin-GSSG•Pt umfaßt und zur Behandlung von Krankheiten vorgesehen ist, die ausgewählt sind aus der Gruppe, bestehend aus Meningitis, Sepsis,
ausgewählt ist aus der Gruppe, bestehend aus GSSG•Pt und Tetra-Dopamin-GSSG•Pt und Kombinationen davon, und zur Behandlung von Peritonitis vorgesehen ist,
ausgewählt ist aus der Gruppe, bestehend aus Antigen-GSSG•Pt und/oder Antikörper-GSSG•Pt, und zur Behandlung von besonders gefährlichen Infektionen viralen Ursprungs, insbesondere Rifttalfieber, und bakteriellen Ursprungs, insbesondere Tularämie, vorgesehen ist,
ausgewählt ist aus der Gruppe, bestehend aus GSSG•Pt und Inosin-5-monophosphatyl-GSSG•Pt und Kombinationen davon, und zur Behandlung akuter Pankreatitis vorgesehen ist,
ausgewählt ist aus der Gruppe, bestehend aus GSSG•Pt und Inosin-5-monophosphatyl-GSSG•Pt und Kombinationen davon, und zur Behandlung eitriger postoperativer Folgeerkrankungen vorgesehen ist,
ausgewählt ist aus der Gruppe, bestehend aus GSSG•Pt und Uridin-[5-monophosphatyl]-GSSG•Pt und Kombinationen davon, und zur Behandlung von AIDS vorgesehen ist,
ausgewählt ist aus Gruppe, bestehend aus GSSG•Pt und Uridin-[5-monophosphatyl]-GSSG•Pt und Kombinationen davon, und zur Behandlung von Immunsuppressionen infektiöser Herkunft vorgesehen ist,
ausgewählt ist aus der Gruppe, bestehend aus GSSG•Pt und einem Lithiumsalz von GSSG•Pt und Kombinationen davon, und zur Behandlung von Glomerulonephritis, rheumatoider Arthritis, Kollagenose oder systemischem Lupus erythematosus vorgesehen ist,
ausgewählt ist aus der Gruppe, bestehend aus GSSG•Pt und Dihydrofluorid-GSSG•Pt und Kombinationen davon, und zur Behandlung von atopischen Formen eines allergischen Zustands vorgesehen ist,
aus dem Vanadiumsalz der GSSG•Pt-Mischung besteht und zur Behandlung von Diabetes Typ I vorgesehen ist,
bis-[Lipoyl]-GSSG•Pt umfaßt und zur Behandlung von Diabetes Typ II vorgesehen ist,
bis-[Phenylalanyl]-GSSG•Pt umfaßt und zur Behandlung eines ischämischen zerebralen Zustands vorgesehen ist,
[β-Alanyl-L-histidyl]-GSSG•Pt umfaßt und zur Behandlung einer ischämischen Herzkrankheit vorgesehen ist,
Glycerin-[1,3-Diphosphatyl]-GSSG•Pt umfaßt und zur Behandlung einer ischämischen Herzkrankheit, die sich vornehmlich als Herzmuskelfunktionsversagensyndrom manifestiert, vorgesehen ist,
bis-[3,4-Dihydroxyphenylalanyl)-GSSG•Pt umfaßt und zur Behandlung einer neurodegenerativen Krankheit vorgesehen ist,
bis-[3,4-Dihydroxyphenylalanyl]-GSSG•Pt umfaßt und zur Behandlung einer entmyelinisierenden Krankheit vorgesehen ist,
Gammaamino-[butanoyl]-GSSG•Pt umfaßt und zur Behandlung zerebraler Hypoxie vorgesehen ist,
Gammaamino-[butanoyl]-GSSG•Pt umfaßt und zur Behandlung manisch-depressiver Psychose vorgesehen ist,
bis-[Nicotinoyl]-GSSG•Pt umfaßt und zur Behandlung einer Krankheit vorgesehen ist, die sich mit metabolischen Funktionsstörungen im Gleichgewicht von gefäßatherosklerosebildenden Lipoproteinen manifestiert, oder
Folliculyl-[succinyl]-GSSG•Pt umfaßt und zur Behandlung einer endokrinen Krankheit vorgesehen ist.

## Revendications

1. Composite comprenant un composé à base de glutathion oxydé et une matière métallique en un rapport compris dans l'intervalle d'environ 3000:1 à environ 1:1, dans lequel la matière métallique comprend un métal choisi dans le groupe consistant en platine et palladium.

2. Composite suivant la revendication 1, ledit composite comprenant le composé à base de glutathion oxydé et la matière métallique en un rapport compris dans l'intervalle d'environ 1000:1 à environ 1:1, 10:1 ou 100:1.

3. Composite suivant la revendication 1, dans lequel le métal est le platine.

4. Composite suivant la revendication 3, dans lequel la matière à base de platine est choisie dans le groupe consistant en un sel de platine, un composé de coordination de platine et un composé organométallique de platine.

5. Composite suivant la revendication 4, dans lequel la matière à base de platine est un composé de coordination de platine et, éventuellement, le *cis*-platine.

6. Composite suivant la revendication 1, dans lequel le composé à base de glutathion oxydé répond à la formule : 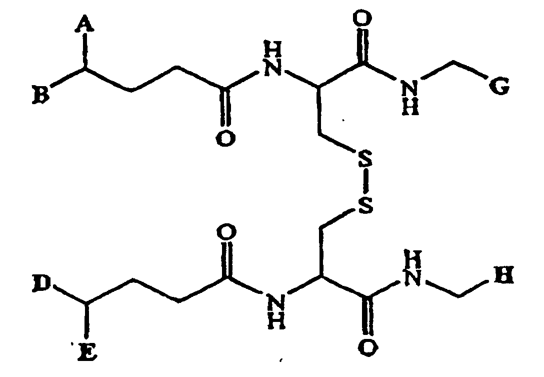 dans laquelle A, B, D, E, G et H peuvent être identiques ou différents et chacun est choisi dans le groupe consistant en un motif organique et des sels du motif organique.

7. Composite suivant la revendication 6, dans lequel A, B, D, E, G et H peuvent être identiques ou différents et chacun comprend un motif choisi dans le groupe consistant en des groupes amine, des groupes carboxyle et des amides.

8. Composite suivant la revendication 7, dans lequel :
(a) deux quelconques de A, B, D, E, G et H sont liés l'un à l'autre par au moins une liaison covalente ;
(b) deux quelconques de A, B, D, E, G et H sont liés l'un à l'autre par une liaison amide ;
(c) A, B, D, E, G et H peuvent être identiques ou différents et comprennent chacun un aminoacide ; ou
(d) le composé à base de glutathion oxydé est le glutathion oxydé et A et E représentent l'un et l'autre un groupe -CO₂H, B et D représentent l'un et l'autre un groupe - NH₂ et G et H représentent l'un et l'autre un groupe -CO₂M, M étant un ion complémentaire.

9. Composite suivant la revendication 7, dans lequel le composé à base de glutathion oxydé est le disulfure de S-thioéthylamine-glutathion, le disulfure de bis-[acide DL-6,8-thioétique]glutathion, le disulfure de [β-alanyl-L-histidyl]glutathion, le disulfure de [9-β-D-ribofurannosyladényl]glutathion, le disulfure de *bis*-[acide L-2-amino-4-[méthylthiobutanoïque]glutathion, le disulfure de *bis*-[L-phénylalanyl]glutathion, le disulfure de tétradopamine-glutathion ou le disulfure de théophylline-glutathion.

10. Composite suivant la revendication 7, dans lequel le composé à base de glutathion oxydé possède un groupe amino d'acide glutamique primaire acylé du glutathion oxydé et, éventuellement, est choisi dans le groupe consistant en le disulfure de *bis*-[méthionyl]glutathion, le disulfure de *bis*-[aspartyl]glutathion, le disulfure de *bis*-[histidyl] glutathion, le disulfure de *bis*-[3-iodo-tyrosyl]glutathion, le disulfure de [γ-aminobutanoyl]glutathion, le disulfure de *bis*-[γ-hydroxybutanoyl]-glutathion, le disulfure de *bis*-[lipoyl]glutathion et le disulfure de *bis*-[3,4-dihydroxyphénylalaninyl]glutathion.

11. Composite suivant la revendication 7, dans lequel la matière à base de platine est le *cis*-platine et le composé à base de glutathion oxydé possède une liaison amide ou phosphoramide à un motif choisi dans le groupe consistant en des acides carboniques hétérocycliques et des nucléotides et, éventuellement, est choisi dans le groupe consistant en le disulfure de *bis*-[pyridine-3-carbonyl]glutathion, le disulfure d'uridine-5'-monophosphatoyl-glutathion, le disulfure d'inosine-5'-monophosphatoyl-glutathion, le disulfure de folliculylsuccinyl-glutathion et le disulfure de glycérol-1,3-diphosphatyl-glutathion.

12. Composite suivant la revendication 1, dans lequel le composé à base de glutathion oxydé est soumis à une interaction chimique avec la matière métallique et, facultativement, répond à la formule :

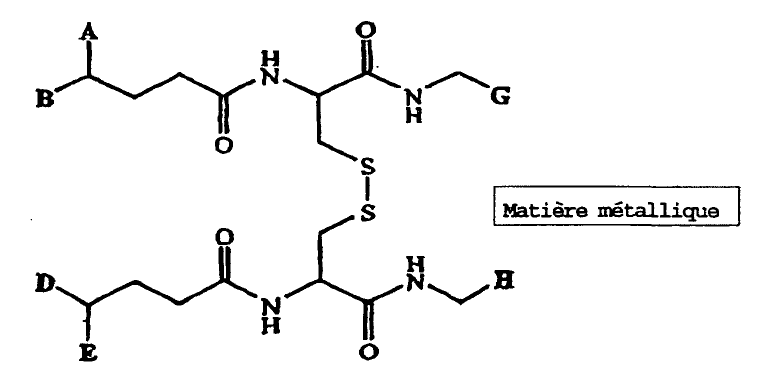

13. Procédé pour stabiliser une liaison disulfure d'un composé à base de glutathion oxydé, comprenant l'interaction du composé à base de glutathion oxydé avec une matière métallique comprenant un métal choisi dans le groupe consistant en platine et palladium.

14. Procédé suivant la revendication 13, dans lequel le métal est le platine.

15. Procédé suivant la revendication 14, dans lequel la matière à base de platine est présente en une quantité comprise dans l'intervalle d'environ 0,0003 équivalent à environ 1 équivalent par rapport au composé à base de glutathion oxydé,
d'environ 0,001 équivalent à environ 0,01 équivalent par rapport au composé à base de glutathion oxydé,
d'environ 0,001 équivalent à environ 0,1 équivalent par rapport au composé à base de glutathion oxydé, ou
d'environ 0,001 équivalent à environ 1 équivalent par rapport au composé à base de glutathion oxydé.

16. Procédé suivant la revendication 14, dans lequel la matière à base de platine est choisie dans le groupe consistant en platine métallique, un sel de platine, un composé de coordination de platine et un composé organométallique de platine et est éventuellement le *cis*-platine.

17. Procédé suivant la revendication 13, dans lequel le composé à base de glutathion oxydé répond à la formule : 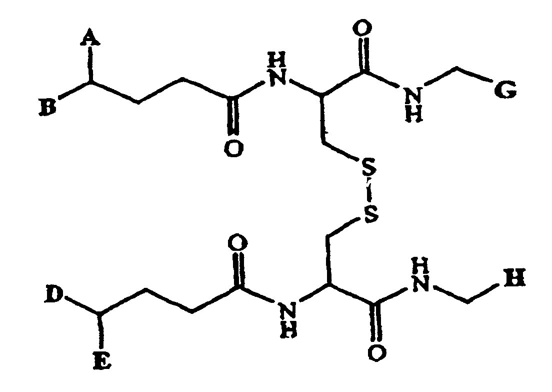 dans laquelle A, B, D, E, G et H peuvent être identiques ou différents et sont choisis chacun dans le groupe consistant en un motif organique et des sels du motif organique.

18. Procédé suivant la revendication 13, dans lequel l'interaction comprend les étapes consistant :
à fournir un composé à base de tripeptide-glutathion ;
à conduire une réaction d'interaction du sel de sodium de tripeptide-glutathion avec un oxydant et une matière métallique choisie dans le groupe comprenant le platine et le palladium pour obtenir un dimère de tripeptide, c'est-à-dire un composé qui est un analogue structural d'un glutathion oxydé, c'est-à-dire un hexapeptide ayant une liaison disulfure stabilisée.

19. Procédé suivant la revendication 18, dans lequel l'oxydant est l'oxygène ou le peroxyde d'hydrogène.

20. Procédé suivant la revendication 19, dans lequel l'étape de réaction comprend la réaction d'un équivalent du composé à base de glutathion avec une quantité inférieure à environ 1, ou environ 0,9, équivalent de peroxyde d'hydrogène à une quantité comprise dans l'intervalle d'environ 0,0003 équivalent à environ 1 équivalent, d'environ 0,001 équivalent à environ 0,1 équivalent, d'environ 0,001 équivalent à environ 0,01 équivalent ou d'environ 0,001 équivalent à environ 1 équivalent de la matière à base de platine.

21. Procédé suivant la revendication 13, dans lequel l'interaction comprend l'addition d'environ 0,0003 équivalent à environ 1 équivalent de la matière à base de platine à environ 1 équivalent du composé à base de glutathion oxydé.

22. Procédé suivant la revendication 13, dans lequel le composé à base de glutathion oxydé est un sel choisi dans le groupe consistant en :
a) des sels de métaux alcalins, des sels de métaux alcalino-terreux, des sels de métaux de transition ou
b) des sels de lithium, des sels de sodium, des sels de potassium, des sels de magnésium, des sels de calcium, des sels de vanadium, des sels de manganèse, des sels de fer, des sels de molybdène, des sels de zinc et des sels d'argent.

23. Procédé suivant la revendication 18 ou 22, dans lequel les sels à base de glutathion oxydé, choisis dans le groupe comprenant des sels de métaux alcalins, des sels de métaux alcalino-terreux et des sels de métaux de transition, sont obtenus, c'est-à-dire synthétisés.

24. Procédé suivant la revendication 13, dans lequel le composé à base de glutathion oxydé est un sel contenant un fluorure.

25. Composite suivant l'une quelconque des revendications 1 à 12, destiné à être utilisé dans une méthode pour stimuler la production endogène de cytokines et/ou de facteurs hématopoïétiques pour obtenir un effet thérapeutique dans l'organisme d'un mammifère.

26. Composite suivant la revendication 25, pour l'administration orale à l'organisme d'un mammifère.

27. Composite suivant la revendication 25, destiné à être utilisé dans le traitement d'une maladie choisie dans le groupe consistant en des maladies oncologiques, infectieuses, immunologiques, ischémiques, neurodégénératives, métaboliques, endocriniennes et d'autres maladies.

28. Composite suivant la revendication 27, dans lequel :
(a) la maladie oncologique est choisie dans le groupe consistant en le cancer du poumon, un mélanome, des tumeurs cérébrales, le cancer colorectal, le cancer du sein, le cancer de la prostate, le cancer des ovaires, la leucose lymphoblastique aiguë et la leucose myéloblastique aiguë ;
(b) la maladie infectieuse est choisie dans le groupe consistant en la tuberculose, l'hépatite virale B, l'hépatite virale C, des infections mixtes (HBV et HCV), l'herpès, la méningite (septicémie), la péritonite, la pancréatite aiguë et les séquelles post-chirurgicales suppurées ;
(c) la maladie immunologique est choisie dans le groupe consistant en le SIDA, des immunosuppressions d'origine infectieuse, des immunosuppressions dues à un rayonnement, des immunosuppressions d'origine toxique, la glomérulonéphrite, la polyarthrite rhumatoide, la collagénose, le lupus érythémateux disséminé et des états allergiques ;
(d) la maladie ischémique est choisie dans le groupe consistant en des affections cérébrales ischémiques et une maladie cardiaque ischémique ;
(e) la maladie neurodégénérative est choisie dans le groupe consistant en la maladie d'Alzheimer, la maladie de Parkinson, la chorée héréditaire (de Huntington), la sclérose latérale amyotrophique, le neuro-SIDA et des maladies démyélinisantes ;
(f) la maladie neurodégénérative est une maladie neurocomportementale choisie dans le groupe consistant en l'abstinence des narcotiques, l'hypoxie cérébrale, la psychose maniaco-dépressive et la schizophrénie ;
(g) la maladie métabolique est l'athérosclérose ; et/ou
(h) la maladie endocrinienne est associée à un trouble du fonctionnement hypothalamo-hypophysaire-ovarien.

29. Composite suivant la revendication 25, dans lequel l'effet thérapeutique comprend un processus choisi dans le groupe consistant en une régulation de la prolifération des cellules normales et une régulation de la différenciation des cellules normales et l'induction de l'apoptose de cellules transformées.

30. Composite suivant la revendication 25, ledit composite étant destiné à l'administration à l'organisme d'un mammifère, sous forme d'une solution choisie dans le groupe consistant en des solutions pour inhalation, des solutions pour instillations locales, des gouttes ophtalmiques, des solutions pour introduction intranasale, des pommades pour des applications épicutanées, des solutions intraveineuses, des solutions injectables et des suppositoires.

31. Composite suivant la revendication 30, dans lequel la solution a une concentration comprise dans l'intervalle d'environ 1 % à environ 10 % du composite.

32. Composite suivant la revendication 25, destiné à l'administration à l'organisme d'un mammifère sous une forme injectable.

33. Composite suivant la revendication 32, dans lequel la forme injectable comprend le composite dans une solution à une concentration comprise dans l'intervalle d'environ 0,01 % à environ 3,0 %.

34. Composite suivant la revendication 25 ou 32, destiné à l'administration à l'organisme d'un mammifère à une posologie comprise dans l'intervalle d'environ 0,01 mg/kg à environ 1,0 mg/kg de poids corporel, ou dans l'intervalle d'environ 1 mg/m² à environ 100 mg/m² de surface corporelle.

35. Procédé pour accroître et prolonger l'aptitude d'un composé à base de glutathion oxydé à stimuler la production endogène d'une cytokine et de facteurs hématopoïétiques, ledit procédé comprenant l'interaction du composé à base de glutathion oxydé avec une matière métallique en un rapport compris dans l'intervalle d'environ 3000:1 à environ 1:1, dans lequel la matière métallique comprend un métal choisi dans le groupe consistant en platine et palladium.

36. Composite suivant la revendication 25, dans lequel lesdites cellules sont présentes dans l'organisme d'un mammifère et ledit composite est introduit dans ledit organisme à un taux d'environ 0,01 mg/kg à environ 1,0 mg/kg de poids corporel au moins une fois par jour pendant au moins un jour, ou bien ledit composite est introduit par application topique à une zone topique à une dose comprise dans l'intervalle d'environ 1,0 mg/m² à environ 100 mg/m² de zone topique.

37. Composite suivant l'une quelconque des revendications 1 à 12, pour le traitement d'un sujet présentant une maladie par administration dudit composite au sujet en une quantité efficace pour stimuler la production endogène de cytokines et/ou de facteurs hématopoïétiques et pour obtenir un effet thérapeutique.

38. Agent médicamenteux pour réguler la production endogène de cytokines et de facteurs hématopoiétiques et/ou reproduire des effets induits par des cytokines, pour réguler le métabolisme, la prolifération, la différenciation et l'induction de l'apoptose dans des cellules normales, des cellules tumorales et/ou des cellules transformées par un virus, ledit agent contenant une quantité efficace d'un composite suivant l'une quelconque des revendications 1 à 12 comme principe actif.

39. Agent médicamenteux suivant la revendication 38, pour le traitement d'un trouble oncologique, infectieux, immunologique, hématologique, ischémique, neurodégénératif et/ou métabolique et/ou d'une maladie endocrinienne.

40. Agent suivant la revendication 38 ou 39, dans lequel le composite comprend du GSSG•Pt et est destiné au traitement du cancer du poumon,
comprend du bis-[3-iodine-tyrosyl]-GSSG•Pt et est destiné au traitement d'un mélanome,
comprend du bis-[dopamine]-GSSG•Pt et est destiné au traitement de tumeurs cérébrales,
comprend du bis-[cystéamine]-GSSG•Pt et est destiné au traitement du cancer colorectal,
comprend du cystéamine-GSSG•Pt et est destiné au traitement du cancer du sein,
comprend un sel double de zinc de GSSG•Pt et est destiné au traitement du cancer de la prostate,
comprend du théophylline-GSSG•Pt et est destiné au traitement du cancer des ovaires,
comprend un sel de lithium-GSSG•Pt et est destiné au traitement de la leucose lymphoblastique aiguë,
est choisi dans le groupe consistant en sel de dilithium-GSSG•Pt et cystamine-GSSG•Pt et leurs associations et est destiné au traitement de la leucose myéloblastique aiguë,
comprend du bis-[histzdyl]-GSSG•Pt et est destiné au traitement de la tuberculose,
est choisi dans le groupe consistant en GSSG•Pt et inosine-5-monophosphatyl-GSSG•Pt et est destiné au traitement de maladies choisies dans le groupe consistant en l'hépatite virale B, l'hépatite virale C et les infections mixtes correspondantes,
est choisi dans le groupe consistant en GSSG•Pt et inosine-5-monophosphatyl-GSSG•Pt et est destiné au traitement de l'herpès,
comprend du tétradopamine-GSSG•Pt et est destiné au traitement de maladies choisies dans le groupe consistant en la méningite et la septicémie,
est choisi dans le groupe consistant en GSSG•Pt et tétradopamine-GSSG•Pt et leurs associations et est destiné au traitement de la péritonite,
est choisi dans le groupe consistant en antigène-GSSG•Pt et/ou anticorps-GSSG•Pt et est destiné au traitement d'infections extrêmement dangereuses d'origine virale, en particulier de la fièvre de la Rift Valley, et d'origine bactérienne, en particulier de la tularémie,
est choisi dans le groupe consistant en GSSG•Pt et inosine-5-monophosphatyl-GSSG•Pt et leurs associations et est destiné au traitement de la pancréatite aiguë,
est choisi dans le groupe consistant en GSSG•Pt et inosine-5-monophosphatyl-GSSG•Pt et leurs associations et est destiné au traitement des séquelles post-chirurgicales suppurées,
est choisi dans le groupe consistant en GSSG•Pt et uridine-[5-monophosphatyl]-GSSG•Pt et leurs associations et est destiné au traitement du SIDA,
est choisi dans le groupe consistant en GSSG•Pt et uridine-[5-monophosphatyl]-GSSG•Pt et leurs associations et est destiné au traitement d'immunosuppressions d'origine infectieuse,
est choisi dans le groupe consistant en GSSG•Pt et un sel de lithium de GSSG•Pt et leurs associations et est destiné au traitement de la glomérulonéphrite, de la polyarthrite rhumatoide, de la collagénose ou du lupus érythémateux disséminé,
est choisi dans le groupe consistant en GSSG•Pt et difluorhydrate-GSSG•Pt et leurs associations et est destiné au traitement d'une forme atopique d'un état allergique,
consiste en le sel de vanadium du composite GSSG•Pt et est destiné au traitement du diabète de type I,
comprend du bis-[lipoyl]-GSSG•Pt et est destiné au traitement du diabète de type II,
comprend bis-[phénylalanyl]-GSSG•Pt et est destiné au traitement d'un état cérébral ischémique,
comprend du [β-alanyl-L-histidyl]-GSSG•Pt et est destiné au traitement d'une maladie cardiaque ischémique,
comprend du glycérol-[1,3-diphosphatyl]-GSSG•Pt et est destiné au traitement d'une maladie cardiaque ischémique se manifestant principalement comme syndrome d'insuffisance cardiaque fonctionnelle,
comprend du bis-[3,4-dihydroxyphénylalanyl]-GSSG•Pt et est destiné au traitement d'une maladie neurodégénérative,
comprend du bis-[3,4-duhydroxyphénylalanyl]-GSSG•Pt et est destiné au traitement d'une maladie démyélinisante,
comprend du gamma-hydroxy-[butanoyl]-GSSG•Pt et est destiné au traitement de l'hypoxie cérébrale,
comprend du gamma-amino-[butanoyl]-GSSG•Pt et est destiné au traitement de la psychose maniaco-dépressive,
comprend du bis-[nicotinoyl]-GSSG•Pt et est destiné au traitement d'une maladie se manifestant par des troubles métaboliques dans l'équilibre des lipoprotéines responsables de la formation de l'athérosclérose des vaisseaux ou
comprend du folliculyl-[succinyl]-GSSG•Pt et est destiné au traitement d'une maladie endocrinienne.
